# EUROPEAN PATENT SPECIFICATION

(11) **EP 2 450 975 B1**
(45) Date of publication and mention of the grant of the patent: **08.01.2014**
(21) Application number: 11179192.7
(22) Date of filing: 29.08.2011
(51) Int. Cl.: H01L 51/50, H01L 51/00, C07D 307/93, C07D 333/50, C07D 405/12, C07D 409/12, C07D 409/14, C07D 491/04, C07D 495/04

(54) **Compound for organic optoelectronic device and organic light emitting diode including the same**
Verbindung für eine organische optoelektrische Vorrichtung, organische lichtemittierende Diode damit und Anzeige mit der organischen lichtemittierenden Diode
Composé pour dispositif opto-électronique organique, diode électroluminescente organique l'incluant et affichage incluant la diode électroluminescente organique

(30) Priority: 04.11.2010 KR 20100109425
(43) Date of publication of application: 09.05.2012
(73) Proprietor: Cheil Industries Inc., Kumi-city, Kyungsangbuk-do 730-030 (KR)
(72) Inventor: Lee, Kyoung-Mi, Uiwang-si, Gyeonggi-do (KR); Jung, Sung-Hyun, Uiwang-si, Gyeonggi-do (KR); Ryu, Dong-Wan, Uiwang-si, Gyeonggi-do (KR); Lee, Seung-Min, Uiwang-si, Gyeonggi-do (KR); Lee, Hyon-Gyu, Uiwang-si, Gyeonggi-do (KR); Chae, Mi-Young, Uiwang-si, Gyeonggi-do (KR); Huh, Dal-Ho, Uiwang-si, Gyeonggi-do (KR); Hong, Jin-Seok, Uiwang-si, Gyeonggi-do (KR)
(74) Representative: Michalski Hüttermann & Partner Patentanwälte

(56) References cited:
- DE-A1-102006 025 846
- DE-A1-102006 031 990
- DE-A1-102009 005 289

## Description

### BACKGROUND OF THE INVENTION

### (a) Field of the Invention

A compound for an organic optoelectronic device that is capable of providing an organic optoelectronic device having good life span, efficiency, electrochemical stability, and thermal stability, an organic light emitting diode including the same, and a display including the organic light emitting diode are disclosed.

### (b) Description of the Related Art

An organic optoelectronic device is, in a broad sense, a device for transforming photo-energy to electrical energy, or conversely, a device for transforming electrical energy to photo-energy.

An organic optoelectronic device may be classified as follows in accordance with its driving principles. A first organic optoelectronic device is an electron device driven as follows: excitons are generated in an organic material layer by photons from an external light source; the excitons are separated into electrons and holes; and the electrons and holes are transferred to different electrodes as a current source (voltage source).

A second organic optoelectronic device is an electron device driven as follows: a voltage or a current is applied to at least two electrodes to inject holes and/or electrons into an organic material semiconductor positioned on the interface of the electrodes; and the device is driven by the injected electrons and holes.

As examples, the organic optoelectronic device includes an organic light emitting diode (OLED), an organic solar cell, an organic photo-conductor drum, an organic transistor, an organic memory device, etc., and it requires a hole injecting or transporting material, an electron injecting or transporting material, or a light emitting material.

Particularly, the organic light emitting diode (OLED) has recently drawn attention due to an increase in demand for flat panel displays. In general, organic light emission refers to transformation of electrical energy to photo-energy.

The organic light emitting diode transforms electrical energy into light by applying current to an organic light emitting material. It has a structure in which a functional organic material layer is interposed between an anode and a cathode. The organic material layer includes a multi-layer including different materials, for example a hole injection layer (HIL), a hole transport layer (HTL), an emission layer, an electron transport layer (ETL), and an electron injection layer (EIL), in order to improve efficiency and stability of an organic light emitting diode.

In such an organic light emitting diode, when a voltage is applied between an anode and a cathode, holes from the anode and electrons from the cathode are injected to an organic material layer. The holes and electrons are recombined to generate excitons having high energy. The generated excitons generate light having certain wavelengths while shifting to a ground state.

Recently, it has become known that a phosphorescent light emitting material can be used for a light emitting material of an organic light emitting diode in addition to the fluorescent light emitting material. Such a phosphorescent material emits lights by transiting the electrons from a ground state to an exited state, non-radiance transiting of a singlet exciton to a triplet exciton through intersystem crossing, and transiting a triplet exciton to a ground state to emit light.

As described above, in an organic light emitting diode, an organic material layer includes a light emitting material and a charge transport material, for example a hole injection material, a hole transport material, an electron transport material, an electron injection material, and so on.

The light emitting material is classified as blue, green, and red light emitting materials according to emitted colors, and yellow and orange light emitting materials to emit colors approaching natural colors.

When one material is used as a light emitting material, a maximum light emitting wavelength is shifted to a long wavelength or color purity decreases because of interactions between molecules, or device efficiency decreases because of a light emitting quenching effect. Therefore, a host/dopant system is included as a light emitting material in order to improve color purity and increase luminous efficiency and stability through energy transfer.

In order to implement excellent performance of an organic light emitting diode, a material constituting an organic material layer, for example a hole injection material, a hole transport material, a light emitting material, an electron transport material, an electron injection material, and a light emitting material such as a host and/or a dopant, should be stable and have good efficiency. However, development of an organic material layer forming material for an organic light emitting diode has thus far not been satisfactory and thus there is a need for a novel material. This material development is also required for other organic photoelectric devices.

In addition, a low molecular organic light emitting diode is fabricated in a form of a thin film device using a vacuum deposition method and has excellent efficiency and life-span characteristics. On the other hand, a polymer organic light emitting diode is fabricated using an inkjet or spin coating method and thus, may cost low but have a large area.

Both low molecular organic light emitting diode and polymer organic light emitting diode may have advantages of spontaneous light emission, high speed response, an optical viewing angle, ultra thin film type, high image quality, durability, wide operation temperature range, and the like and thus, draws attention as a next generation display. In particular, compared with a conventional liquid crystal display (LCD), these low molecular organic light emitting diode and polymer organic light emitting diode spontaneously emit a light and thus, have good readability in a dark place or a light-entering place and needs no backlight. Accordingly, these diodes may have 1/3 of the thickness and the weight of LCD.

In addition, these diodes have more than 1000 times a faster response speed than LCD, that is, a microsecond unit and thus, may realize a perfect motion picture without an after-image. Accordingly, these diodes have made rapid technology developments of 80 times better efficiency and 100 times longer life-span since 1980's first model and are expected to draw attention as an optimal display in a multimedia era and. Recently, these diodes have been rapidly larger like a 40 inch-large organic light emitting diode panel.

A large-sized device should accompany luminous efficiency increase and life-span improvement. Herein, the luminous efficiency of a device may be improved based on smooth combination of holes with electron in an emission layer. However, since an organic material may in general have slower electron mobility than hole mobility, an electron transport layer (ETL) may be used to increase electron injection and mobility from a cathode and simultaneously, block hole mobility, so that holes may be efficiently combined with electron in an emission layer.

In addition, life-span of a device may be improved by preventing crystallization of a material due to Joule heat generated during the operation. Therefore, an organic compound having excellent electron injection and mobility and high electrochemical stability needs to be developed.

Compounds for organic optoelectronic devices are disclosed in the DE 10 2009 005 89 and DE 10 2006 025846.

### SUMMARY OF THE INVENTION

A compound for an organic optoelectronic device that may act as a hole injection and hole transport, or an electron injection and transport, and also act as a light emitting host along with an appropriate dopant is provided.

An organic optoelectronic device having excellent life span, efficiency, driving voltage, electrochemical stability, and thermal stability is provided.

According to one embodiment of the present invention, a compound, according to claim 1, for an organic optoelectronic device is provided.

The compound for an organic optoelectronic device may be a compound represented by the following Chemical Formula 6.

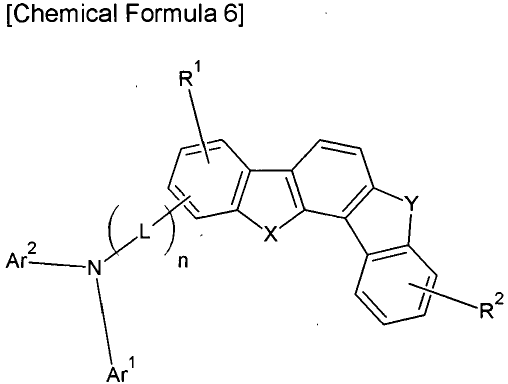

In Chemical Formula 6, X is O, S, SO₂ (O=S=O), PO (P=O) or CO (C=O), Y i s CR'R" or NR', R', R", R¹ and R² are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, the Ar¹ and Ar² are the same or different and independently a substituted or unsubstituted C6 to C30 aryl group, or a substituted or unsubstituted C2 to C30 heteroaryl group, L is a single bond, a substituted or unsubstituted C2 to C6 alkenylene group, a substituted or unsubstituted C2 to C6 alkynylene group, a substituted or unsubstituted C6 to C30 arylene group, a substituted or unsubstituted heteroarylene group, or a combination thereof, and n is 0 or 1.

The compound for an organic optoelectronic device may be a compound represented by the following Chemical Formula 7.

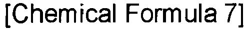

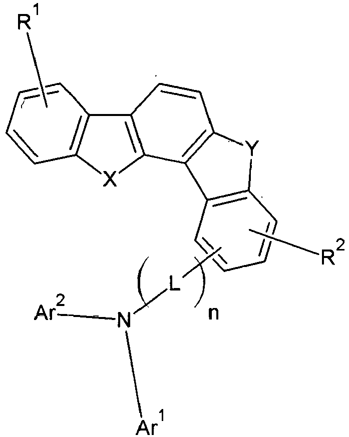

In Chemical Formula 7, X is O, S, SO₂ (O=S=O), PO (P=O) or CO (C=O), Y is CR'R" or NR', R', R", R¹ and R² are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, the Ar¹ and Ar² are the same or different and independently a substituted or unsubstituted C6 to C30 aryl group, or a substituted or unsubstituted C2 to C30 heteroaryl group, L is a single bond, a substituted or unsubstituted C2 to C6 alkenylene group, a substituted or unsubstituted C2 to C6 alkynylene group, a substituted or unsubstituted C6 to C30 arylene group, a substituted or unsubstituted heteroarylene group, or a combination thereof, and n is 0 or 1.

Y may be CR'R", R', R " are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

Y may be NR', wherein R' is hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

The compound for an organic optoelectronic device may be a compound represented by one of the following Chemical Formulas A-1 to A-51.
[Chemical Formula A-1] [Chemical Formula A-2]
[Chemical Formula A-3] [Chemical Formula A-4] 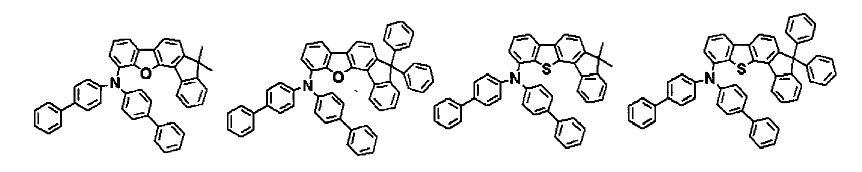
[Chemical Formula A-5] [Chemical Formula A-6]
[Chemical Formula A-7] [Chemical Formula A-8] 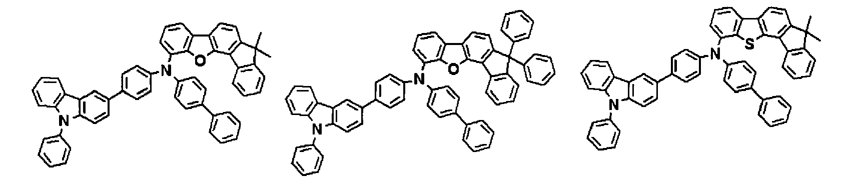
[Chemical Formula A-9] [Chemical Formula A-10] 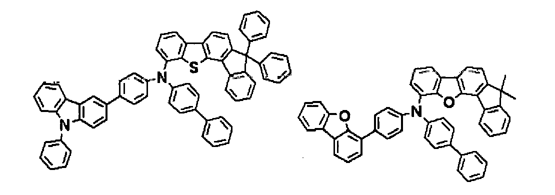
[Chemical Formula A-11] [Chemical Formula A-12]
[Chemical Formula A-13] 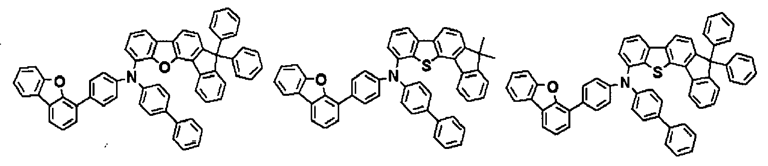
[Chemical Formula A-14] [Chemical Formula A-15]
[Chemical Formula A-16] 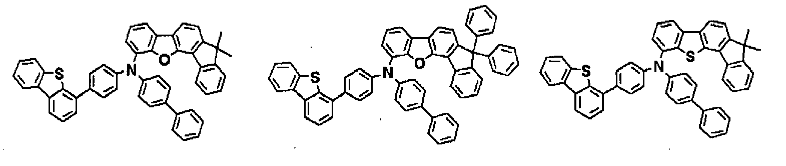
[Chemical Formula A-17] [Chemical Formula A-18] 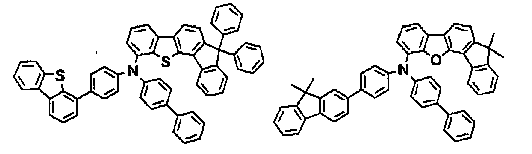
[Chemical Formula A-19] [Chemical Formula A-20]
[Chemical Formula A-21] 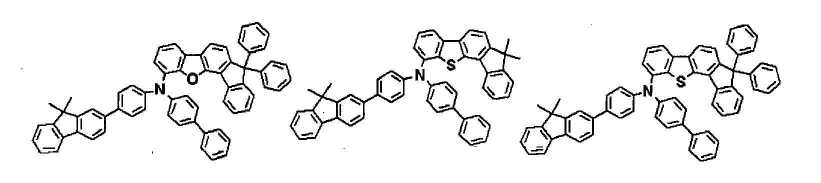
[Chemical Formula A-22] [Chemical Formula A-23] 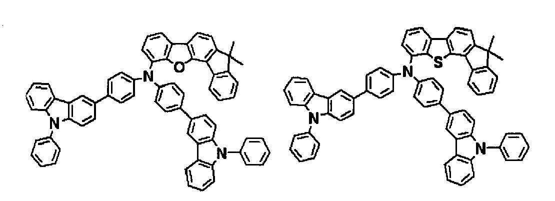
[Chemical Formula A-24] [Chemical Formula A-25]
[Chemical Formula A-26] [Chemical Formula A-27] 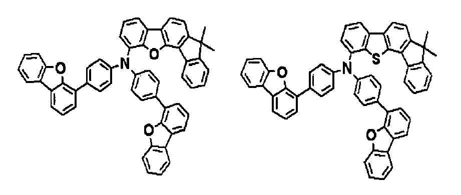
[Chemical Formula A-28] [Chemical Formula A-29]
[Chemical Formula A-30] [Chemical Formula A-31] 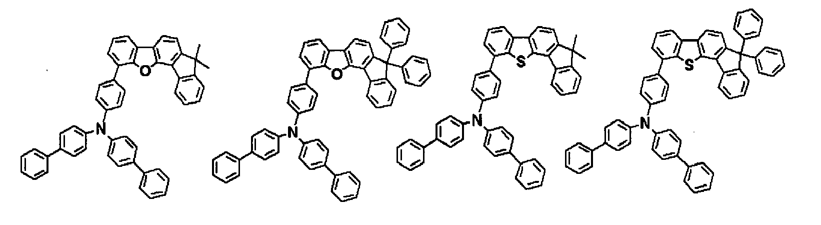
[Chemical Formula A-32] [Chemical Formula A-33]
[Chemical Formula A-34] [Chemical Formula A-35] 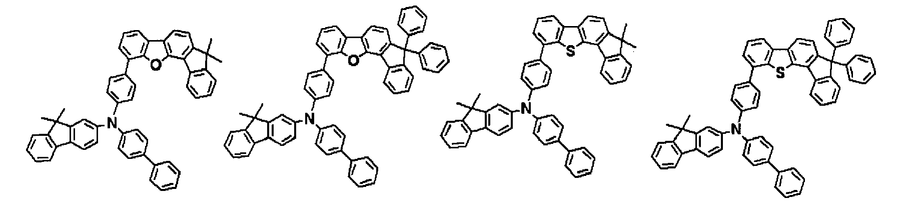
[Chemical Formula A-36] [Chemical Formula A-37]
[Chemical Formula A-38] [Chemical Formula A-39] 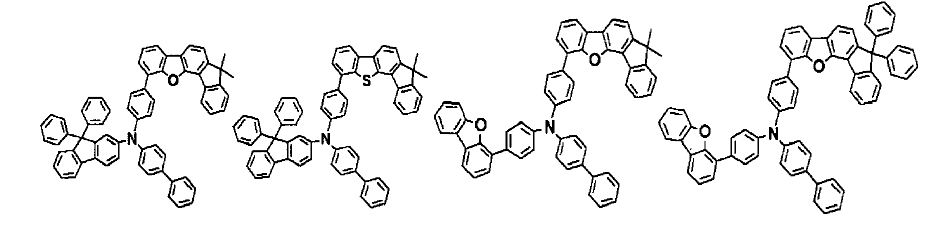
[Chemical Formula A-40] [Chemical Formula A-41] 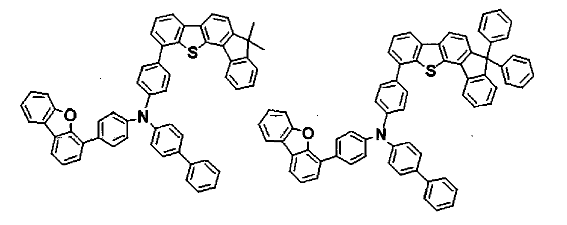
[Chemical Formula A-42] [Chemical Formula A-43] [Chemical Formula A-44] [Chemical Formula A-45] 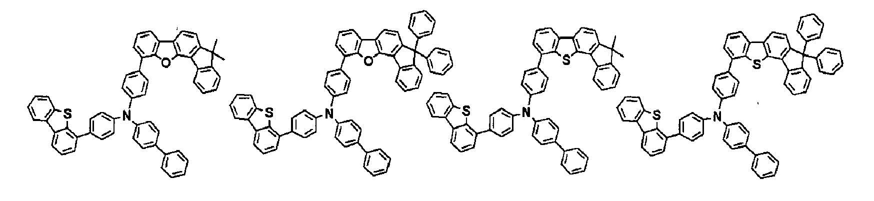
[Chemical Formula A-46] [Chemical Formula A-47]
[Chemical Formula A-48] [Chemical Formula A-49] 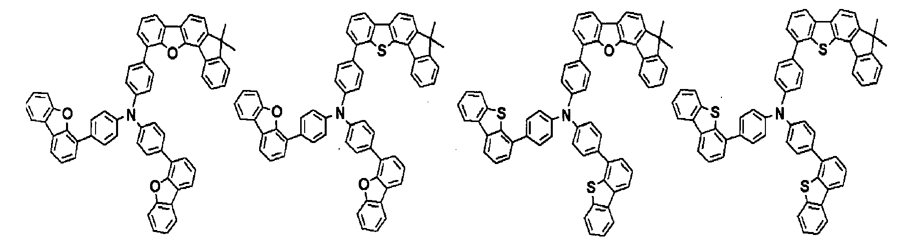
[Chemical Formula A-50] [Chemical Formula A-51]

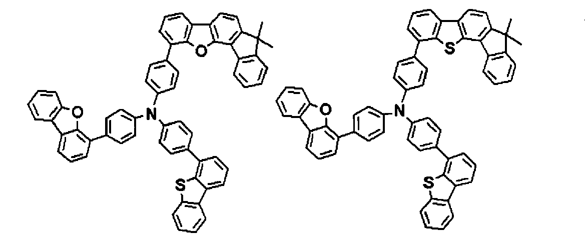

The compound for an organic optoelectronic device may be a compound represented by one of the following Chemical Formulas B-1 to B-32.
[Chemical Formula B-1] [Chemical Formula B-2]
[Chemical Formula B-3] [Chemical Formula B-4] 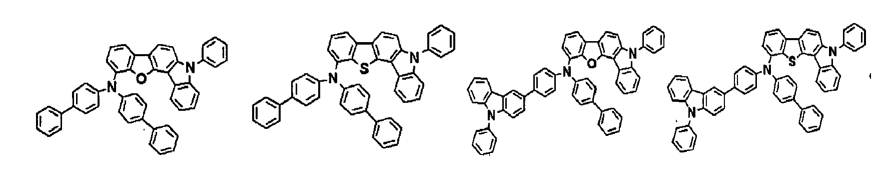
[Chemical Formula B-5] [Chemical Formula B-6]
[Chemical Formula B-7] [Chemical Formula B-8] 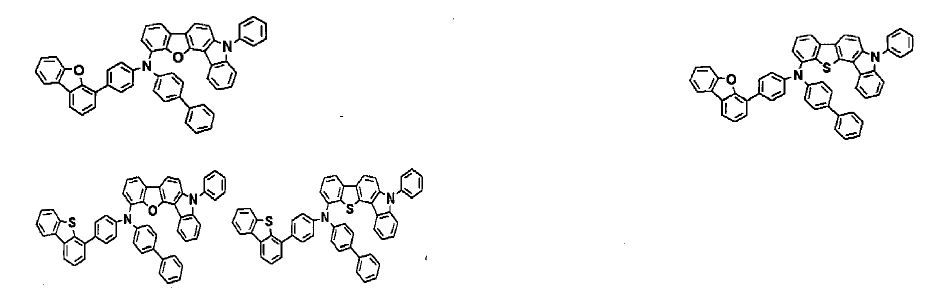
[Chemical Formula B-9] [Chemical Formula B-10]
[Chemical Formula B-11] 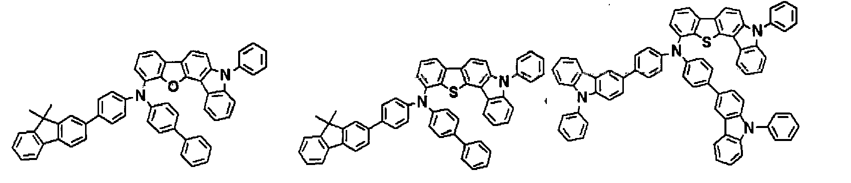
[Chemical Formula B-12] [Chemical Formula B-13]
[Chemical Formula B-14] 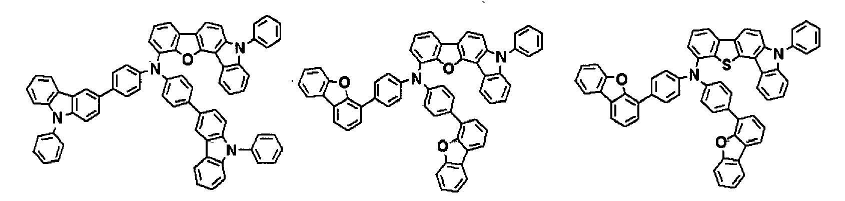
[Chemical Formula B-15] [Chemical Formula B-16]
[Chemical Formula B-17] [Chemical Formula B-18] 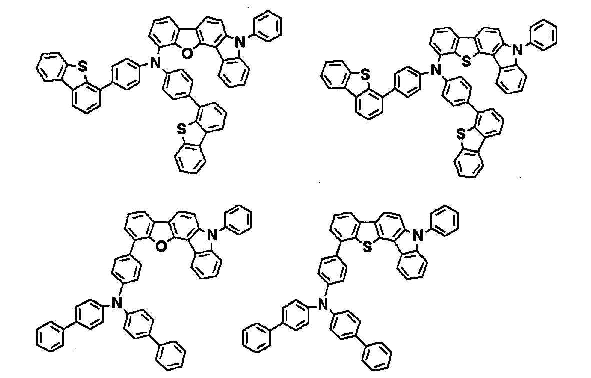
[Chemical Formula B-19] [Chemical Formula B-20]
[Chemical Formula B-21] [Chemical Formula B-22] 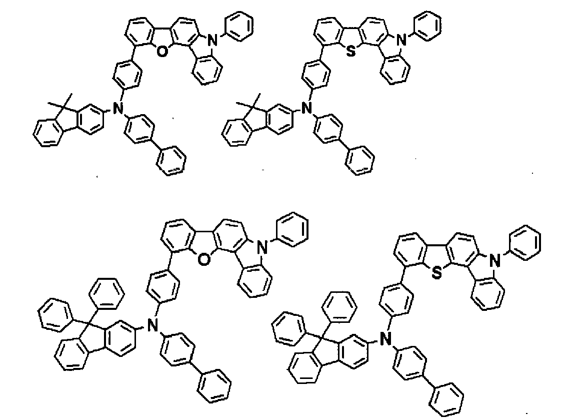
[Chemical Formula B-23] [Chemical Formula B-24]
[Chemical Formula B-25] [Chemical Formula B-26] 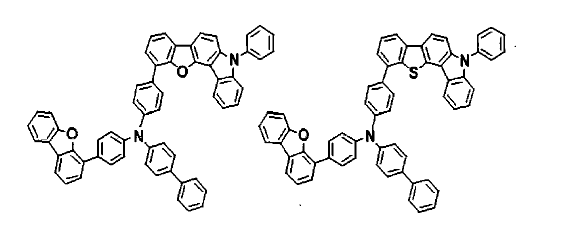 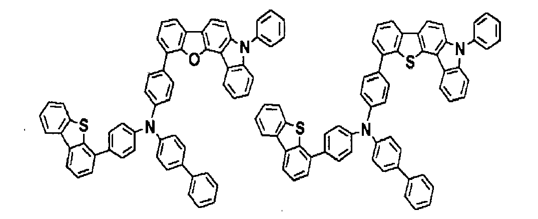
[Chemical Formula B-27] [Chemical Formula B-28]
[Chemical Formula B-29] [Chemical Formula B-30] 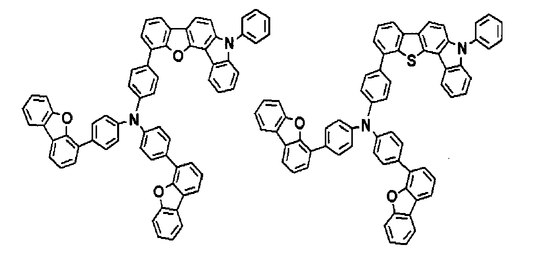 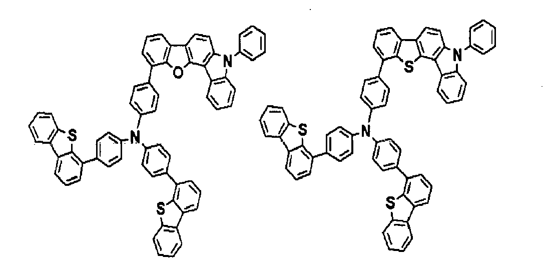
[Chemical Formula B-31] [Chemical Formula B-32]

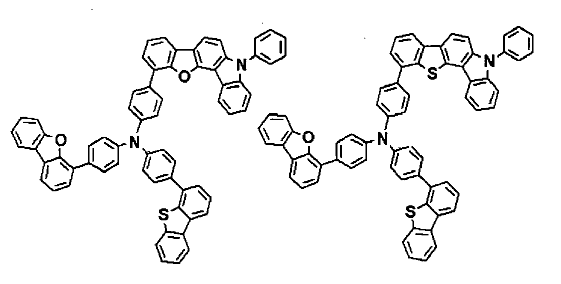

The compound for an organic optoelectronic device may be a compound represented by one of the following Chemical Formulas C-1 to C-41.
[Chemical Formula C-1] [Chemical Formula C-2] 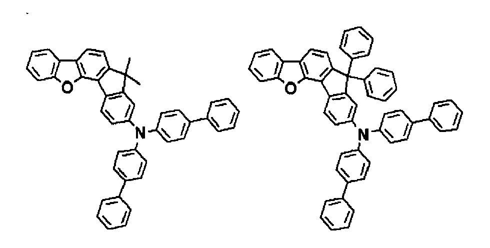
[Chemical Formula C-3] [Chemical Formula C-4]
[Chemical Formula C-5] [Chemical Formula C-6]
[Chemical Formula C-7] 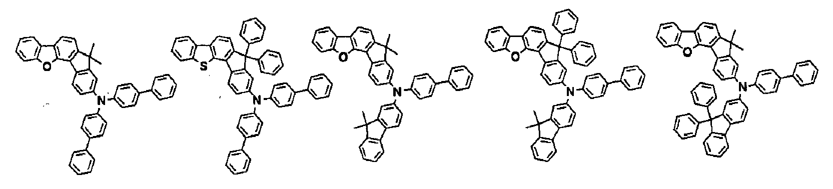
[Chemical Formula C-8] [Chemical Formula C-9]
[Chemical Formula C-10] 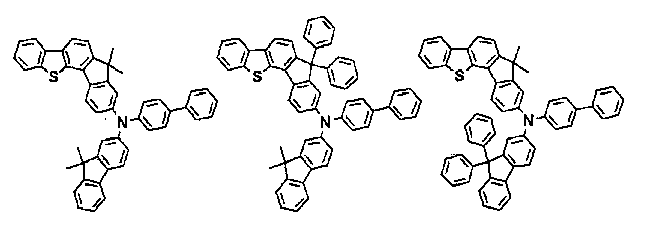
[Chemical Formula C-11] [Chemical Formula C-12]
[Chemical Formula C-13] [Chemical Formula C-14] 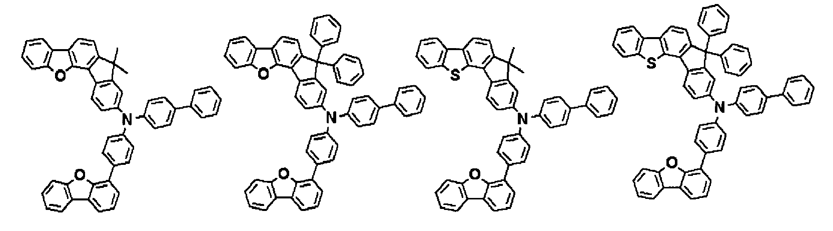
[Chemical Formula C-15] [Chemical Formula C-16]
[Chemical Formula C-17] [Chemical Formula C-18] 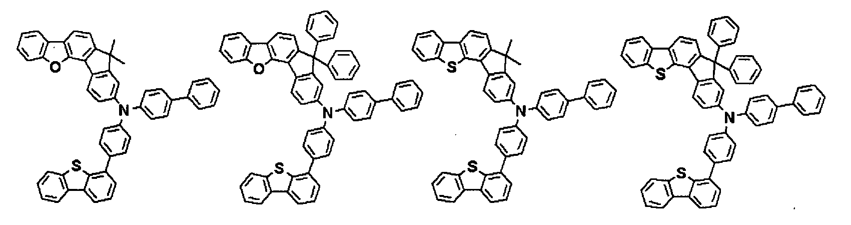
[Chemical Formula C-19] [Chemical Formula C-20]
[Chemical Formula C-21] [Chemical Formula C-22] 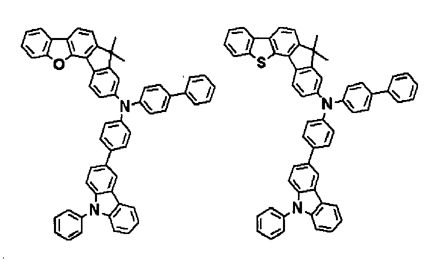 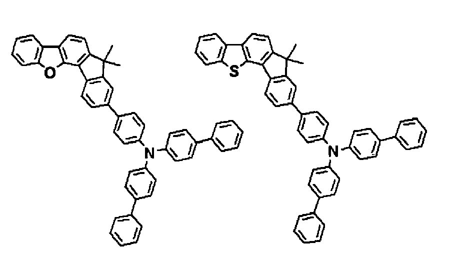
[Chemical Formula C-23] [Chemical Formula C-24]
[Chemical Formula C-25] [Chemical Formula C-26] 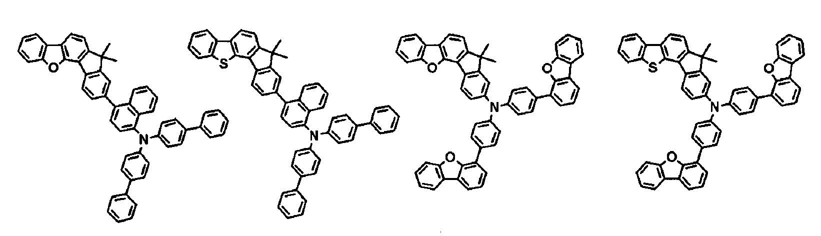
[Chemical Formula C-27] [Chemical Formula C-28]
[Chemical Formula C-29] [Chemical Formula C-30] 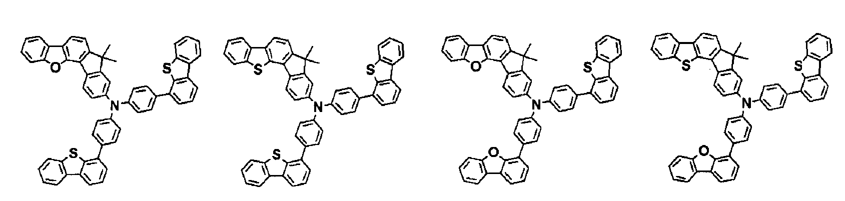
[Chemical Formula C-31] [Chemical Formula C-32]
[Chemical Formula C-33] 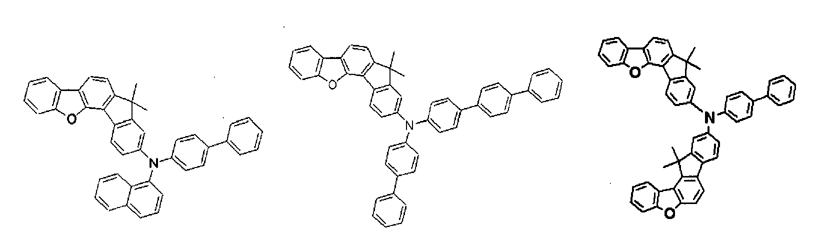
[Chemical Formula C-34] [Chemical Formula C-35]
[Chemical Formula C-36] 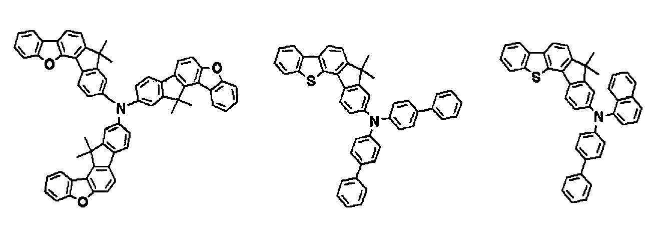
[Chemical Formula C-37] [Chemical Formula C-38]
[Chemical Formula C-39] 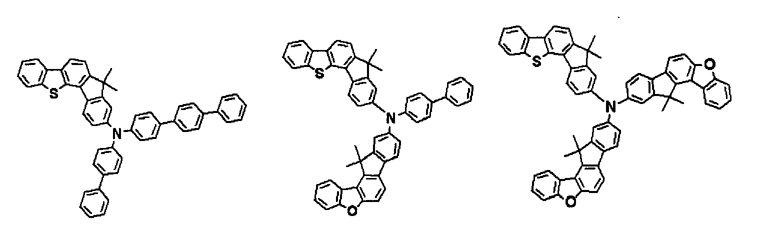
[Chemical Formula C-40] [Chemical Formula C-41]

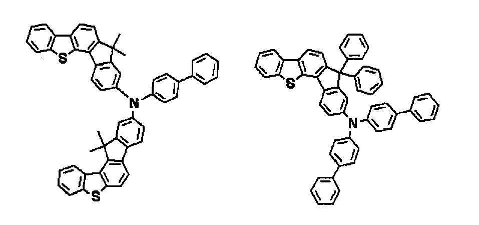

The compound for an organic optoelectronic device may be a compound represented by one of the following Chemical Formulas D-1 to D-20.
[Chemical Formula D-1] [Chemical Formula D-2]
[Chemical Formula D-3] [Chemical Formula D-4] 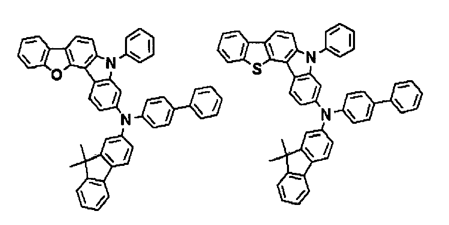 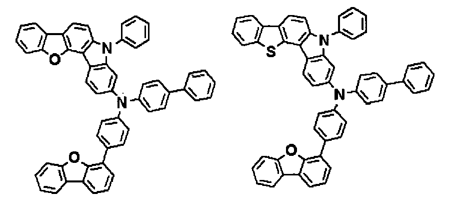
[Chemical Formula D-5] [Chemical Formula D-6]
[Chemical Formula D-7] 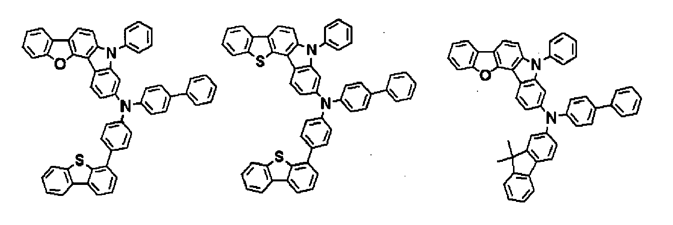
[Chemical Formula D-8] [Chemical Formula D-9] [Chemical Formula D-10] [Chemical Formula D-11] [Chemical Formula D-12] 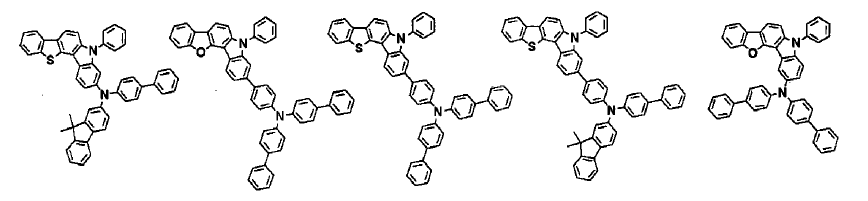
[Chemical Formula D-13] [Chemical Formula D-14]
[Chemical Formula D-15] [Chemical Formula D-16]
[Chemical Formula D-17] 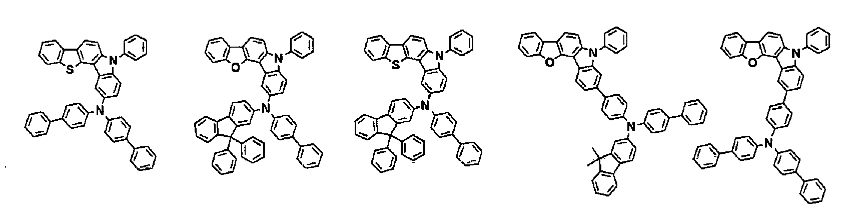
[Chemical Formula D-18] [Chemical Formula D-19]
[Chemical Formula D-20]

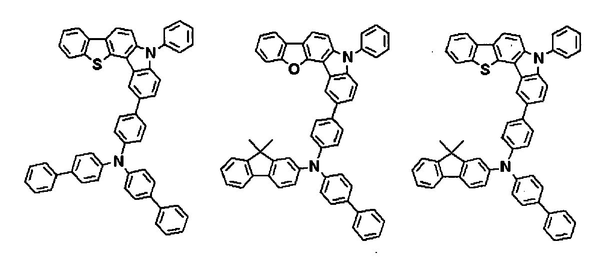

The compound for an organic optoelectronic device may be used as a hole transport material or hole injection material of an organic light emitting diode.

The compound for an organic optoelectronic device may have triplet excitation energy (T1) of 2.0eV or more.

The organic optoelectronic device may be selected from an organic photoelectronic device, an organic light emitting diode, an organic solar cell, an organic transistor, an organic photosensitive drum, and organic memory device.

According to another embodiment of the present invention, organic light emitting diode includes an anode, a cathode, and at least one organic thin layer interposed between the anode and the cathode, wherein at least one organic thin layer may provide an organic photoelectric device including the compound for an organic optoelectronic device according to one embodiment.

The compound for an organic optoelectronic device may be included in one of a hole transport layer (HTL), a hole injection layer (HIL), an electron transport layer (ETL), or an electron injection layer (EIL).

The compound for an organic optoelectronic device may be included in an em ission layer.

The compound for an organic optoelectronic device may be included in an emission layer as a phosphorescent or fluorescent host material.

The compound for an organic optoelectronic device may be included in an emission layer as a fluorescent blue dopant material.

According to yet another embodiment of the present invention, a display including the organic light emitting diode is provided.

The compound has high hole or electron transporting properties, film stability, thermal stability and high triplet excitation energy.

The compound may be used in an emission layer as a hole injection/transport material, a host material, or an electron injection/transport material. The organic photoelectric device including the compound excellent life-span, and luminous efficiency while having low driving voltages due to excellent electrochemical and thermal stability.

### BRIEF DESCRIPTION OF THE DRAWINGS

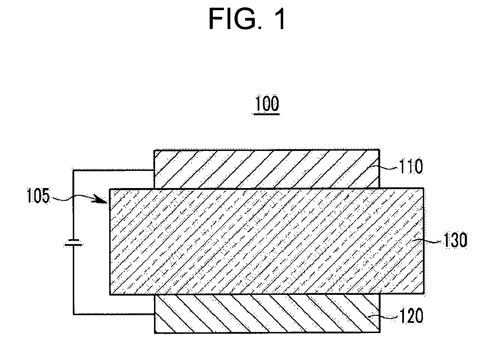

FIGS. 1 to 5 are cross-sectional views showing organic light emitting diodes including compounds according to various embodiments of the present invention.

### DETAILED DESCRIPTION

Exemplary embodiments of the present invention will hereinafter be described in detail. However, these embodiments are only exemplary, and the present invention is not limited thereto.

As used herein, when a definition is not otherwise provided, the term "substituted" may refer to one substituted with deuterium, a halogen, a hydroxy group, an amino group, a substituted or unsubstituted C1 to C20 amine group, nitro group, a substituted or unsubstituted C3 to C40 silyl group, a C1 to C30 alkyl group, a C1 to C10 alkylsilyl group, a C3 to C30 cycloalkyl group, a C6 to C30 aryl group, a C1 to C20 alkoxy group, a fluoro group, a C1 to C10 trifluoroalkyl group such as a trifluoromethyl group, or a cyano group, instead of at least hydrogen of substituents or compounds.

As used herein, when specific definition is not otherwise provided, the prefix "hetero" may refer to one including 1 to 3 of N, O, S, or P, and remaining carbons in one ring.

As used herein, when a definition is not otherwise provided, the term "combination thereof" may refer to at least two substituents bound to each other by a linker, or at least two substituents condensed to each other.

As used herein, when a definition is not otherwise provided, the term "alkyl group" may refer to an aliphatic hydrocarbon group. The alkyl group may be a saturated alkyl group that does not include any alkene group or alkyne group. Alternatively, the alkyl may be an unsaturated alkyl group that includes at least one alkene group or alkyne group. The term "alkene" group may refer to a group in which at least two carbon atoms are bound in at least one carbon-carbon double bond, and the term "alkyne" group may refer to a group in which at least two carbon atoms are bound in at least one carbon-carbon triple bond. Regardless of being saturated or unsaturated, the alkyl may be branched, linear, or cyclic.

The alkyl group may have 1 to 20 carbon atoms. The alkyl group may be a medium-sized alkyl having 1 to 10 carbon atoms. The alkyl group may be a lower alkyl having 1 to 6 carbon atoms.

For example, a C1-C4 alkyl may have 1 to 4 carbon atoms and may be selected from the group consisting of methyl, ethyl, propyl, iso-propyl, n-butyl, iso-butyl, sec-butyl, and t-butyl.

Examples of an alkyl group may be selected from a methyl group, an ethyl group, a propyl group, an isopropyl group, a butyl group, an isobutyl group, a t-butyl group, a pentyl group, a hexyl group, an ethenyl group, a propenyl group, a butenyl group, a cyclopropyl group, a cyclobutyl group, a cyclopentyl group, a cyclohexyl group, which may be individually and independently substituted.

The "aromatic group" may refer to a cyclic functional group where all elements have conjugated p-orbital. Examples of the aromatic group include an aryl group and a heteroaryl group.

The term "aryl" group may refer to an aryl group including a carbocyclic aryl (e.g., phenyl) having at least one ring having a covalent pi electron system.

The term "heteroaryl group" may refer to an aryl group where 1 to 3 heteroatoms selected from N, O, S, and P, and remaining carbon. When the heteroaryl group is a fused ring, each ring may include 1 to 3 heteroatoms.

In the specification, a carbazole-based derivative indicates a substituted or unsubstituted carbazolyl group in which nitrogen is substituted with a hetero atom.

According to one embodiment of the present invention, a compound for an organic optoelectronic device has a core structure that an aryl amine group is linked to a fused ring including at least one hetero atom.

The core structure includes a hetero fused ring and an amine group having excellent hole properties and thus, may be used as a hole injection material or a hole transport material for an organic light emitting diode.

In addition, the fused ring may decrease symmetry property inside a molecule and thus, deteriorate crystalline of a compound and suppress its recrystallization in a device.

At least one substituent linked to the core may have excellent electron properties. Accordingly, the compound is reinforced with electron properties in addition to excellent hole properties and thus, may satisfy a condition required of an emission layer. In particular, the compound may be used as a host material for an emission layer.

Furthermore, the compound for an organic optoelectronic device has a core part and various substituents introduced into a substituent in the core and thus, various energy bandgaps.

When the compound having an appropriate energy level depending on a substituent is used for an organic photoelectric device, it may strengthen hole or electron transport capabilities and accomplish excellent efficiency and driving voltage and excellent electrochemical and thermal stability, resultantly improving life-span characteristics of the organic photoelectric device. According to one embodiment of the present invention, a compound, according to claim 1, for an organic optoelectronic device is provided.

With the above, a compound for an organic optoelectronic device may be prepared to have hole or electron properties, film stability, thermal stability, and high triplet excitation energy (T1).

More specifically, the compound for an organic optoelectronic device may be a compound represented by the following Chemical Formula 6 or 7.

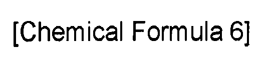

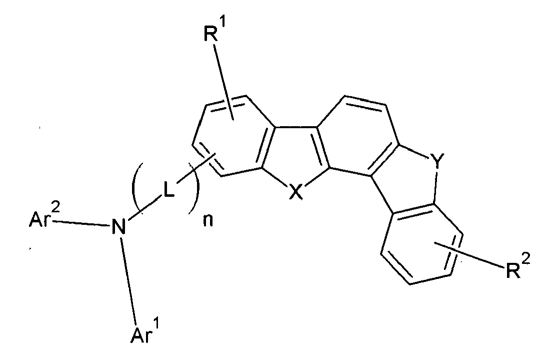

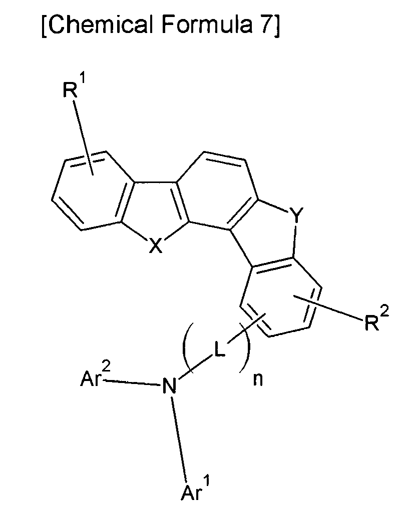

In Chemical Formulas 6 and 7, X is O, S, SO₂ (O=S=O), PO (P=O) or CO (C=O), Y is CR'R " or NR', R', R " , R¹ and R² are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof, Ar¹ and Ar² are the same or different and independently a substituted or unsubstituted C6 to C30 aryl group, or a substituted or unsubstituted C2 to C30 heteroaryl group, L is a single bond, a substituted or unsubstituted C2 to C6 alkenylene group, a substituted or unsubstituted C2 to C6 alkynylene group, a substituted or unsubstituted C6 to C30 arylene group, a substituted or unsubstituted heteroarylene group, or a combination thereof, and n is 0 or 1.

When an aryl amine group is combined as shown in the above Chemical Formula 6, a compound may have characteristic of a material including the X. When an aryl amine group is combined as shown in the above Chemical Formula 7, a compound may have characteristic of a material including the Y.

The substituents may be appropriately combined to prepare a compound with an unsymmetrical bipolar structure. The unsymmetrical bipolar structure may improve hole and electron transport capabilities and thus, luminous efficiency and performance of a device.

In addition, the substituents may be regulated to prepare a compound with a bulky structure and thus, lower crystallinity. The compound with lower crystalline may improve life-span of a device.

Y may be CR'R " , R', R " are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

When Y is carbon as aforementioned, a compound may have fluorene characteristics.

Y may be NR', wherein R' is hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

When Y is nitrogen as shown above, a com pound may have carbazole characteristic.

Examples of Ar¹ and Ar² are as follows: a substituted or unsubstituted phenyl group, a substituted or unsubstituted carbazolyl group, a substituted or unsubstituted dibenzofuranyl, a substituted or unsubstituted dibenzothiophenyl group, a substituted or unsubstituted fluorenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenylyl group, a substituted or unsubstituted p-terphenyl group, a substituted or unsubstituted m-terphenyl group, a substituted or unsubstituted chrysenyl group, a substituted or unsubstituted triperylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quninolinyl group, a substituted or unsubstituted isoquninolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, and the like.

The Ar¹ and Ar² may be controlled regarding length to regulate an entirerr-conjugation length and thus, a triplet energy bandgap of a compound. Accordingly, the compound may be used as a phosphorescent host and usefully applied to the emission layer of an organic photoelectric device. In addition, when a heteroaryl group is introduced into a compound, the compound may have bipolar characteristic in its molecule structure. The compound as a phosphorescence host may bring about high efficiency of a device.

When a substituent for the Ar¹ and Ar² is a substituted or unsubstituted carbazolyl group, a substituted or unsubstituted dibenzofuranyl, and a substituted or unsubstituted dibenzothiophenyl group, a compound may not be easily recrystallized due to asymmetry of entire molecules as well as excellent hole transport properties of a carbazolyl-based derivative. Accordingly, when the compound is applied to a hole injection and a hole transport layer (HTL) for an organic electric field light emitting device, the organic electric field light emitting device may have long life-span and high efficiency.

In addition, a substituted or unsubstituted fluorenyl group is used in a compound and may increase planarity of molecules and thus increase mobility of holes. Accordingly, when it is applied to a hole injection layer and a hole transport layer (HTL) for an organic light emitting diode, the organic light emitting diode may have long life-span and high efficiency.

The compound for an organic optoelectronic device may be a compound represented by one of the following Chemical Formulas A-1 to A-51. In a compound with the following structure, since dibenzofuran or dibenzothiophene is combined with fullerene in one molecule, the compound may have main characteristic of dibenzofuran or dibenzothiophene and auxiliary characteristic of fullerene.
[Chemical Formula A-1] [Chemical Formula A-2]
[Chemical Formula A-3] [Chemical Formula A-4] 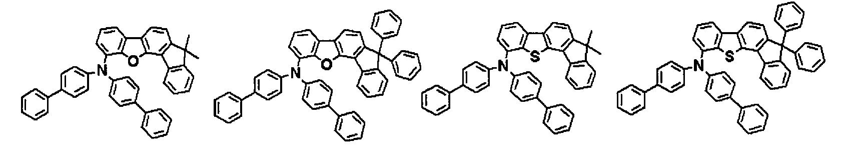
[Chemical Formula A-5] [Chemical Formula A-6]
[Chemical Formula A-7] [Chemical Formula A-8] 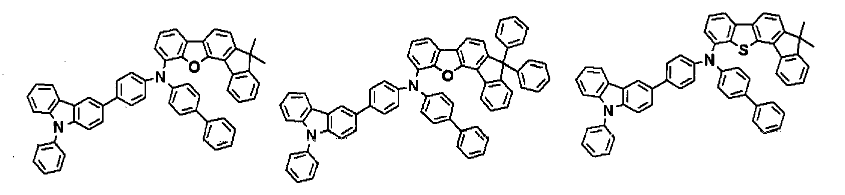
[Chemical Formula A-9] [Chemical Formula A-10] 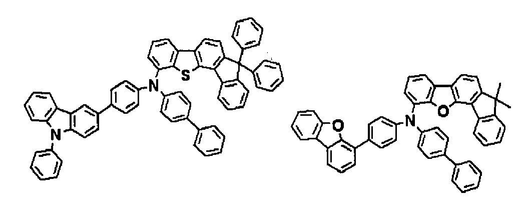
[Chemical Formula A-11] [Chemical Formula A-12]
[Chemical Formula A-13] 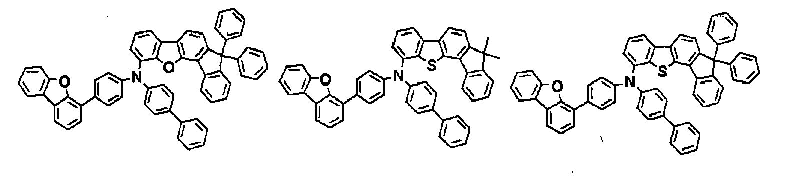
[Chemical Formula A-14] [Chemical Formula A-15]
[Chemical Formula A-16] 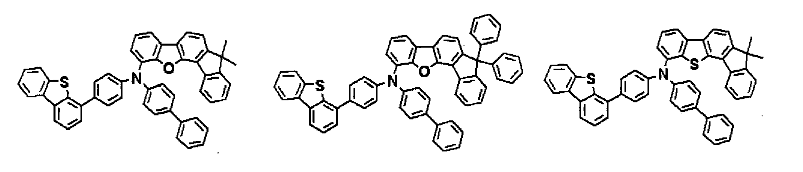
[Chemical Formula A-17] [Chemical Formula A-18] 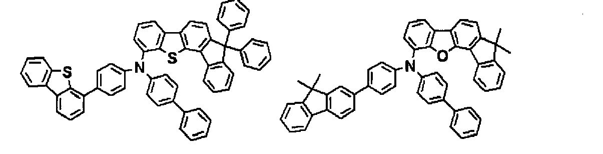
[Chemical Formula A-19] [Chemical Formula A-20]
[Chemical Formula A-21] 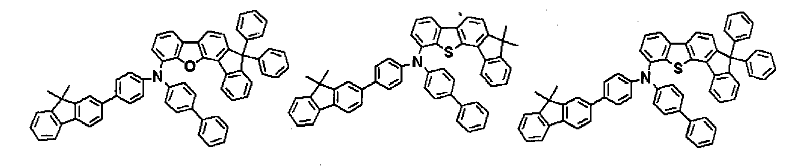
[Chemical Formula A-22] [Chemical Formula A-23] 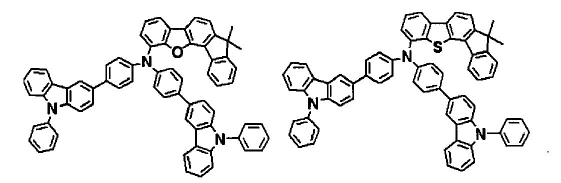
[Chemical Formula A-24] [Chemical Formula A-25]
[Chemical Formula A-26] [Chemical Formula A-27] 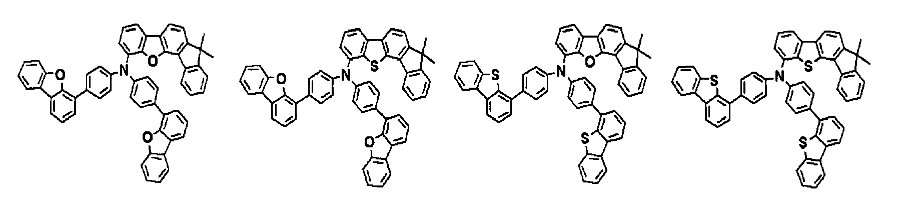
[Chemical Formula A-28] [Chemical Formula A-29]
[Chemical Formula A-30] [Chemical Formula A-31] 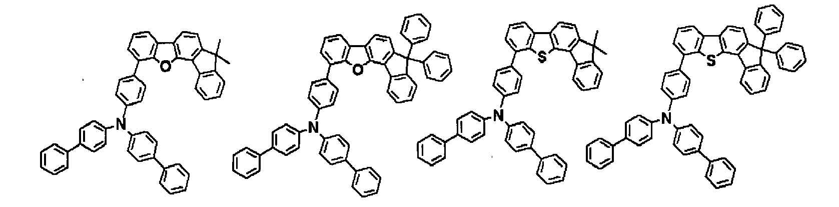
[Chemical Formula A-32] [Chemical Formula A-33]
[Chemical Formula A-34] [Chemical Formula A-35] 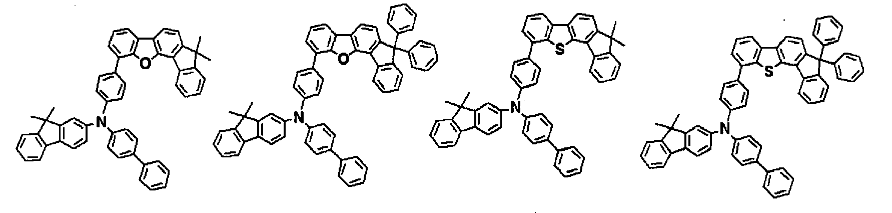
[Chemical Formula A-36] [Chemical Formula A-37]
[Chemical Formula A-38] [Chemical Formula A-39] 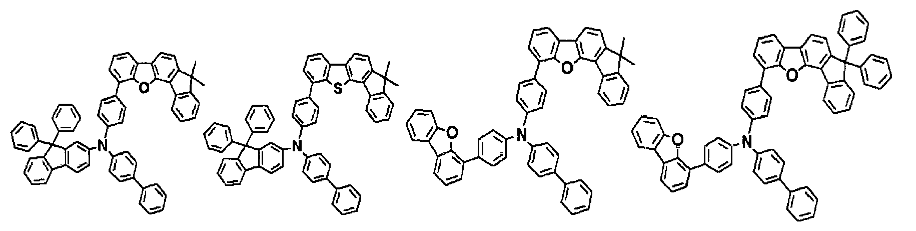
[Chemical Formula A-40] [Chemical Formula A-41] 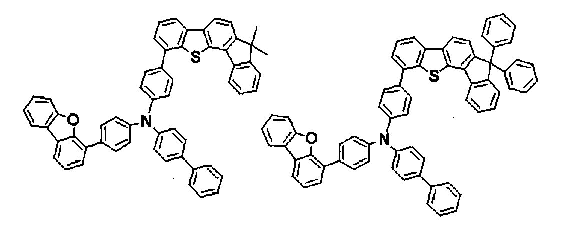
[Chemical Formula A-42] [Chemical Formula A-43]
[Chemical Formula A-44] [Chemical Formula A-45] 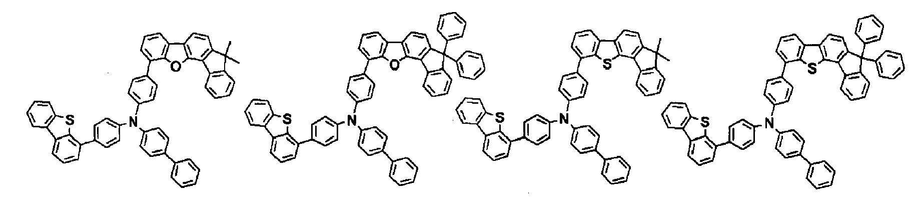
[Chemical Formula A-46] [Chemical Formula A-47]
[Chemical Formula A-48] [Chemical Formula A-49] 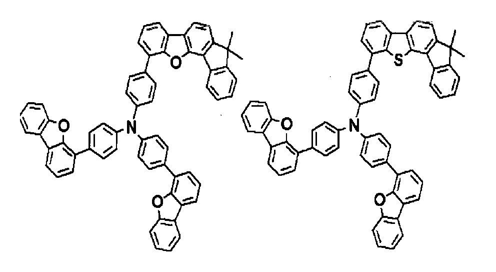 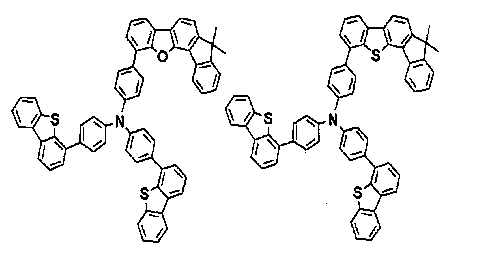
[Chemical Formula A-50] [Chemical Formula A-51]

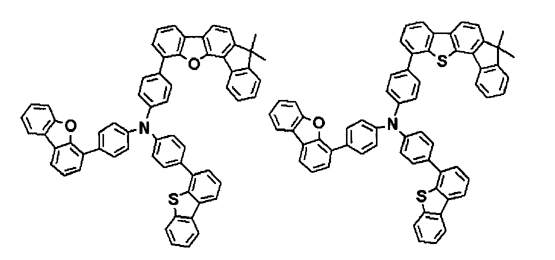

The compound for an organic optoelectronic device may be a compound represented by one of the following Chemical Formulas B-1 to B-32. In a compound with the following structure, since dibenzofuran or dibenzothiophene is combined with carbazole in one molecule, the compound may have main characteristic of dibenzofuran or dibenzothiophene and auxiliary characteristic of carbazole.
[Chemical Formula B-1] [Chemical Formula B-2]
[Chemical Formula B-3] [Chemical Formula B-4] 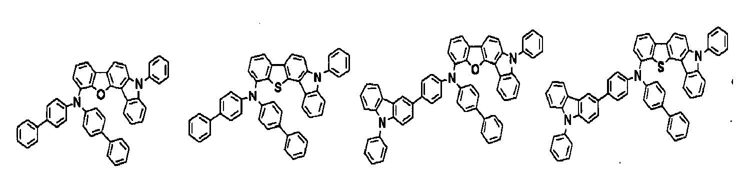
[Chemical Formula B-5] [Chemical Formula B-6]
[Chemical Formula B-7] [Chemical Formula B-8] 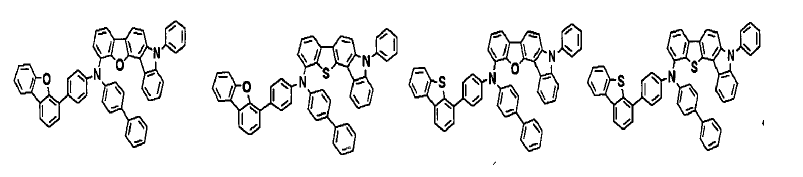
[Chemical Formula B-9] [Chemical Formula B-10]
[Chemical Formula B-11] 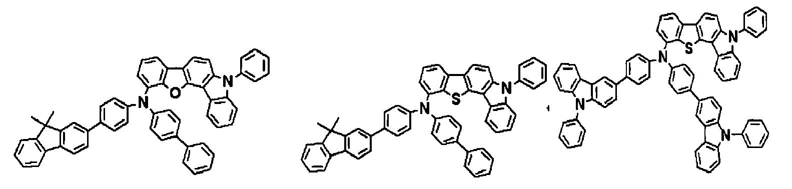
[Chemical Formula B-12] [Chemical Formula B-13]
[Chemical Formula B-14] 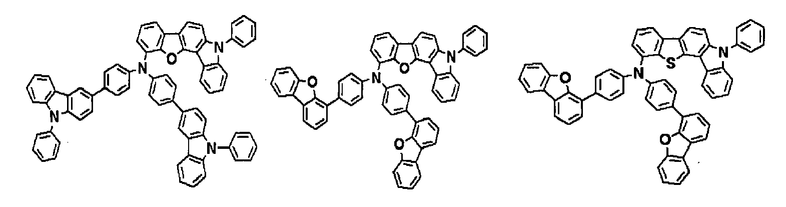
[Chemical Formula B-15] [Chemical Formula B-16]
[Chemical Formula B-17] [Chemical Formula B-18] 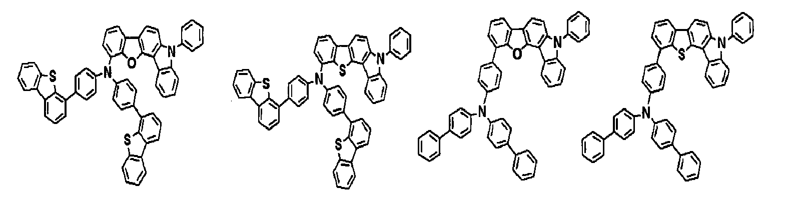
[Chemical Formula B-19] [Chemical Formula B-20]
[Chemical Formula B-21] [Chemical Formula B-22] 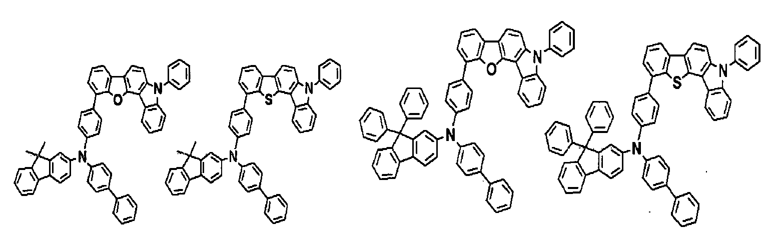
[Chemical Formula B-23] [Chemical Formula B-24]
[Chemical Formula B-25] [Chemical Formula B-26] 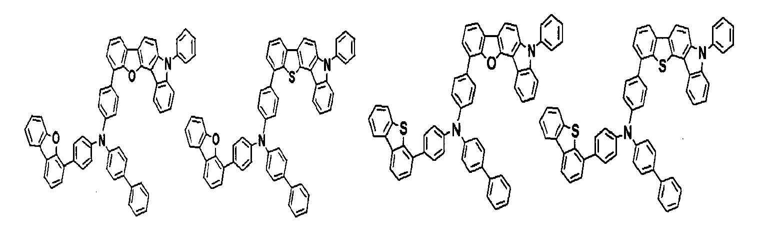
[Chemical Formula B-27] [Chemical Formula B-28]
[Chemical Formula B-29] [Chemical Formula B-30] 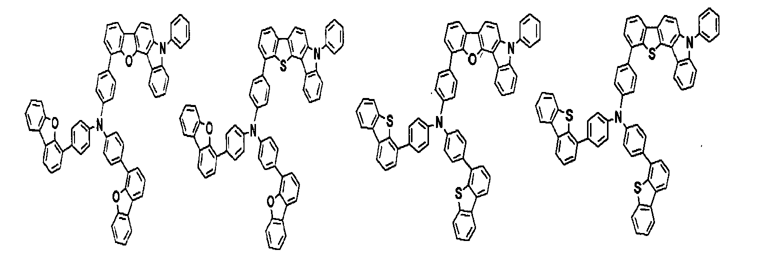
[Chemical Formula B-31] [Chemical Formula B-32]

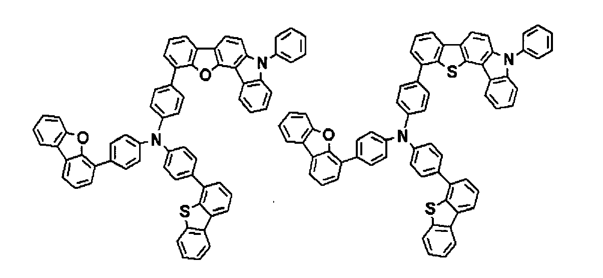

The compound for an organic optoelectronic device may be a compound represented by one of the following Chemical Formulas C-1 to C-41. In a compound with the following structure, since dibenzofuran or dibenzothiophene is combined with fullerene in one molecule, the compound may have main characteristic of dibenzofuran or dibenzothiophene and auxiliary characteristic of fullerene.
[Chemical Formula C-1] [Chemical Formula C-2] 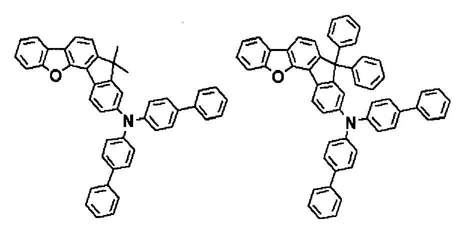
[Chemical Formula C-3][Chemical Formula C-4]
[Chemical Formula C-5][Chemical Formula C-6][Chemical Formula C-7] 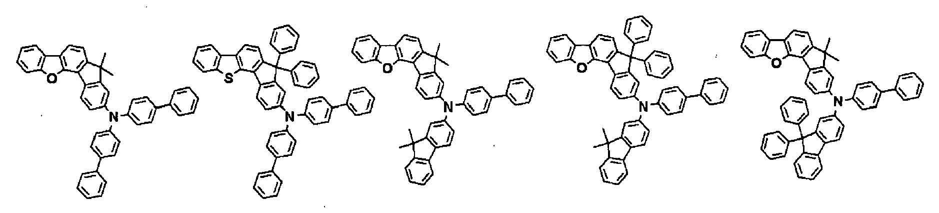
[Chemical Formula C-8] [Chemical Formula C-9]
[Chemical Formula C-10] 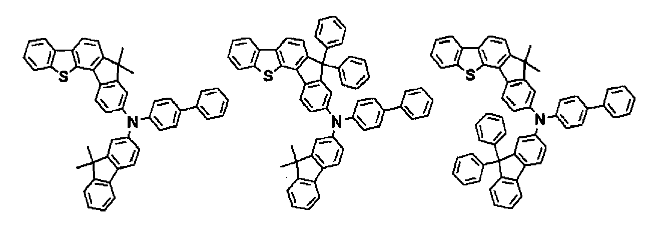
[Chemical Formula C-11] [Chemical Formula C-12]
[Chemical Formula C-13] [Chemical Formula C-14] 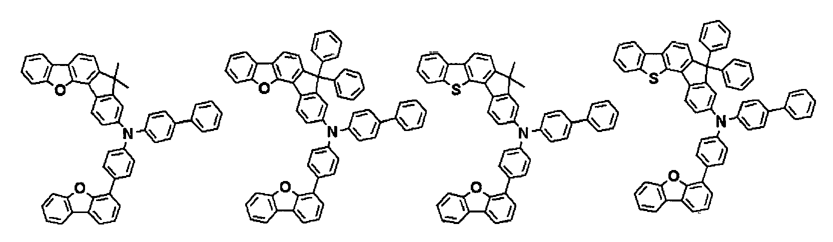
[Chemical Formula C-15] [Chemical Formula C-16]
[Chemical Formula C-17] [Chemical Formula C-18] 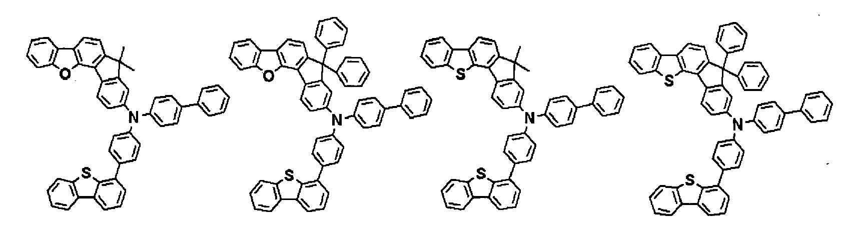
[Chemical Formula C-19] [Chemical Formula C-20]
[Chemical Formula C-21] [Chemical Formula C-22] 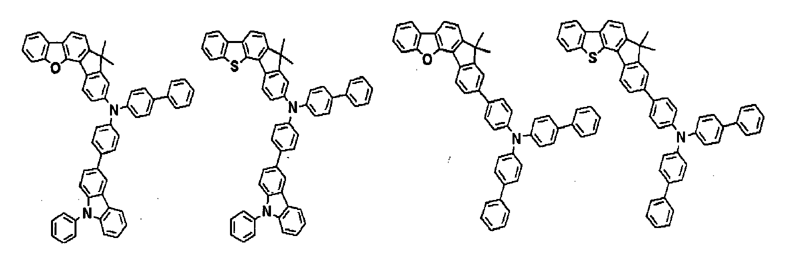
[Chemical Formula C-23] [Chemical Formula C-24]
[Chemical Formula C-25] [Chemical Formula C-26] 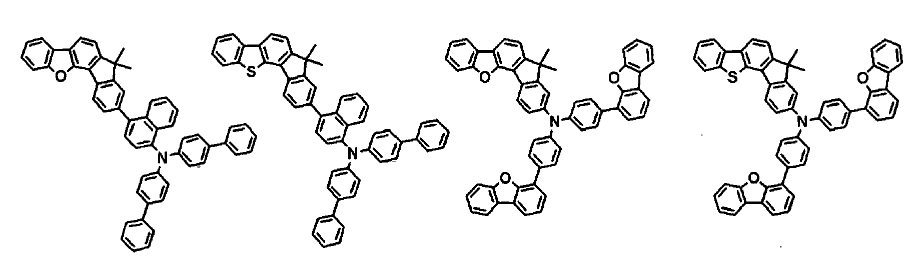
[Chemical Formula C-27] [Chemical Formula C-28]
[Chemical Formula C-29] [Chemical Formula C-30] 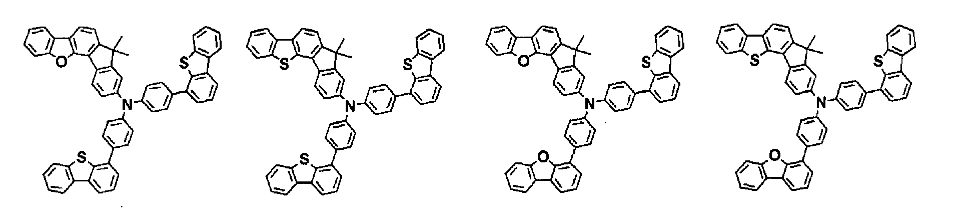
[Chemical Formula C-31] [Chemical Formula C-32]
[Chemical Formula C-33] 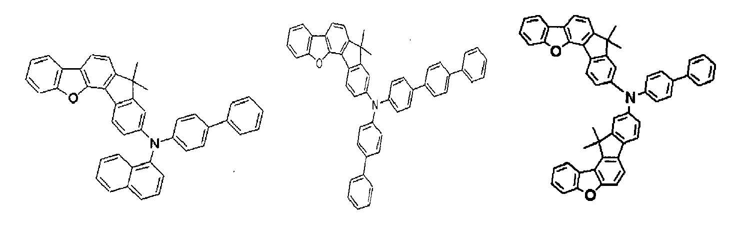
[Chemical Formula C-34] [Chemical Formula C-35]
[Chemical Formula C-36] 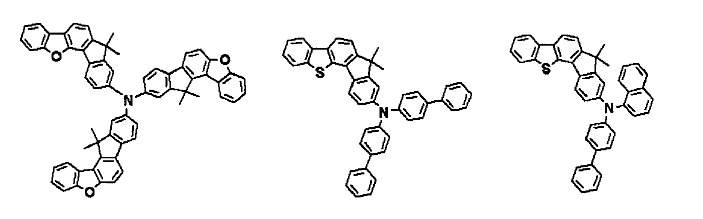
[Chemical Formula C-37] [Chemical Formula C-38]
[Chemical Formula C-39] 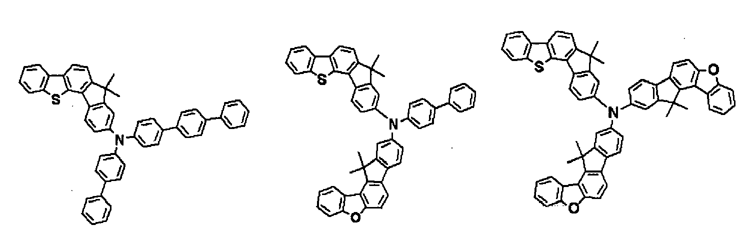
[Chemical Formula C-40] [Chemical Formula C-41]

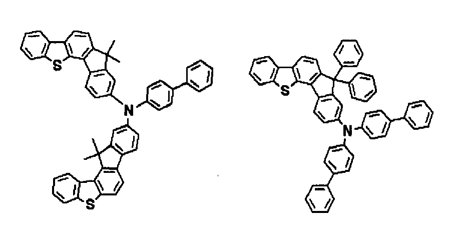

The compound for an organic optoelectronic device may be a compound represented by one of the following Chemical Formulas D-1 to D-20. In a compound with the following structure, since dibenzofuran or dibenzothiophene is combined with carbazole in one molecule, the compound may have main characteristic of carbazole and auxiliary characteristic of dibenzofuran or dibenzothiophene.
[Chemical Formula D-1] [Chemical Formula D-2]
[Chemical Formula D-3] [Chemical Formula D-4] 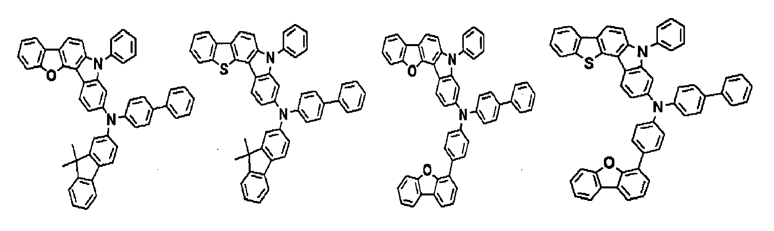
[Chemical Formula D-5] [Chemical Formula D-6]
[Chemical Formula D-7] 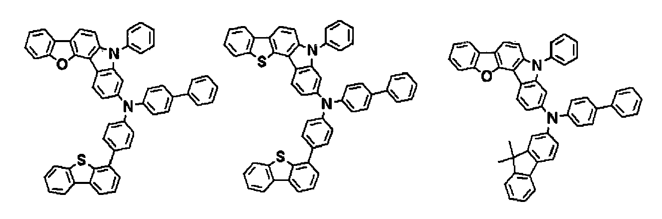
[Chemical Formula D-8] [Chemical Formula D-9]
[Chemical Formula D-10] [Chemical Formula D-11]
[Chemical Formula D-12] 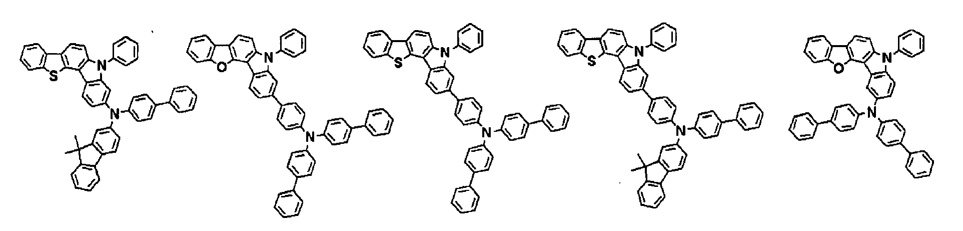
[Chemical Formula D-13] [Chemical Formula D-14]
[Chemical Formula D-15] [Chemical Formula D-16]
[Chemical Formula D-17] 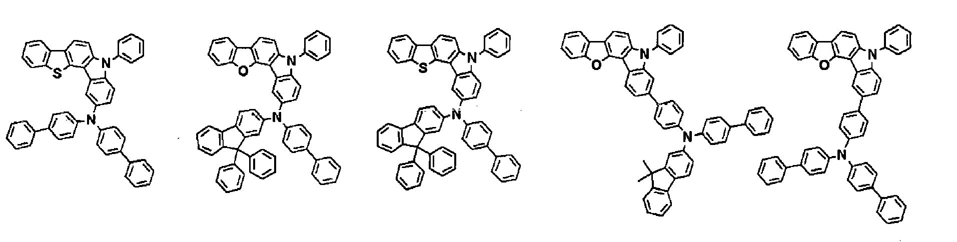
[Chemical Formula D-18] [Chemical Formula D-19]
[Chemical Formula D-20]

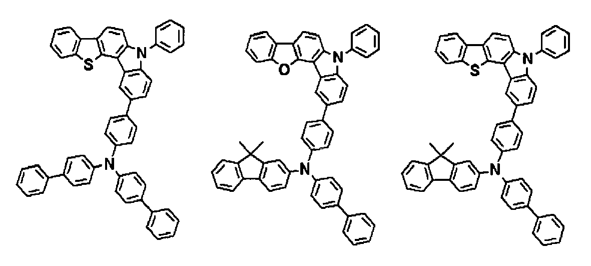

When a compound for an organic optoelectronic device according to the aforementioned embodiment of the present invention is used to prepare electron blocking layer (or a hole transport layer (HTL)), a functional group with electron properties in a molecule may tend to deteriorate electron blocking property. Accordingly, the compound should not include a functional group with electron properties in order to be used for an electron blocking layer. Examples of the functional group with electron properties may include benzoimidazole, pyridine, pyrazine, pyrimidine, triazine, quinoline, isoquinoline, and the like. However, the compound has this restriction only when applied to an electron-blocking film or a hole transport layer (HTL) (or a hole injection layer (HIL)).

When the compound according to one embodiment of the present invention is required of both electron properties and hole characteristic, the functional group with electron properties may be included in the compound, improving life-span of an organic optoelectronic device, such as an organic light emitting diode, and decreasing its driving voltage.

According to one embodiment of the present invention, the compound for an organic optoelectronic device may have maximum light emitting wavelength ranging from about 320 to 500 nm and triplet excitation energy (T1) of 2.0 eV or more, specifically, from 2.0 to 4.0 eV. Accordingly, the compound is used as a host material or a charge transport material, since charges of the host material with high triplet excitation energy are well transported to a dopant and may increase luminous efficiency of the dopant and since HOMO and LUMO energy levels of the host material may be freely adjusted.

In addition, the compound for an organic optoelectronic device has photoactive and electric activity and thus, may be used as an optic material, electrode material, discoloring material, photo switch, sensor, module, wave guide, organic transistor, laser, light-absorbing agent, dielectric material, membrane, and the like.

The compound for an organic optoelectronic device has a glass transition temperature of 90°C or higher, a thermal decomposition temperature of 400°C or higher and thus, excellent thermal stability. Accordingly, the compound may provide an organic optoelectronic device with high efficiency.

The compound for an organic optoelectronic device including the above compound may play a role in emitting light or injecting and/or transporting electrons, and it may act as a light emitting host together with a suitable dopant. In other words, the compound for an organic optoelectronic device may be used as a phosphorescent or fluorescent host material, a blue light emitting dopant material, or an electron transporting material.

Since the compound for an organic optoelectronic device according to one embodiment is used for an organic thin layer, it may improve the life span characteristic, efficiency characteristic, electrochemical stability, and thermal stability of an organic photoelectric device, and decrease the driving voltage.

Therefore, according to another embodiment, an organic optoelectronic device is provided that includes the compound for an organic optoelectronic device. The organic photoelectric device may include an organic light emitting diode, an organic solar cell, an organic transistor, an organic photosensitive drum, an organic memory device, or the like. For example, the compound for an organic optoelectronic device according to one embodiment may be included in an electrode or an electrode buffer layer in the organic solar cell to improve quantum efficiency, and it may be used as an electrode material for a gate, a source-drain electrode, or the like in the organic transistor.

Hereinafter, a detailed described relating to the organic light emitting diode will be provided.

The organic thin layer that may include the compound for an organic optoelectronic device may include a layer selected from the group consisting of an emission layer, a hole transport layer (HTL), a hole injection layer (HIL), an electron transport layer (ETL), an electron injection layer (EIL), a hole blocking film, and a combination thereof. The at least one layer includes the compound for an organic optoelectronic device according to one embodiment.

The organic thin layer that may include the compound for an organic optoelectronic device may include a layer selected from the group consisting of an emission layer, a hole transport layer (HTL), a hole injection layer (HIL), an electron transport layer (ETL), an electron injection layer (EIL), a hole blocking film, and a combination thereof. The at least one layer includes the compound for an organic optoelectronic device according to one embodiment. Particularly, the electron transport layer (ETL) or the electron injection layer (EIL) may include the compound for an organic optoelectronic device according to one embodiment. In addition, when the compound for an organic optoelectronic device is included in the emission layer, the compound for an organic optoelectronic device may be included as a phosphorescent or fluorescent host, and particularly, as a fluorescent blue dopant material.

FIGS. 1 to 5 are cross-sectional views showing an organic light emitting diode including the compound for an organic optoelectronic device according to one embodiment of the present invention.

Referring to FIGS. 1 to 5, organic light emitting diode 100, 200, 300, 400, and 500 according to one embodiment include at least one organic thin layer 105 interposed between an anode 120 and a cathode 110.

The anode 120 includes an anode material laving a large work function to help hole injection into an organic thin layer. The anode material includes: a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combined metal and oxide such as ZnO-Al or SnO₂:Sb; or a conductive polymer such as poly(3-methylthiophene), poly[3,4-(ethylene-1,2-dioxy)thiophene] (PEDT), polypyrrole, and polyaniline, but is not limited thereto. It is preferable to include a transparent electrode including indium tin oxide (ITO) as an anode.

The cathode 110 includes a cathode material having a small work function to help electron injection into an organic thin layer. The cathode material includes: a metal such as magnesium, calcium, sodium, potassium, titanium, indium, yttrium, lithium, gadolinium, aluminum, silver, tin, lead, cesium, barium, and the like, or or alloys thereof; or a multi-layered material such as LiF/Al, LiO₂/Al, LiF/Ca, LiF/Al, and BaF₂/Ca, but is not limited thereto. It is preferable to include a metal electrode including aluminum as a cathode.

Referring to FIG. 1, the organic light emitting diode 100 includes an organic thin layer 105 including only an emission layer 130.

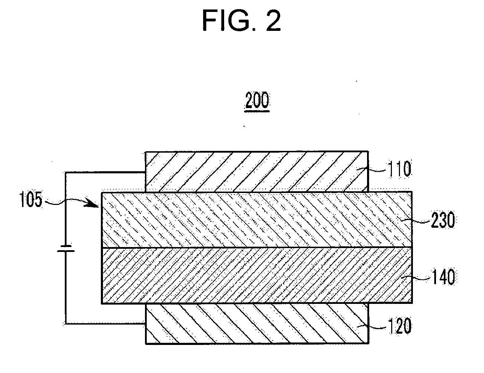

Referring to FIG. 2, the emission layer 130 also functions as an electron transport layer (ETL), and the hole transport layer (HTL) 140 layer has an excellent binding property with a transparent electrode such as ITO or an excellent hole transporting property. The emission layer 130 also functions as an electron transport layer (ETL), and the hole transport layer (HTL) 140 layer has an excellent binding property with a transparent electrode such as ITO or an excellent hole transporting property.

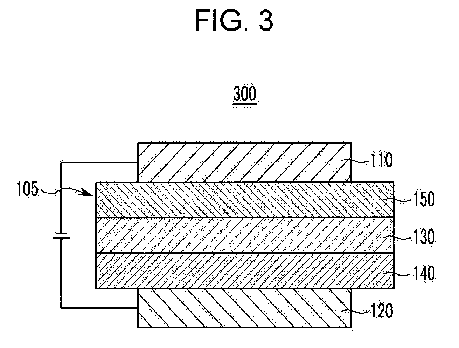

Referring to FIG. 3, a three-layered organic light emitting diode 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

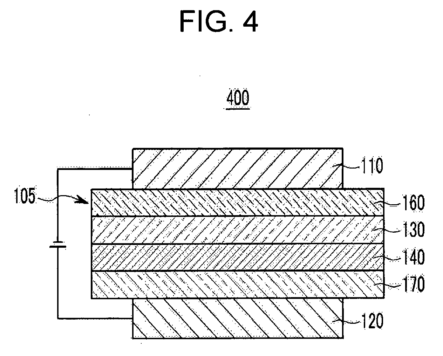

Referring to FIG. 4, a four-layered organic light emitting diode 400 includes an organic thin layer 105 including an electron injection layer (EIL) 160, an emission layer 130, a hole transport layer (HTL) 140, and a hole injection layer (HIL) 170 for binding with the cathode of ITO.

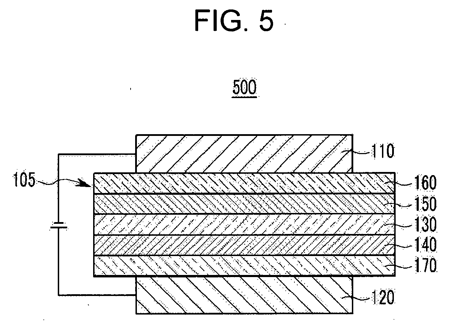

Referring to FIG. 5, a five-layered organic light emitting diode 500 includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

In FIG. 1 to FIG. 5, the organic thin layer 105 including at least one selected from the group consisting of an electron transport layer (ETL) 150, an electron injection layer (EIL) 160, an emission layer 130 and 230, a hole transport layer (HTL) 140, a hole injection layer (HIL) 170, and combinations thereof, includes a compound for an organic optoelectronic device. The material for the organic optoelectronic device may be used for an electron transport layer (ETL) 150 including the electron transport layer (ETL) 150 or electron injection layer (EIL) 160. When it is used for the electron transport layer (ETL), it is possible to provide an organic light emitting diode having a simpler structure because it does not require an additional hole blocking layer (not shown).

Furthermore, when the compound for an organic optoelectronic device is included in the emission layer 130 and 230, the material for the organic light emitting diode may be included as a phosphorescent or fluorescent host or a fluorescent blue dopant.

The organic light emitting diode may be fabricated by: forming an anode on a substrate; forming an organic thin layer in accordance with a dry coating method such as evaporation, sputtering, plasma plating, and ion plating, or a wet coating method such as spin coating, dipping, and flow coating; and providing a cathode thereon.

Another embodiment of the present invention provides a display including the organic light emitting diode according to the above embodiment.

Hereinafter, the embodiments are illustrated in more detail with reference to examples. However, the following are exemplary embodiments and are not limiting.

### (Preparation of Compound for Organic optoelectronic device)

### Synthesis of Intermediate

### Synthesis of Intermediate M-1

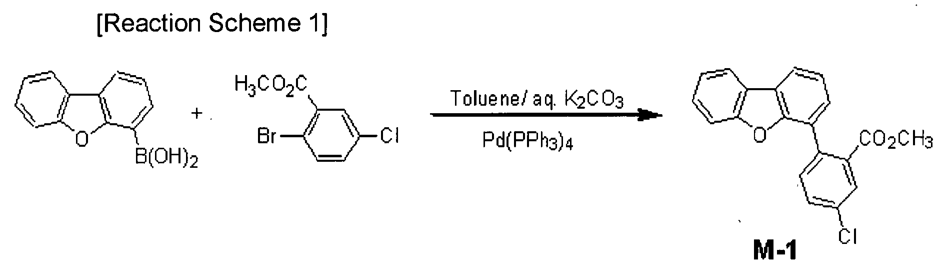

30g (141.5mmol) of (4-dibenzofuranyl)boronic acid, 37.1g (148.6 mmol) of methyl-2-bromo-5-chlorobenzoate, and 8.2 g (7.1 mmol) of tetrakistriphenyl phosphine palladium were put in a flask and dissolved in 550 ml of toluene under a nitrogen atmosphere, and 353.8 ml of an aqueous solution in which 104.2 g (707.51 mmol) of potassium carbonate was dissolved was added thereto. The mixture was agitated for 12 hours. When the reaction was complete, the resulting product was extracted with ethylacetate. The extraction solution was dried with magnesium sulfate and filtered and then, concentrated under a reduced pressure. The concentrated product was purified through a silica gel column chromatography by using n-hexane/dichloromethane mixed in a volume ratio of 7 : 3, obtaining a desired compound, an intermediate M-1 in a white solid of 38.2 g (yield: 80%).

LC-Mass (theoretical value: 336.06 g/mol, measured value: M+1 = 336 g/mol)

### Synthesis of Intermediate M-2

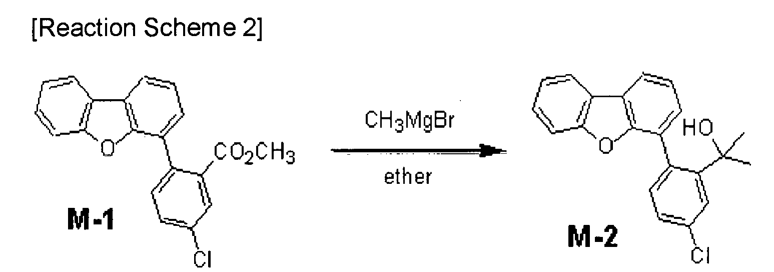

38.18 g (113.37mmol) of the intermediate M-1 was put in a flask and dissolved in 500 ml of anhydrous ether under a nitrogen atmosphere. The solution was cooled down to 0°C and agitated. Then, 110mL of 3M methyl magnesium bromide (in 340.1 mmol of diethyl ether) was slowly added to the agitated solution. The mixture was agitated at room temperature under a nitrogen atmosphere for 5 hours. When the reaction was complete, the resulting solution was concentrated under a reduced pressure to remove the solvent and then, extracted with distilled water and dichloromethane. The extracted solution was dried with anhydrous magnesium sulfate and filtered and then, concentrated under a reduced pressure, obtaining a desired compound, an intermediate M-2 as a liquid with high viscosity.

### Synthesis of Intermediate M-3

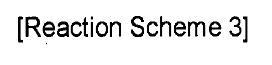

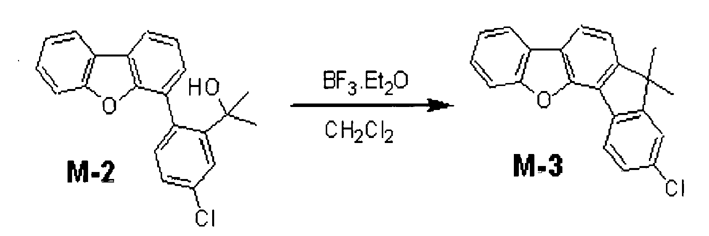

The intermediate M-3 was dissolved in 250 ml of dichloromethane. The solution was cooled down to 0°C and then, agitated. Then, a solution prepared by dissolving borontrifluoride in 20 mL of dichloromethane and 15.18 g (56.7mmol) of diethyl ether complex were slowly added to the above agitated solution. The mixture was agitated for 5 hours. When the reaction was complete, the resulting product was extracted with ethylacetate. The extracted solution was dried with magnesium sulfate and filtered and then, concentrated under a reduced pressure. The product was purified through a silica gel column chromatography by using n-hexane, obtaining a desired compound, an intermediate M-3 in a white solid of 29 g (yield: 80%).

GC-Mass (theoretical value: 318.8 g/mol, measured value: M+1 = 318 g/mol)

### Synthesis of Intermediate M-4

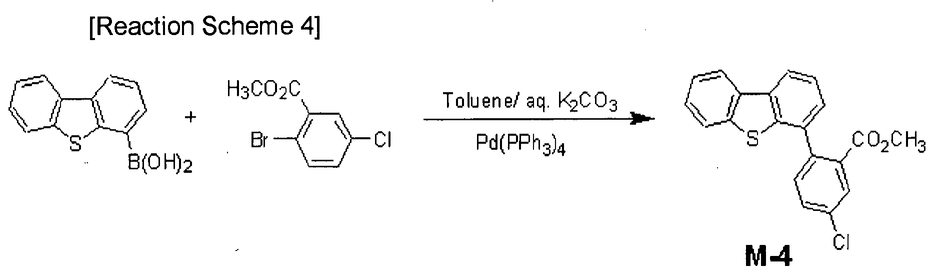

30g (141.5mmol) of (4-dibenzofuranyl)boronic acid as an intermediate, 37.1 g (148.6 mmol) of methyl-2-bromo-5-chlorobenzoate, and 8.2 g (7.1 mmol) of tetrakistriphenyl phosphine palladium were put in a flask and dissolved in 550 ml of toluene under a nitrogen atmosphere, and 353.8 ml of an aqueous solution in which 104.2 g (707.51 mmol) of potassium carbonate was dissolved was added thereto. The mixture was refluxed and agitated for 12 hours. When the reaction was complete, the agitated product was extracted with ethylacetate. The extracted solution was dried with magnesium sulfate and filtered and then, concentrated under a reduced pressure. The concentrated product was purified through n-hexane/dichloromethane mixed in a volume ratio of 7 : 3, obtaining a desired compound, an intermediate M-1 in a white solid of 38.2 g (yield: 80%).

LC-Mass (theoretical value: 336.06 g/mol, measured value: M+1 = 336 g/mol)

### Synthesis of Intermediate M-5

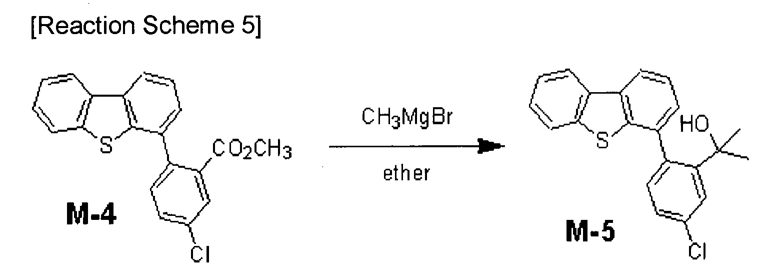

38.2 g (113.37mmol) of the intermediate M-4 was put in a flask and dissolved in 500 ml of anhydrous ether under a nitrogen atmosphere. The resulting reactant was cooled down to 0°C and agitated. Then, 110mL of 3M methyl magnesium bromide (in 324.63 mmol of diethyl ether) was slowly added to the resulting product. The mixture was cooled down to a room temperature and agitated under a nitrogen atmosphere for 5 hours. When the reaction was complete, the reactant was concentrated under a reduced pressure to remove the solvent, extracted with distilled water and dichloromethane, dried with magnesium sulfate, filtered, and concentrated under a reduced pressure, obtaining a desired compound, an intermediate M-2, as a liquid with high viscosity.

### Synthesis of intermediate M-6

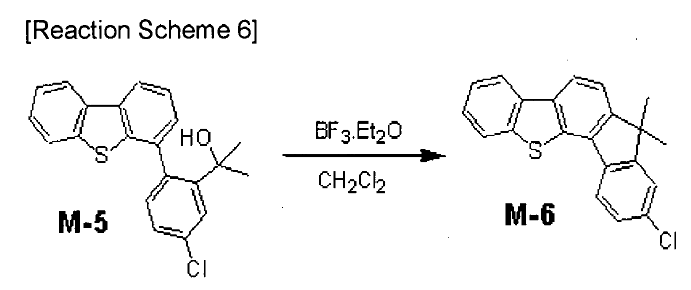

The intermediate M-5 was dissolved in 250 ml of dichloromethane and then, cooled down to 0°C and agitated. Then, boronitrilefluoride dissolved in 20 mL of borontrifluoride and 14.5 g (54.1mmol) of diethyl ether complex were slowly added to the agitated product. The mixture was agitated at room temperature for 5 hours. When the reaction was complete, the resulting product was extracted with ethylacetate. The extracted solution was dried with magnesium sulfate and filtered and then, concentrated under a reduced pressure. The concentrated product was purified through a silica gel column chromatography by using n-hexane, obtaining a desired compound, an intermediate M-6, in a white solid of 31 g (yield: 85.5%).

GC-Mass (theoretical value: 334.86 g/mol, measured value: 335 g/mol)

### Synthesis of Intermediate M-7

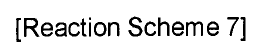

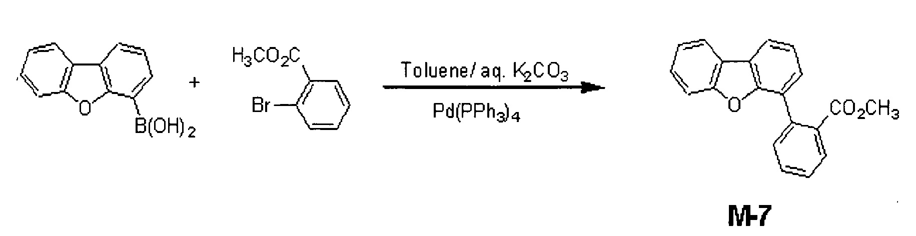

23g (108.5 mmol) of (4-dibenzofuranyl)boronic acid as an intermediate, 24.5 g (113.9 mmol) of methyl-2-bromo-benzoate, and 6.3 g (5.47 mmol) of tetrakistriphenyl phosphine palladium were put in a flask and dissolved in 500 ml of toluene under a nitrogen atmosphere, and 271.2 ml of an aqueous solution prepared by dissolving 79.9 g (542.4 mmol) of potassium carbonate was added thereto. The mixture was refluxed and agitated for 12 hours. When the reaction was complete, the agitaed solution was extracted with ethylacetate. The extracted solution was dried with magnesium sulfate and filtered and then, concentrated under a reduced pressure. The concentrated product was purified through a silica gel column chromatography by using n-hexane/dichloromethane mixed in a volume ratio of 7 : 3, obtaining a desired compound, an intermediate M-7, in a white solid of 31 g (yield: 94.5 %).

LC-Mass (theoretical value: 302.32 g/mol, measured value: M+1 = 303 g/mol)

### Synthesis of Intermediate M-8

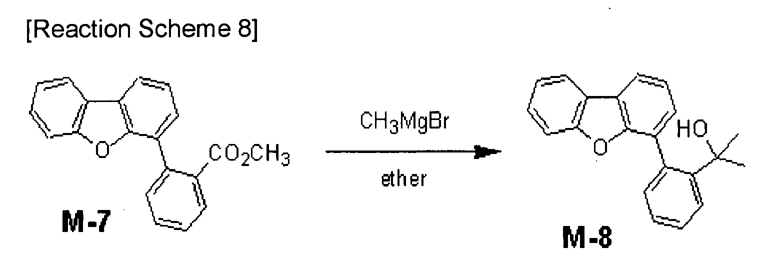

29 g (95.92mmol) of the intermediate M-7 was put in a flask and dissolved in 400 ml of anhydrous tetrahydrofuran (THF) under a nitrogen atmosphere. The solution was cooled down to 0°C and agitated. Then, 95.9mL of 3M methyl magnesium bromide (in 287.8 mmol of diethyl ether) was slowly added to the agitated solution. The mixture was agitated at room temperature under a nitrogen atmosphere for 5 hours. When the reaction was complete, the agitated solution was concentrated under a reduced pressure to remove the solvent. The resulting product was extracted with distilled water and dichloromethane. The extracted solution was dried with magnesium sulfate and filtered and then, concentrated under a reduced pressure, obtaining a desired compound, an intermediate M-8, as a liquid with high viscosity.

### Synthesis of Intermediate M-9

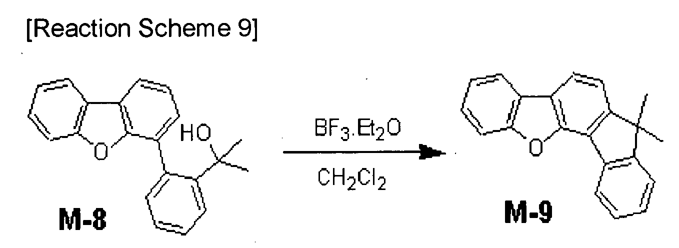

The intermediate M-8 was dissolved in 250 ml of dichloromethane and cooled down to 0°C and then, agitated. Next, 12.84 g (47.5mmol) of boronitrilefluoride and diethyl ether complex dissolved in 20 mL of dichloromethane were slowly added to the agitated product. The mixture was agitated at room temperature for 5 hours. When the reaction was complete, the agitated solution was extracted with ethylacetate. The extracted solution was dried with magnesium sulfate and filtered and then, concentrated under a reduced pressure. The resulting product was purified through a silica gel column chromatography using n-hexane, obtaining a desired compound, an intermediate M-9, in a white solid of 22 g (yield: 71.9%).

LC-Mass (theoretical value: 284.35 g/mol, measured value: M+1 = 284 g/mol)

### Synthesis of Intermediate M-10

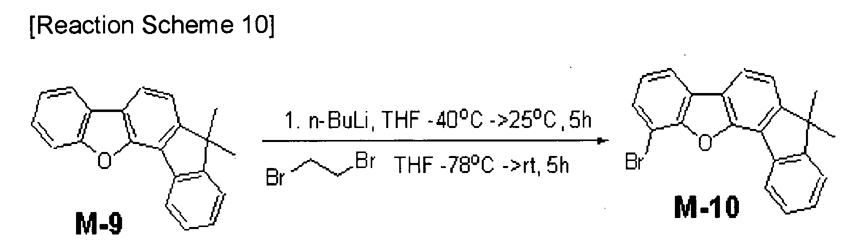

10g (59.5mmol) of the intermediate M-9 was put under a nitrogen atmosphere in a 2-necked round-bottomed flask heated and dried under vacuum, and 119mL of anhydrous tetrahydrofuran was added thereto. The mixture was cooled down to 40°C and agitated. Next, 26mL of 1.6M n-butyllithium (in 65.5mmol of hexane) was slowly added to the agitated solution. The mixture was agitated at room temperature under a nitrogen atmosphere for 5 hours. The resulting product was cooled down to -78°C, and 22.4g (119mmol) of 1,2-dibromoethane dissolved in 10mL of anhydrous tetrahydrofuran was slowly added thereto. The mixture was agitated at room temperature for 5 hours. When the reaction was complete, the agitated solution was concentrated under a reduced pressure to remove the solvent. The concentrated solution was extracted with distilled water and dichloromethane. The extracted solution was dried with magnesium sulfate and filtered and then, concentrated under a reduced pressure. The concentrated product was recrystallized with n-hexane, obtaining a desired compound, an intermediate M-1 0, in a white solid of 11 g (yield: 75%).

GC-Mass (theoretical value: 245.97 g/mol, measured value: 246 g/mol)

### Synthesis of Intermediate M-11

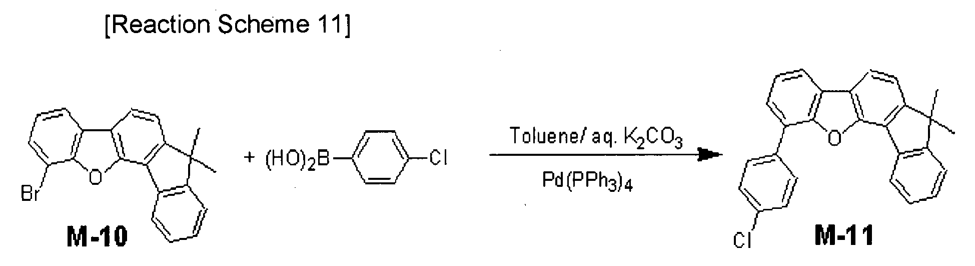

11g (30.28mmol) of the intermediate M-10, 5.0 g (31.8 mmol) of 4-chlorophenylboronic acid, and 1.8 g (1.5 mmol) of tetrakistriphenyl phosphine palladium were put in a flask and dissolved in120 ml of toluene under a nitrogen atmosphere, and 75.7ml of an aqueous solution prepared by dissolving 22.3 g (151.4 mmol) of potassium carbonate. The mixture was refluxed and agitated for 12 hours. When the reaction was complete, the agitated solution was extracted with ethylacetate. The extracted solution was dried with magnesium sulfate and filtered and then, concentrated under a reduced pressure. The concentrated product is purified through n-hexane/dichloromethane mixed in a volume ratio of 7 : 3, obtaining a desired compound, an intermediate M-11, in a white solid of 8.0 g (yield: 66.7%).

GC-Mass (theoretical value: 394.89 g/mol, measured value: 395 g/mol)

### Synthesis of Intermediate M-12

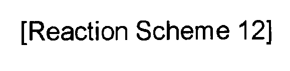

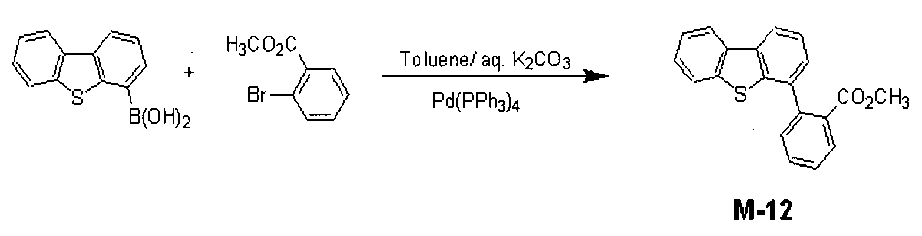

25g (109.62 mmol) of (4-dibenzothiophenyl)boronic acid as an intermediate, 24.8 g (115.1 mmol) of methyl-2-bromo-benzoate, and 6.3 g (5.48 mmol) of tetrakistriphenyl phosphine palladium were put in a flask and dissolved in 500 ml of toluene under a nitrogen atmosphere, and 274 ml of an aqueous solution in which 80.7 g (548.1 mmol) of potassium carbonate was dissolved was added thereto. The mixture was refluxed and agitated for 12 hours. When the reaction was complete, the agitated solution was extracted with ethylacetate. The extracted solution was dried with magnesium sulfate and filtered and then, concentrated under a reduced pressure. The concentrated product was purified through a silica gel column chromatography using n-hexane/dichloromethane mixed in a volume ratio of 7 : 3, obtaining a desired compound, an intermediate M-12, in a white solid of 31 g (yield: 88.8 %).

GC-Mass (theoretical value: 318.39 g/mol, measured value: M+1 = 318 g/mol)

### Synthesis of Intermediate M-13

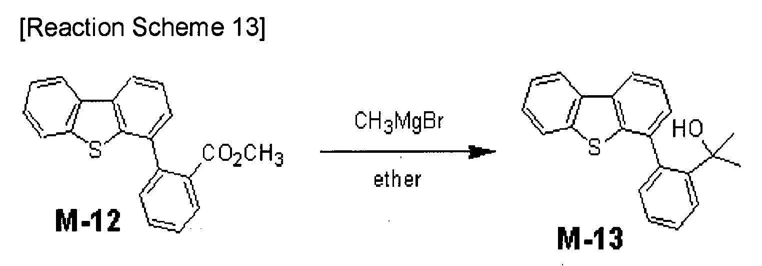

15.4 g (47.1mmol) of the intermediate M-12 was put in a flask and dissolved in 400 ml of anhydrous tetrahydrofuran under a nitrogen atmosphere. The mixture was cooled down to 0°C and agitated. Next, 50mL of 3M methyl magnesium bromide (in 141.34 mmol of diethyl ether) was slowly added to the agitated mixture. The resulting mixture was agitated at room temperature under a nitrogen atmosphere for 5 hours. When the reaction was complete, the agitated solution was concentrated under a reduced pressure to remove the solvent and extracted with water and dichloromethane. The extracted solution was dried with magnesium sulfate and filtered and then, concentrated under a reduced pressure, obtaining a desired compound, an intermediate M-13, as a liquid with high viscosity.

### Synthesis of Intermediate M-14

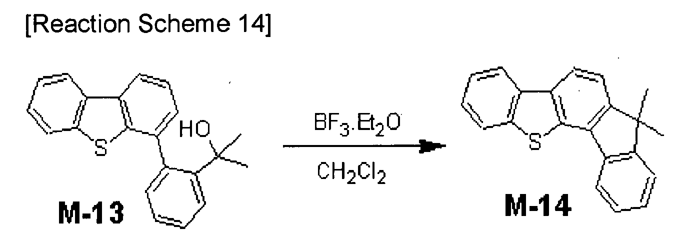

The intermediate M-13 was dissolved in 250 ml of dichloromethane. The solution was cooled down to 0°C and agitated. Next, 12.6 g (47.1mmol) of boronitrilefluoride and diethyl ether complex dissolved in 20 mL of dichloromethane were slowly added to the agitated solution. The mixture was agitated at room temperature for 5 hours. When the reaction was complete, the agitated mixture was extracted with ethylacetate. The extracted solution was dried with magnesium sulfate and filtered and then, concentrated under a reduced pressure. The concentrated product was purified through a silica gel column chromatography by using n-hexane, obtaining a desired compound, an intermediate M-14, in a white solid of 10.5 g (yield: 74.3%).

LC-Mass (theoretical value: 300.42 g/mol, measured value: M+1 = 300 g/mol)

### Synthesis of Intermediate M-15

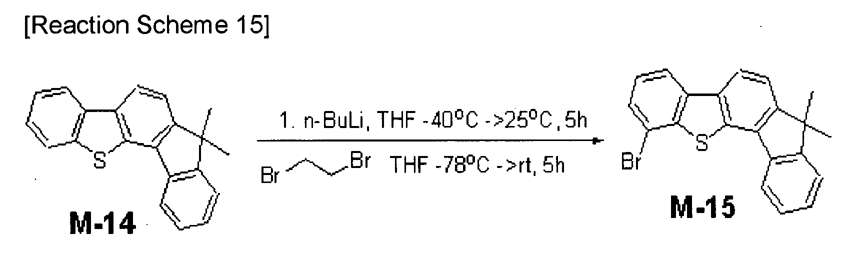

9.6g (31.79mmol) of the intermediate M-14 was put under a nitrogen atmosphere in a 2-necked round-bottomed flask heated and dried under vacuum, and 100mL of anhydrous tetrahydrofuran was added thereto. The mixture was cooled down to -40°C and agitated. Next, 20.7mL of 1.6M n-butyllithium (in 63.6mmol of hexane) was slowly added to the agitated mixture. The resulting mixture was agitated at room temperature under a nitrogen atmosphere for 5 hours. The resulting reactant was cooled down to -78°C, and 11.9g (63.58mmol) of 1,2-dibromoethane dissolved in 10mL of anhydrous tetrahydrofuran was slowly added thereto. The mixture was agitated at room temperature for 5 hours. When the reaction was complete, the agitated product was concentrated under a reduced pressure to remove the solvent and then, extracted with distilled water and dichloromethane. The extracted solution was dried with magnesium sulfate and filtered and then, concentrated under a reduced pressure. The concentrated reactant was recrystallized with n-hexane, obtaining a desired compound, an intermediate M-15, in a white solid of 9.5g (yield: 78.8%).

GC-Mass (theoretical value: 379.31 g/mol, measured value: 380 g/mol)

### Synthesis of Intermediate M-16

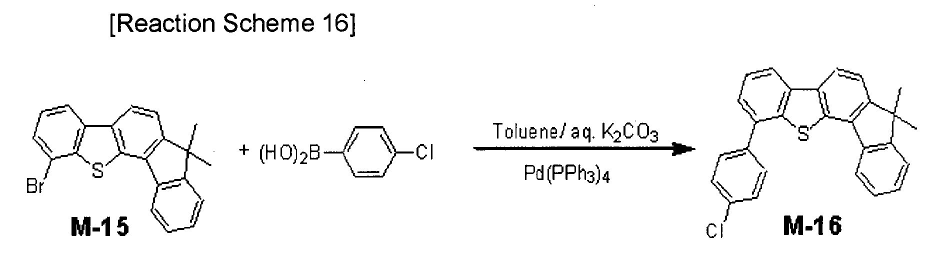

9.5g (26.4mmol) of the intermediate M-15, 4.3 g (27.7 mmol) of 4-chlorophenylboronic acid, and 1.5 g (1.3 mmol) of tetrakistriphenyl phosphine palladium were put in a flask and dissolved in 150 ml of toluene under a nitrogen atmosphere, and 64.9 ml of an aqueous solution in which 19.4 g (130.9 mmol) of potassium carbonate was dissolved was added thereto. The mixture was refluxed and agitated for 12 hours. When the reaction was complete, the agitated product was extracted with ethylacetate. The extracted solution was dried with magnesium sulfate and then, concentrated under a reduced pressure. The concentrated product was purified through n-hexane/dichloromethane in a volume ratio of 7: 3, obtaining a desired compound, an intermediate M-16, in a white solid of 5.3 g (yield: 48.5%).

GC-Mass (theoretical value: 394.89 g/mol, measured value: 395 g/mol)

### Synthesis of Intermediate M-17

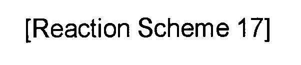

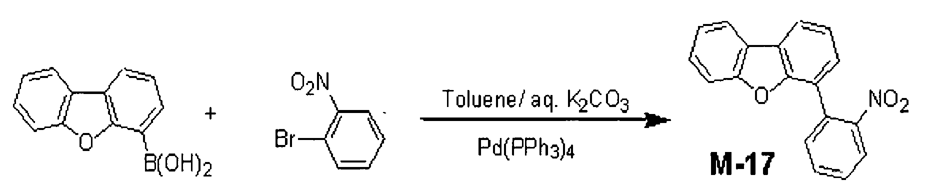

15.0 g (70.7 mmol) of dibenzofuran boronic acid, 19.38 g (77.83 mmol) of 1-bromo-2-nitro benzene, and 2.46g (2.12 mmol) of tetrakistriphenyl phosphine palladium were put in a 500 Mℓ round-bottomed flask under a nitrogen atmosphere and dissolved in 200 mL of toluene, and 19.56 g (141 mmol) of potassium acetate and 70 Mℓ of water were added thereto. The mixture was agitated at 90 °C for 24 hours. When the reaction was complete, the reactant was cooled down to a room temperature and extracted with toluene and water. The extracted reactant was dried with anhydrous magnesium sulfate and filtered. Then, the solvent was removed from the reactant. The resulting product was purified through a silica gel column using methylene chloride/hexane mixed in a ratio of 1:1, obtaining an intermediate M-17 of 15 g (yield: 73.3 %).

### Synthesis of Intermediate M-18

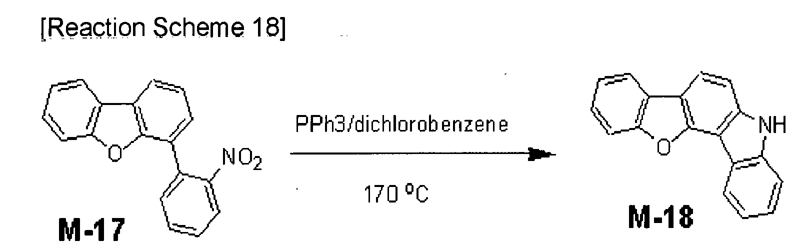

15.0 g (51.8 mmol) of the intermediate M-17 and 36 g (137.5 mmol) of triphenylphosphine were put in a 500 Mℓ round-bottomed flask under atmosphere and dissolved in dichlorobenzene. The solution was agitated at 160 °C for 24 hours. When the reaction was complete, the reactant was cooled down to a room temperature, and dichlorobenzene was removed therefrom under a reduced pressure. The resulting product was purified through a silica gel column using a solvent of methylene chloride/hexane mixed in a ratio of 1:1, obtaining an intermediate M-18 of 9.1 g (yield: 68.2 %).

### Synthesis of Intermediate M-19

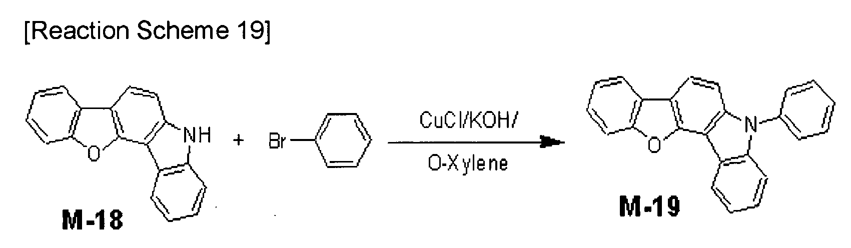

9g (34.9 mmol) of the intermediate M-18, 10.9 g (69.9 mmol) of bromobenzene, 1.6g (17.4 mmol) of copper chloride, 20g (356.4 mmol) of potassium hydroxide were dissolved in 100Mℓ of xylene in a 250Mℓ round-bottomed flask under a nitrogen atmosphere. The solution was agitated at 150°C for 48 hours and cooled down to a room temperature, and xylene therein was removed under a reduced pressure. The remaining solid was dissolved in methylene chloride and several times washed with water and then, treated with anhydrous magnesium sulfate to remove moisture and filtered. Then, the solvent was removed from the filtered solution. The resulting product was purified through a silica gel column using a solvent of hexane/methylene chloride mixed in a ratio of 1:2, obtaining a desired compound M-19 of 10 g (yield: 86 %)

### Synthesis of Intermediate M-20

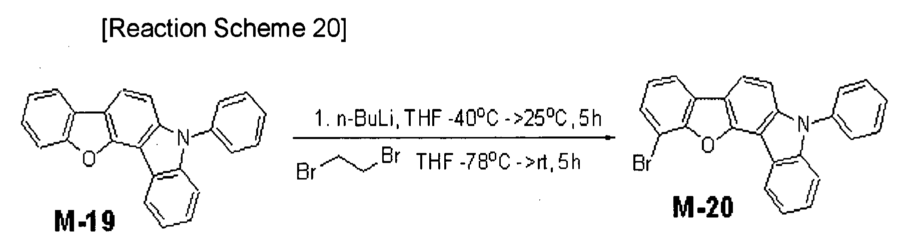

10.0g (30.0mmol) of the intermediate M-19 was put under a nitrogen atmosphere in a two-necked round-bottomed flask heated and dried under vacuum, and 100mL of anhydrous tetrahydrofuran was added thereto. The mixture was cooled down to 40°C and agitated. Then, 10.4 mL of 1.6M n-butyllithium (in 31.20mmol of hexane) was slowly added to the agitated mixture. The resulting mixture was agitated at room temperature under a nitrogen atmosphere for 5 hours. The reactant solution was cooled down to -78°C, and 11.3g (60.0mmol) of 1,2-dibromoethane dissolved in 10mL of anhydrous tetrahydrofuran was slowly added thereto. The mixture was agitated at room temperature for 5 hours. When the reaction was complete, the agitated reactant was concentrated under a reduced pressure concentrate to remove the solvent and then, extracted with distilled water and dichloromethane. The extracted solution was dried with magnesium sulfate and filtered and then, concentrated under a reduced pressure. The reactant was recrystallized with n-hexane, obtaining a desired compound, an intermediate M-20, in a white solid of 9.7g (yield: 78.4%).

GC-Mass (theoretical value: 412.28 g/mol, measured value: 412 g/mol)

### Synthesis of Intermediate M-21

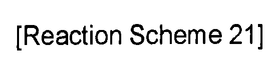

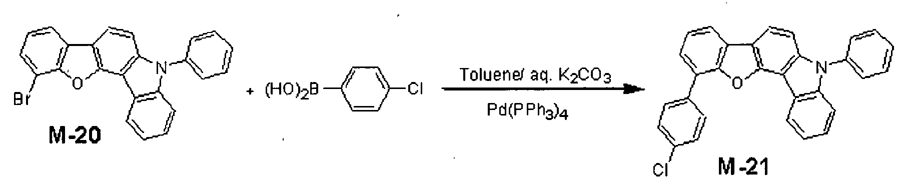

9.7g (23.5mmol) of the intermediate M-20, 3.9 g (24.7 mmol) of 4-chlorophenyl boronic acid, and 1.4 g (1.2 mmol) of tetrakistriphenyl phosphine palladium were put in a flask and dissolved in 100 ml of toluene under a nitrogen atmosphere, and 58.8 ml of an aqueous solution in which 17.3 g (117.6 mmol) of potassium carbonate was dissolved was added thereto. The mixture was refluxed and agitated for 12 hours. When the reaction was complete, the agitated reactant was extracted with ethylacetate. The extracted solution was dried with anhydrous magnesium sulfate and filtered and then, concentrated under a reduced pressure. The concentrated product was purified through a silica gel column chromatography using n-hexane/methylene chloride mixed in a volume ratio of 7 : 3, obtaining a desired compound, an intermediate M-21, in a white solid of 7.0 g (yield: 67.0%).

GC-Mass (theoretical value:443.92 g/mol, measured value: 444 g/mol)

### Synthesis of Intermediate M-22

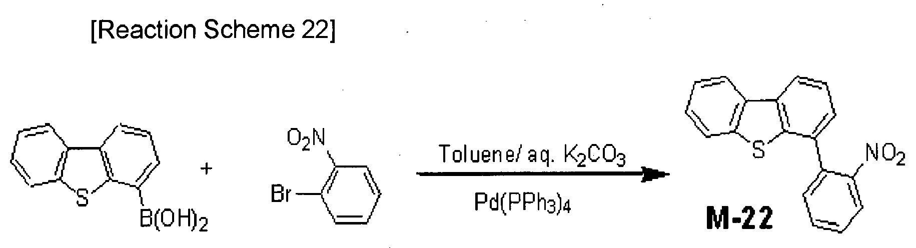

15.0 g (65.7 mmol) of dibenzofuran boronic acid, 18.0 g (72.3 mmol) of 1-bromo-2-nitro benzene, and 2.3g (1.97 mmol) of tetrakistriphenyl phosphine palladium were put in a 500 Mℓ round-bottomed flask and dissolved in 200 mL of toluene under a nitrogen atmosphere, and 18.2 g (131.5 mmol) of potassium acetate and 70 Mℓ of water were added thereto. The mixture was agitated at 90 °C for 24 hours. When the reaction was complete, the reactant was cooled down to a room temperature and extracted with toluene and water and then, treated with anhydrous magnesium sulfate to remove moisture. The resulting product was filtered, and the solvent therein was removed. The resulting product was purified through a silica gel column using a mixed solvent of methylene chloride/hexane mixed in a ratio of 1:1, obtaining an intermediate M-22 of 14 g (yield: 69.7 %).

### Synthesis of Intermediate M-23

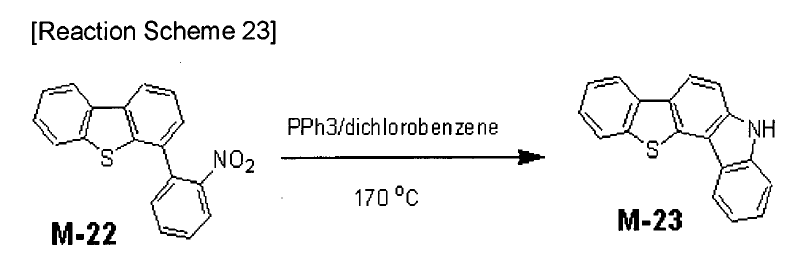

14.0 g (45.8 mmol) of the intermediate M-22 and 36 g (137.5 mmol) of triphenylphosphine were dissolved in dichlorobenzene in a 500 Mℓ round-bottomed flask under a nitrogen atmosphere. The solution was agitated at 160 °C for 24 hours. When the reaction was complete, the reactant was cooled down to a room temperature, and the dichlorobenzene therein was removed under a reduced pressure. The resulting reactant was purified through a silica gel column using methylene chloride/hexane mixed in a ratio of 1:1, obtaining an intermediate M-23 of 9.7 g (yield: 77.6 %).

### Synthesis of Intermediate M-24

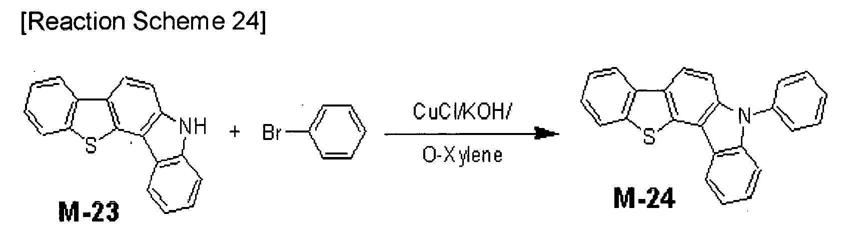

9.7g (35.4 mmol) of the intermediate M-23, 11.1 g (70.9 mmol) of bromobenzene, 1.6g (17.4 mmol) of copper chloride, and 20g (356.4 mmol) of potassium hydroxide were dissolved in 100Mℓ of xylene in a 250Mℓ round-bottomed flask under a nitrogen atmosphere. The solution was agitated at 150°C for 48 hours and cooled down to a room temperature, and the xylene was removed under a reduced pressure. The remaining solid was dissolved in methylene chloride and several times washed with water and then, treated with anhydrous magnesium sulfate to remove moisture. Then, the reactant was filtered, and the solvent was removed therefrom. The resulting product was purified through a silica gel column using a solvent of hexane/methylene chloride mixed in a ratio of 1:2, obtaining a desired compound M-24 of 11 g (yield: 89 %).

### Synthesis of Intermediate M-25

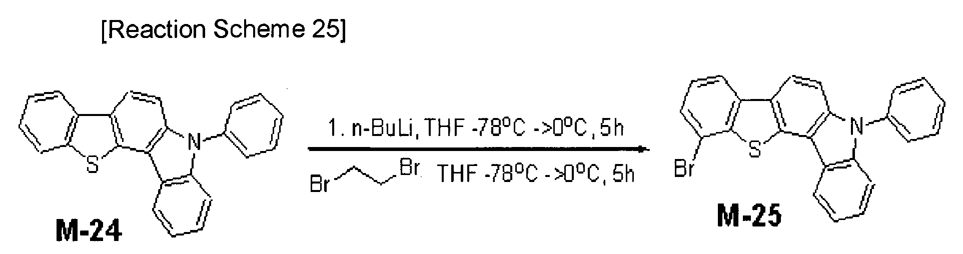

10.0g (28.6mmol) of the intermediate M-24 was put under a nitrogen atmosphere in a 2-necked round-bottomed flask heated and dried under vacuum, and 100mL of anhydrous tetrahydrofuran was added thereto for dissolution. The solution was cooled down to 40°C and agitated. Then, 9.92 mL of 1.6M n-butyllithium (in 29.76mmol of hexane) was slowly added to the agitated solution. The mixture was agitated at room temperature under a nitrogen atmosphere for 5 hours. The agitated reactant was cooled down to - 78°C, and 10.8g (57.2mmol) of 1,2-dibromoethane dissolved in 10mL of anhydrous tetrahydrofuran was slowly added thereto. The mixture was agitated at room temperature for 5 hours. When the reaction was complete, the resulting product was concentrated under a reduced pressure to remove the solvent and extracted with distilled water and dichloromethane. The extracted solution was dried with magnesium sulfate and filtered and then, concentrated under a reduced pressure. The reactant was recrystallized with n-hexane, obtaining a desired compound, an intermediate M-25, in a white solid of 9.8g (yield: 79.9%).

GC-Mass (theoretical value: 428.34 g/mol, measured value: 428 g/mol)

### Synthesis of Intermediate M-26

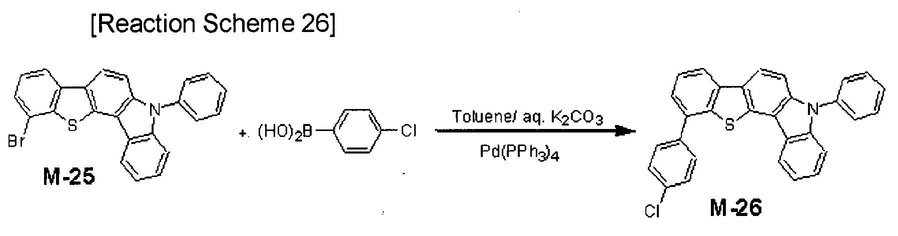

9.8g (22.9mmol) of the intermediate M-25, 3.8 g (24.0 mmol) of 4-chlorophenylboronic acid, and 1.3 g (1.1 mmol) of tetrakistriphenyl phosphine palladium were put in a flask and dissolved in 100 ml of toluene under a nitrogen atmosphere, and 57.2ml of an aqueous solution in which 16.8 g (114.4 mmol) of potassium carbonate was dissolved was added thereto. The mixture was refluxed and agitated for 12 hours. When the reaction was complete, the reactant was extracted with ethylacetate. The extracted solution was dried with magnesium sulfate and filtered and then, concentrated under a reduced pressure. The resulting product was purified through a silica gel column chromatography using n-hexane/dichloromethane mixed in a volume ratio 7 : 3, obtaining a desired compound, an intermediate M-26, in a white solid of 6.0 g (yield; 57.0%).

GC-Mass (theoretical value:459.99 g/mol, measured value: 460 g/mol)

### Synthesis of Intermediate M-27

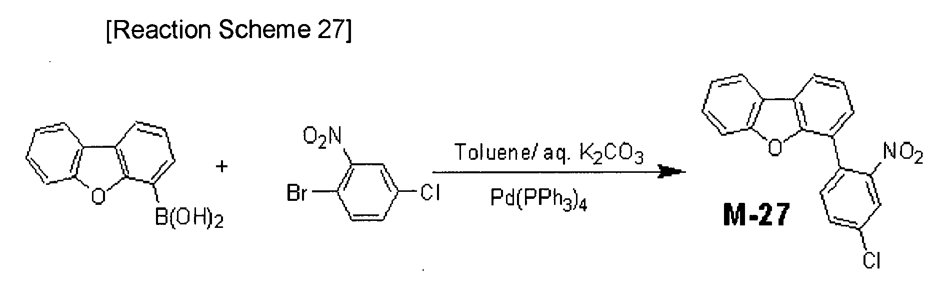

20.0 g (94.3 mmol) of dibenzofuran boronic acid, 24.5 g (103.7 mmol) of 2-bromo-5-chloronitrobenzene, and 3.28 g (2.83 mmol) of tetrakistriphenyl phosphine palladium were put in a 1 L round-bottomed flask and dissolved in toluene of 300 mL under a nitrogen atmosphere, and 26 g (188.6 mmol) of potassium acetate and 100 Me of water were added thereto. The mixture was agitated at 90 °C for 24 hours. When the reaction was complete, the reactant was cooled down to a room temperature and extracted with toluene and water. The extracted reactant was treated with anhydrous magnesium sulfate to remove moisture and treated, and the solvent was removed. Then, the resulting product was purified through a silica gel column using methylene chloride/hexane mixed in a ratio of 1:1, obtaining an intermediate M-27 of 20 g (yield: 65.49 %).

### Synthesis of Intermediate M-28

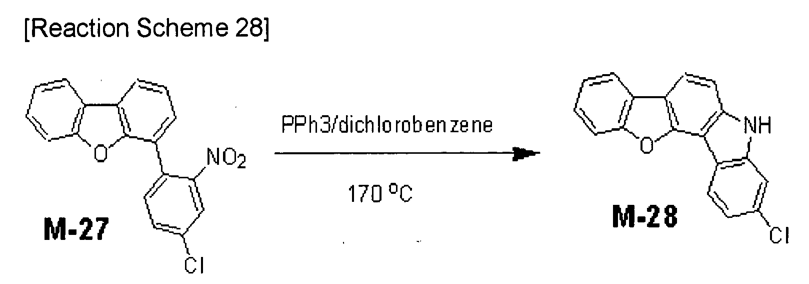

20.0 g (61.7 mmol) of the intermediate M-27 and 48.6 g (185.3 mmol) of triphenylphosphine were dissolved in 500 Mℓ of dichlorobenzene in a round-bottomed flask under a nitrogen atmosphere. The solution was agitated at 160 °C for 24 hours. When the reaction was complete, the reactant was cooled down to a room temperature, and the dichlorobenzene was removed under a reduced pressure. The resulting product was purified through a silica gel column using methylene chloride/hexane mixed in a ratio of 1:1, obtaining an intermediate M-28 of 14.5 g (yield: 81 %).

### Synthesis of Intermediate M-29

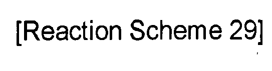

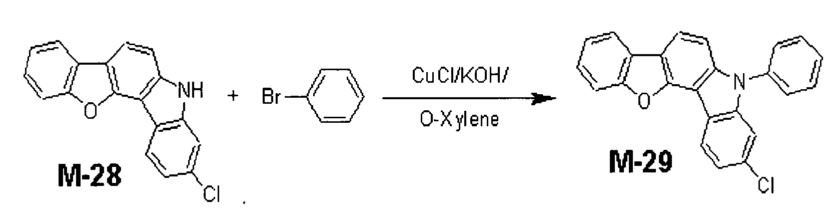

14 g (47.9 mmol) of the intermediate M-28, 15 g (95.9 mmol) of bromobenzene, 3.2g (34.8 mmol) of copper chloride, and 30g (535.7 mmol) of potassium hydroxide were dissolved in 200Mℓ xylene in a 250Mℓ round-bottomed flask under a nitrogen atmosphere. The solution was agitated at 150°C for 48 hours and cooled down to a room temperature, and the xylene was removed under a reduced pressure. The remaining solid was dissolved in methylene chloride, several times washed with water, and treated with anhydrous magnesium sulfate to remove moisture, and then, the solvent was removed. The resulting product was purified through a silica gel column using hexane/methylene chloride mixed in a ratio of 1:2, obtaining a desired compound M-29 of 14 g (yield: 79.5 %).

### Synthesis of Intermediate M-30

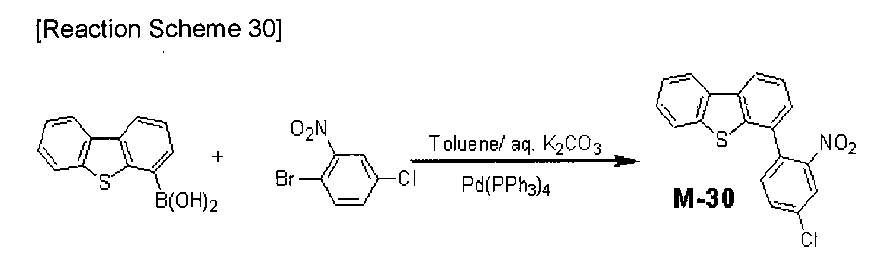

20.0 g (87.6 mmol) of dibenzothiophene boronic acid, 22.8 g (96.4 mmol) of 2-bromo-5-chloronitrobenzene, and 3 g (2.63 mmol) of tetrakistriphenyl phosphine palladium was dissolved in 300 mL of toluene in a 1 L round-bottomed flask under a nitrogen atmosphere, and 24.2 g (175.3 mmol) of potassium acetate and 100 Mℓ of water were added thereto. The mixture was agitated at 90 °C for 24 hours. When the reaction was complete, the reactant was cooled down to a room temperature, extracted with toluene and water, treated with anhydrous magnesium sulfate to remove moisture, and filtered, and then, the solvent was removed therefrom. The resulting product was purified through a silica gel column using methylene chloride/hexane mixed in a ratio of 1:1, obtaining an intermediate M-27 of 23 g (yield: 77.2 %).

### Synthesis of Intermediate M-31

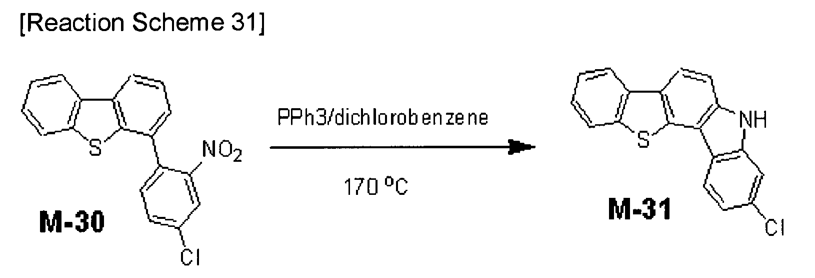

20.0 g (58.8 mmol) of the intermediate M-30 and 48.6 g (185.3 mmol) of triphenylphosphine were dissolved in dichlorobenzene in a 500 Mℓ round-bottomed flask under a nitrogen atmosphere. The solution was agitated at 160 °C for 24 hours. When the reaction was complete, the reactant was cooled down to a room temperature, and the dichlorobenzene was removed under a reduced pressure. The resulting reactant was purified through a silica gel column using methylene chloride/hexane mixed in ratio of 1:1, obtaining an intermediate M-31 of 14 g (yield: 77.3%).

### Synthesis of Intermediate M-32

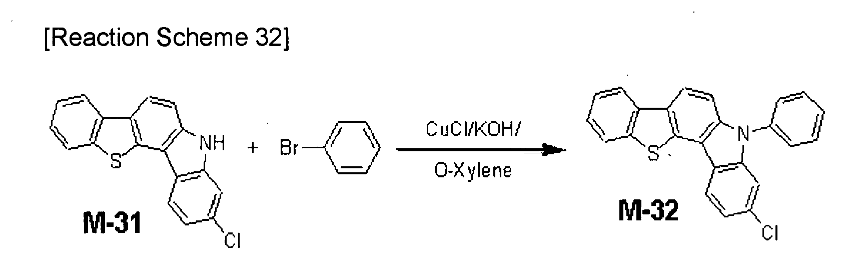

14 g (45.4 mmol) of the M-31, 14.2 g (90.9 mmol) of bromobenzene, 3.2g (34.8 mmol) of copper chloride, and 30g (535.7 mmol) of potassium hydroxide were dissolved in 200Mℓ of xylene in a 250Mℓ round-bottomed flask under a nitrogen atmosphere. The solution was agitated at 150°C for 48 hours and cooled down to a room temperature, and the xylene was removed under a reduced pressure. The remaining solid was dissolved in methylene chloride, several times washed with water, treated with anhydrous magnesium sulfate to remove moisture, and filtered, and then, the solvent was removed. The resulting product was purified through a silica gel column hexane/methylene chloride mixed in a ratio of 1:2, obtaining a compound M-32 of 13 g (74.6 %).

### Synthesis of Intermediate M-33

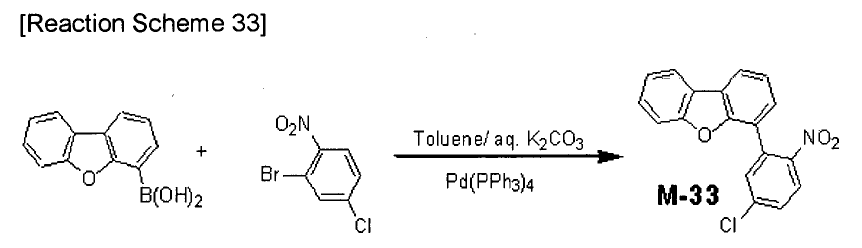

20.0 g (94.3 mmol) of dibenzofuran boronic acid, 24.5 g (103.7 mmol) 2-bromo-5-chloronitrobenzene, and 3.28 g (2.83 mmol) of tetrakistriphenylphosphine palladium were dissolved in 300 mL of toluene in a 1 L round-bottomed flask under a nitrogen atmosphere, and 26 g (188.6 mmol) of potassium acetate and 100 Mℓ of water were added thereto. The mixture was agitated at 90 °C for 24 hours. When the reaction was complete, the reactant was cooled down to a room temperature, extracted with toluene and water, and treated with anhydrous magnesium sulfate to remove moisture, and filtered, and then, the solvent was removed therefrom. The resulting reactant was purified through a silica gel column using methylene chloride/hexane mixed in a ratio of 1:1, obtaining an intermediate M-33 of 21 g (yield: 68.7 %).

### Synthesis of Intermediate M-34

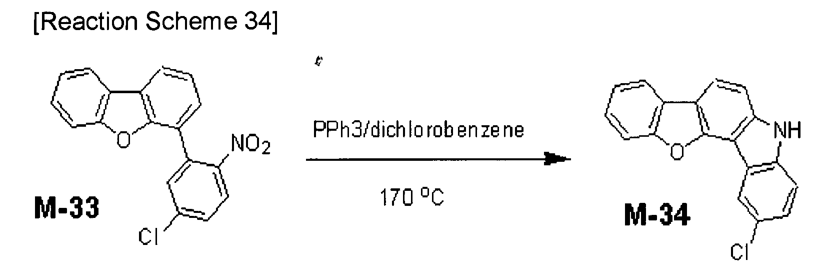

20.0 g (61.7 mmol) of the intermediate M-33 and 48.6 g (185.3 mmol) of triphenylphosphine were dissolved in dichlorobenzene in a 500 Mℓ round-bottomed flask under a nitrogen atmosphere and then, agitated at 160 °C for 24 hours. When the reaction was complete, the reactant was cooled down to a room temperature, and the dichlorobenzene was removed under a reduced pressure. The resulting reactant was purified through a silica gel column using methylene chloride/hexane mixed in a ratio of 1:1, obtaining an intermediate M-34 of 14 g (yield: 77.7 %).

### Synthesis of Intermediate M-35

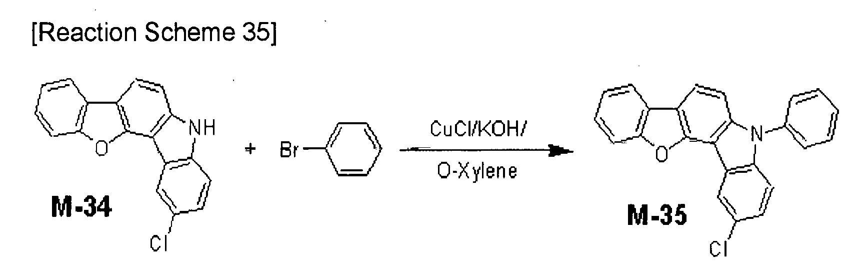

14 g (47.9 mmol) of M-34, 15 g (95.9 mmol) of bromobenzene, 3.2g (34.8 mmol) of copper chloride, and 30g (535.7 mmol) of potassium hydroxide were dissolved in 200Mℓ of xylene in a 250Mℓ round-bottomed flask under a nitrogen atmosphere. The solution was agitated at 150°C for 48 hours and cooled down to a room temperature, and xylene was removed under a reduced pressure. The remaining solid was dissolved in methylene chloride, several times washed with water, treated with anhydrous magnesium sulfate to moisture, and filtered, and the solvent was removed therefrom. The resulting product was purified through a silica gel column using hexane/methylene chloride mixed in a ratio of 1:2, obtaining a desired compound M-5 of 14.5 g (yield: 82.3 %).

### Synthesis of Intermediate M-36

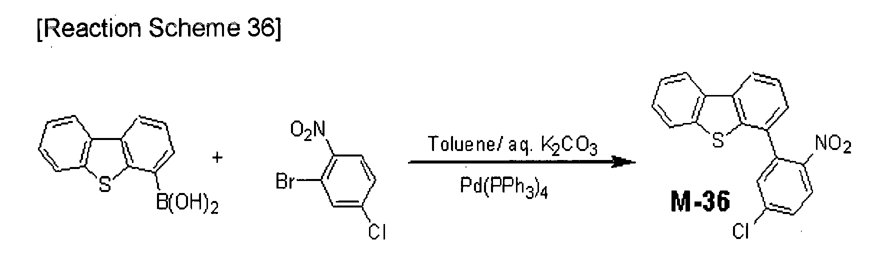

20.0 g (87.6 mmol) of dibenzothiophene boronic acid, 22.8 g (96.4 mmol) of 2-bromo-5-chloronitrobenzene, and 3 g (2.63 mmol) of tetrakistriphenyl phosphine palladium were dissolved in 300 mL of toluene in a 1 L round-bottomed flask under a nitrogen atmosphere, and 24.2 g (175.3 mmol) of potassium acetate and 100 Mℓ of water were added thereto. The mixture was agitated at 90 °C for 24 hours. When the reaction was complete, the reactant was cooled down to a room temperature, extracted with toluene and water, and treated with anhydrous magnesium sulfate to remove moisture, and the solvent was removed therefrom. Then, the reactant was purified through a silica gel column using methylene chloride/hexane mixed in a ratio of 1:1, obtaining an intermediate M-36 of 22 g (yield: 73.8 %).

### Synthesis of Intermediate M-37

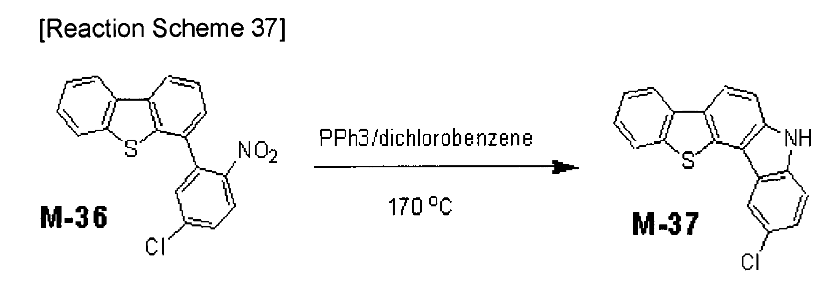

20.0 g (58.8 mmol) of the intermediate M-36 and 48.6 g (185.3 mmol)triphenylphosphine were dissolved in dichlorobenzene in a 500 Mℓ round-bottomed flask under a nitrogen atmosphere and agitated at 160 °C for 24 hours. When the reaction was complete, the reactant was cooled down to a room temperature, and the dichlorobenzene was removed under a reduced pressure. He resulting reactant was purified through a silica gel column using methylene chloride/hexane mixed in a ratio of 1:1, obtaining an intermediate M-37 of 14.5 g (yield: 80.5%).

### Synthesis of Intermediate M-38

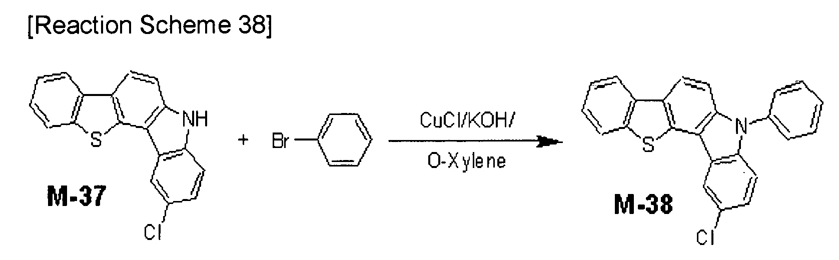

14 g (45.4 mmol) of M-37, 14.2 g (90.9 mmol) of bromobenzene, 3.2g (34.8 mmol) of copper chloride, and 30g (535.7 mmol) of potassium hydroxide were dissolved in 200Mℓ of xylene in a 250Mℓ round-bottomed flask under a nitrogen atmosphere. The solution was agitated at 150°C for 48 hours and cooled down to a room temperature, and the xylene was removed under a reduced pressure. The remaining solid was dissolved in methylene chloride, several times washed with water, treated with anhydrous magnesium sulfate to remove moisture, and the solvent was removed. Then, the reactant was purified through a silica gel column using hexane/methylene chloride mixed in a ratio of 1:2, obtaining a compound M-38 of 13.5 g (yield: 77.5 %).

### Synthesis of intermediate M-39

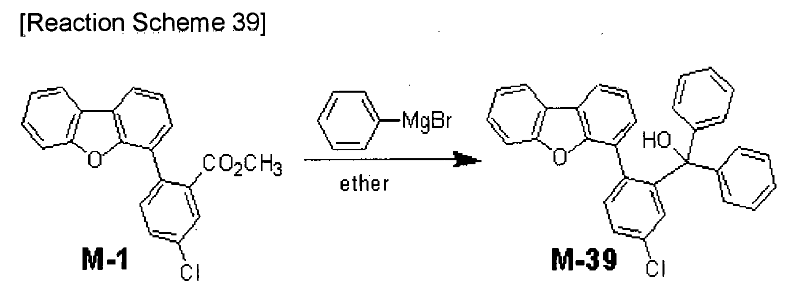

A compound M-39 was synthesized according to the same method as the method of preparing the intermediate M-2 except for using phenylmagnesium bromide instead of methylmagnesium bromide.

### Synthesis of Intermediate M-40

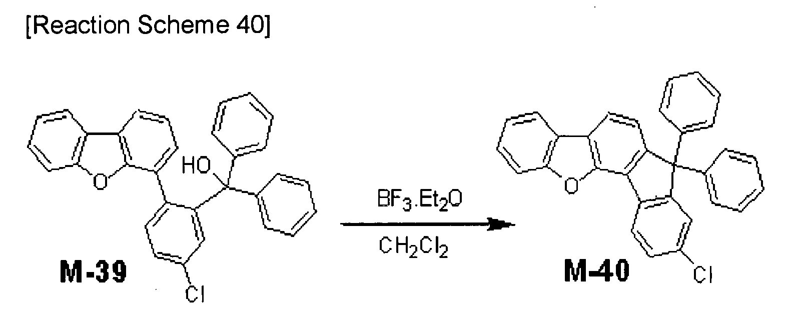

20 g of a compound M-40 (yield: 82 %) was synthesized according to the same method as the method of preparing the intermediate M-3.

### Synthesis of Intermediate M-41

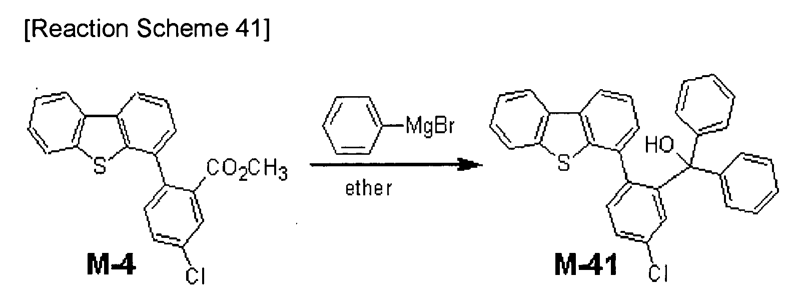

A compound M-41 was synthesized according to the same method as the method of preparing the intermediate M-5 except for using phenylmagnesiumbromide instead of methylmagnesium bromide.

### Synthesis of Intermediate M-42

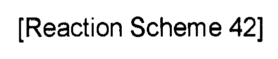

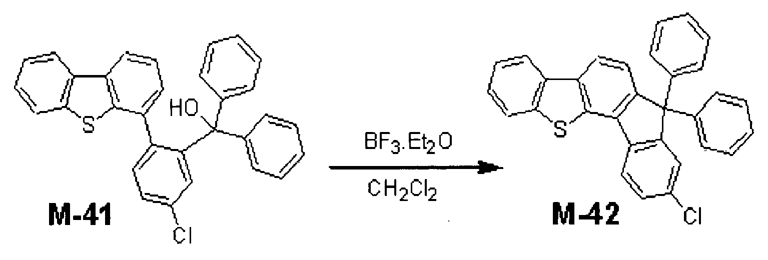

22 g of a compound M-42 (yield: 81 %) was synthesized according to the same method as the method of preparing the intermediate M-6.

### Example 1: Preparation of Compound C-1

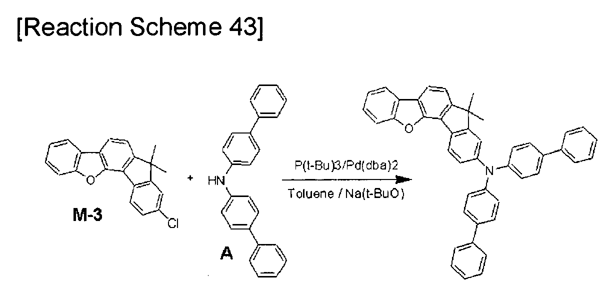

5.0 g (15.68 mmol) of the intermediate M-3, 5.04 g (15.68 mmol) of the intermediate A, sodium 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 : 1, obtaining a desired compound C-1 in a white solid of 7.8g (yield 82.3%).

Calculated value: C, 89.52; H, 5.51; N, 2.32; O, 2.65

Analyzed value: C, 89.51; H, 5.52; N, 2.32; O, 2.65

### Example 2: Preparation of Compound C-31

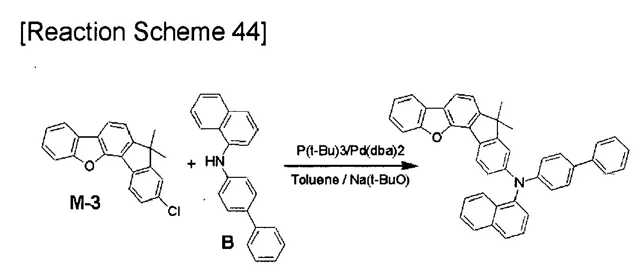

5.0 g (15.68 mmol) of the intermediate M-3, 4.63 g (15.68 mmol) of the intermediate B, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-31 in a white solid of 7.3g (yield: 80.5%).

Calculated value: C, 89.40; H, 5.41; N, 2.42; 0, 2.77

Analyzed value: C, 89.42; H, 5.39; N, 2.42; O, 2.77

### Example 3: Preparation of Compound C-32

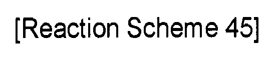

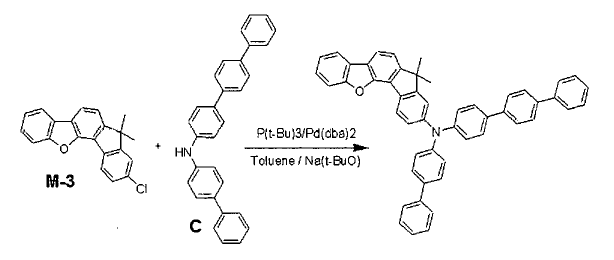

5.0 g (15.68 mmol) of the intermediate M-3, 6.23 g (15.68 mmol) of the intermediate C, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-32 in a white solid of 9.2g (yield: 86.2%).

Calculated value: C, 90.10; H, 5.49; N, 2.06; O, 2.35

Analyzed value: C, 90.12; H, 5.47; N, 2.06; O, 2.35

### Example 4: Preparation of Compound C-5

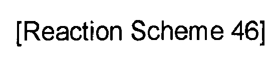

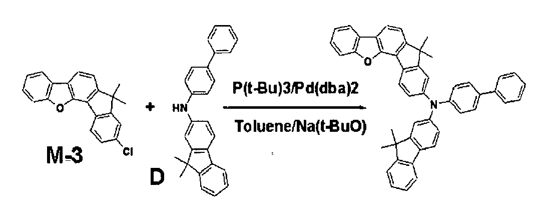

5.0 g (15.68 mmol) of the intermediate M-3, 5.67 g (15.68 mmol) of the intermediate D, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-5 in a white solid of 8.6g (yield 85.1 %).

Calculated value: C, 89.55; H, 5.79; N, 2.18; O, 2.49

Analyzed value: C, 89.56; H, 5.78; N, 2.18; O, 2.49

### Example 5: Preparation of Compound C-19

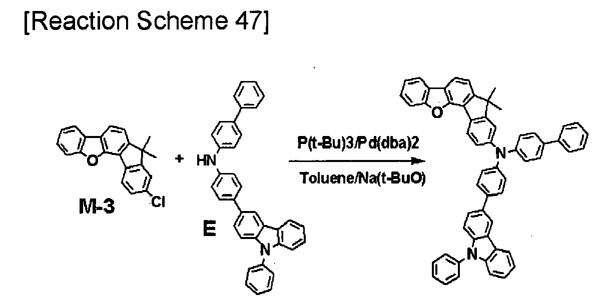

5.0 g (15.68 mmol) of the intermediate M-3, 7.63 g (15.68 mmol) of the intermediate E, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ were added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-19 in a white solid of 10.5g (yield: 87%).

Calculated value: C, 89.03; H, 5.24; N, 3.64; 0,2.08

Analyzed value: C, 89.01; H, 5.26; N, 3.64; O, 2.08

### Example 6: Preparation of Compound C-25

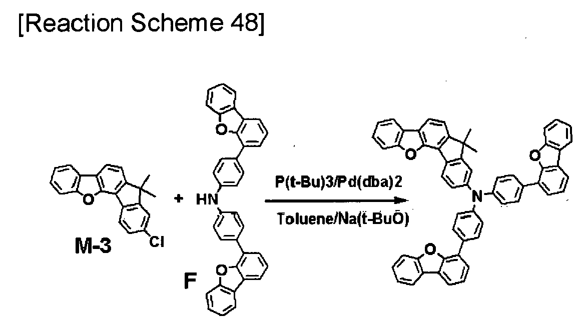

5.0 g (15.68 mmol) of the intermediate M-3, 7.87 g (15.68 mmol) of the intermediate F, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-25 in a white solid of 10.7g (yield: 87%).

Calculated value: C, 87.33; H, 4.76; N, 1.79; 0,6.12

Analyzed value: C, 87.31; H, 4.78; N, 1.79; 0,6.12

### Example 7: Preparation of Compound C-27

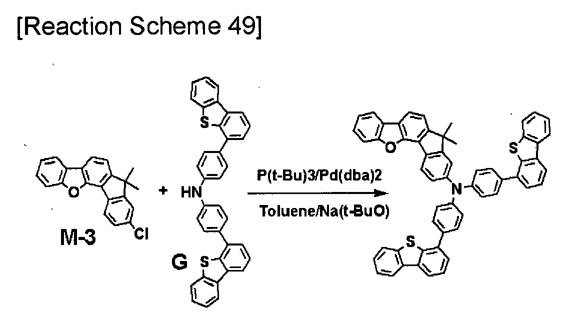

5.0 g (15.68 mmol) of the intermediate M-3, 8.37 g (15.68 mmol) of the intermediate G, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-27 in a white solid of 10.4g (yield: 81.2%).

Calculated value: C, 83.89; H, 4.57; N, 1.72; O, 1.96; S, 7.86

Analyzed value: C, 83.86; H, 4.59; N, 1.72; O, 1.96; S, 7.86

### Example 8: Preparation of Compound C-29

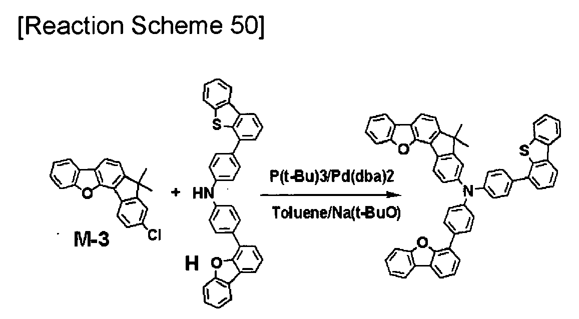

5.0 g (15.68 mmol) of the intermediate M-3, 8.12 g (15.68 mmol) of the intermediate H, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-29 in a white solid of 10.8g (yield: 86%).

Calculated value: C, 85.58; H, 4.66; N, 1.75; 0,4.00; S, 4.01

Analyzed value: C, 85.59; H, 4.67; N, 1.75; 0,4.00; S, 4.01

### Example 9: Preparation of Compound C-33

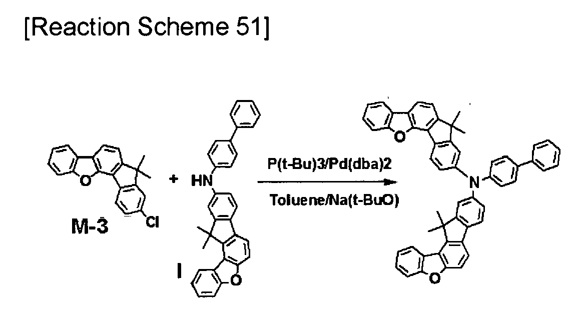

5.0 g (15.68 mmol) of the intermediate M-3, 7.08 g (15.68 mmol) of the intermediate 1, 4.52 g (47.95 mmol) of sodium t-butoxide, and 0.1g (0.47 mmol) of butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-33 in a white solid of 9.4g (yield: 81.6%).

Calculated value: C, 88.37; H, 5.36; N, 1.91; O, 4.36

Analyzed value: C, 88.35; H, 5.38; N, 1.91; O, 4.36

### Example 10: Preparation of Compound C-34

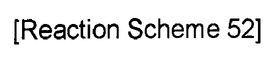

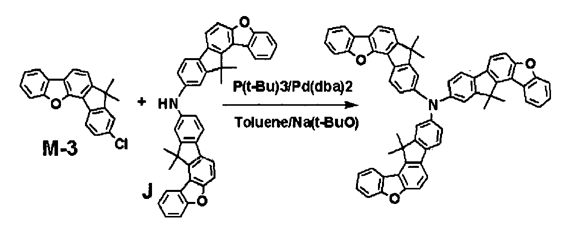

5.0 g (15.68 mmol) of the intermediate M-3, 9.12 g (15.68 mmol) of the intermediate J, 4.52 g (47.95 mmol) of sodium t-butoxide, and 0.1g (0.47 mmol) of tri-tert-butylphosphine were dissolved in 200 ml of toluene, and 0.27g (0.47 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-34 in a white solid of 10.4g (yield: 76.7%).

Calculated value: C, 87.57; H, 5.25; N, 1.62; O, 5.56

Analyzed value: C, 87.59; H, 5.23; N, 1.62; O, 5.56

### Example 11: Preparation of Compound C-2

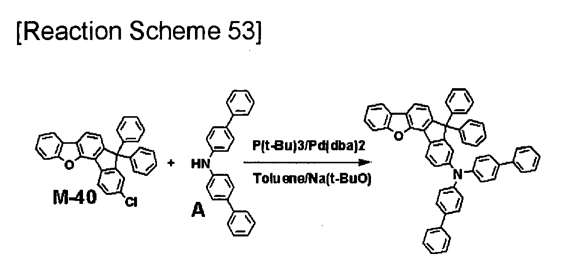

5.0 g (11.29 mmol) of the intermediate M-40, 3.63 g (11.29 mmol) of the intermediate A, 3.25 g (33.87 mmol) of sodium t-butoxide, and 0.07g (0.34 mmol) of tri-tert-butylphosphine were dissolved in 200 ml of toluene, and 0.19g (0.34 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-2 in a white solid of 7.3g (yield: 88.8%).

Calculated value: C, 90.75; H, 5.12; N, 1.92; 0,2.20

Analyzed value: C, 90.73; H, 5.14; N, 1.92; O, 2.20

### Example 12: Preparation of Compound C-35

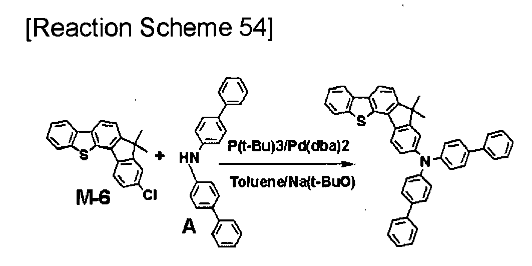

5.0 g (14.93 mmol) of the intermediate M-6, 4.8 g (14.93 mmol) of the intermediate A, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-35 in a white solid of 7.5g (yield: 81 %).

Calculated value: C, 87.20; H, 5.37; N, 2.26; S, 5.17

Analyzed value: C, 87.22; H, 5.35; N, 2.26; S, 5.17

### Example 13: Preparation of Compound C-36

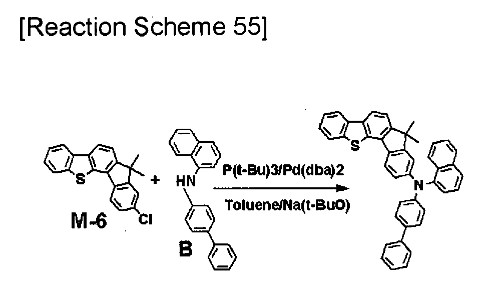

5.0 g (14.93 mmol) of the intermediate M-6, 4.41 g (14.93 mmol) of the intermediate B, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-36, in a white solid of 7.6g (yield: 85.7%).

Calculated value: C, 86.98; H, 5.26; N, 2.36; S, 5.40

Analyzed value: C, 86.99; H, 5.25; N, 2.36; S, 5.40

### Example 14: Preparation of Compound C-37

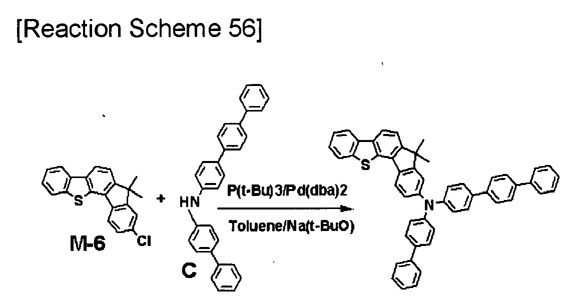

5.0 g (14.93 mmol) of the intermediate M-6, 5.94 g (14.93 mmol) of the intermediate C, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The extracted organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-37 in a white solid of 8.2g (yield: 78.9%).

Calculated value: C, 88.02; H, 5.36; N, 2.01; S, 4.61

Analyzed value: C, 88.00; H, 5.38; N, 2.01; S, 4.61

### Example 15: Preparation of Compound C-8

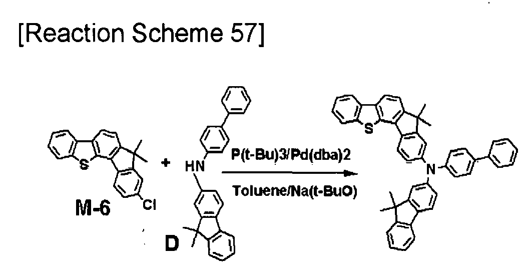

5.0 g (14.93 mmol) of the intermediate M-6, 5.4 g (14.93 mmol) of the intermediate D, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-36, in a white solid of 8.4g (yield: 85.2%).

Calculated value: C, 87.37; H, 5.65; N, 2.12; S, 4.86

Analyzed value: C, 87.35; H, 5.67; N, 2.12; S, 4.86

### Example 16: Preparation of Compound C-20

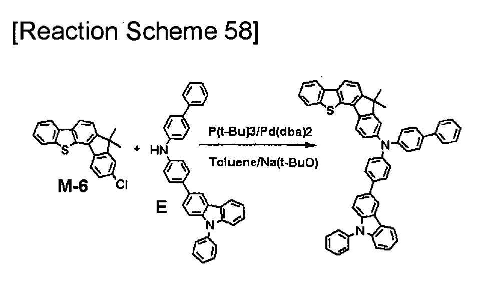

5.0 g (14.93 mmol) of the intermediate M-6, 7.27 g (14.93 mmol) of the intermediate E, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The extracted organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-20 in a white solid of 9.4g (yield: 80.2%).

Calculated value: C, 87.21; H, 5.14; N, 3.57; S, 4.08

Analyzed value: C, 87.23; H, 5.12; N, 3.57; S, 4.08

### Example 17: Preparation of Compound C-26

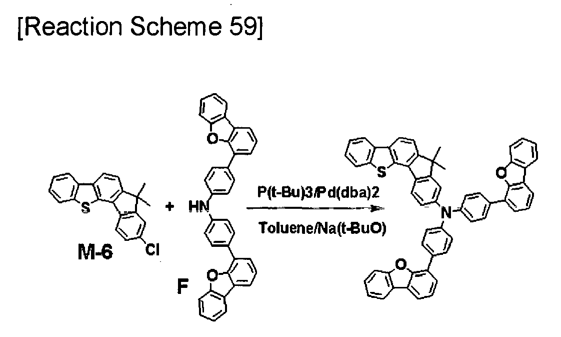

5.0 g (14.93 mmol) of the intermediate M-6, 4.41 g (14.93 mmol) of the intermediate F, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered and concentrated under a reduced pressure. The resulting product was purified through a silica gel column chromatography using n-hexane/dichloromethane mixed in a volume ratio of 2 : 1, obtaining a desired compound C-26, in a white solid of 9.7g (yield: 81.2%).

Calculated value: C, 85.58; H, 4.66; N, 1.75; O, 4.00; S, 4.01

Analyzed value: C, 85.59; H, 4.65; N, 1.78; O, 4.00; S, 4.01

### Example 18: Preparation of Compound C-28

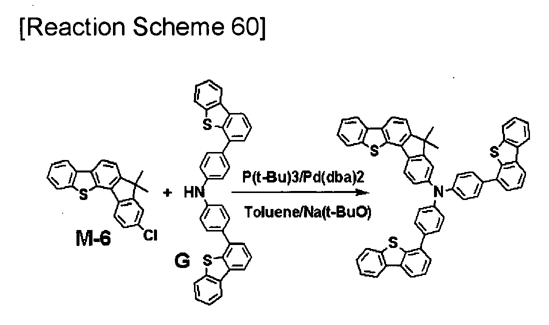

5.0 g (14.93 mmol) of the intermediate M-6, 7.97 g (14.93 mmol) of the intermediate G, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant as extracted with toluene and distilled water. The obtaine organic layer was dried with anhydrous magnesium sulfate, filtered, and concentrated under a reduces pressure. The resulting product was purified through a silica gel column chromatography using n-hexane/dichloromethane mixed in a volume ratio of 2 :1, obtaining a desired compound C-28, in a white solid of 10.3g (yield 82.9%).

Calculated value: C, 82.27; H, 4.48; N, 1.68; S, 11.56

Analyzed value: C, 82.25; H, 4.49; N, 1.68; S, 11.56

### Example 19: Preparation of Compound C-30

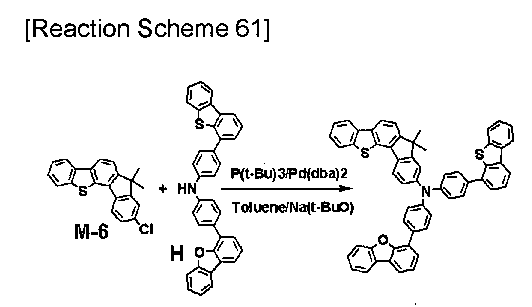

5.0 g (14.93 mmol) of the intermediate M-6, 7.73 g (14.93 mmol) of the intermediate B, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-30, in a white solid of 10.3g (yield 84.5%).

Calculated value: C, 83.89; H, 4.57; N, 1.72; O, 1.96; S, 7.86

Analyzed value: C, 83.86; H, 4.60; N, 1.72; O, 1.96; S, 7.86

### Example 20: Preparation of Compound C-38

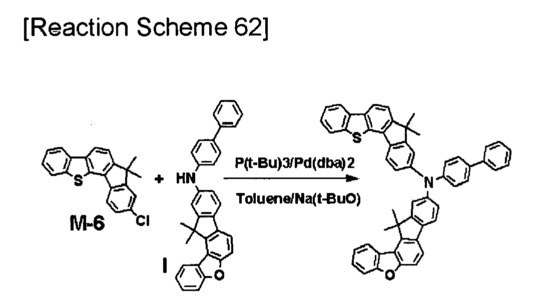

5.0 g (14.93 mmol) of the intermediate M-6, 6.74 g (14.93 mmol) of the intermediate 1, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-38, in a white solid of 9.4g (yield 83.9%).

Calculated value: C, 86.48; H, 5.24; N, 1.87; O, 2.13; S, 4.28

Analyzed value: C, 86.45; H, 5.27; N, 1.87; O, 2.13; S, 4.28

### Example 21: Preparation of Compound C-39

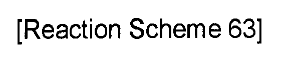

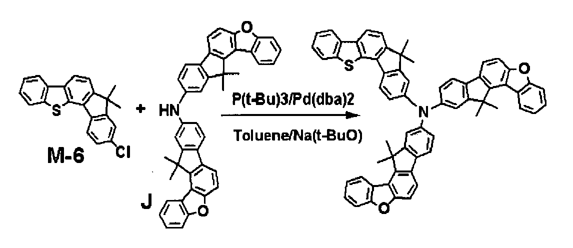

5.0 g (14.93 mmol) of the intermediate M-6, 8.69 g (14.93 mmol) of the intermediate J, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The extracted organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-38 in a white solid of 10.4g (yield: 79.1 %).

Calculated value: C, 85.98; H, 5.15; N, 1.59; O, 3.64; S, 3.64

Analyzed value: C, 85.97; H, 5.17; N, 1.59; O, 3.64; S, 3.64

### Example 22: Preparation of Compound C-40

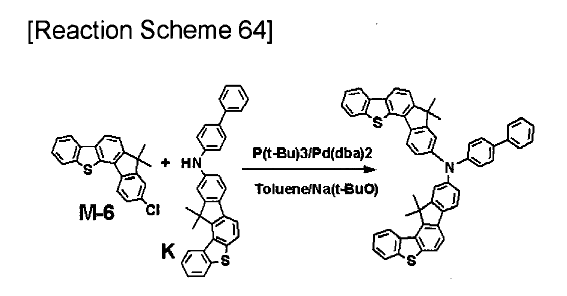

5.0 g (14.93 mmol) of the intermediate M-6, 6.98 g (14.93 mmol) of the intermediate K, 4.31 g (44.79 mmol) of sodium t-butoxide, and 0.09g (0.45 mmol) of tri-tert-butylphosphine were dissolved in 200 ml of toluene, and 0.26g (0.45 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-40, in a white solid of 8.7g (yield 80.2%).

Calculated value: C, 84.38; H, 4.86; N, 1.93; S, 8.83

Analyzed value: C, 84.37; H, 4.87; N, 1.93; S, 8.83

### Example 23: Preparation of Compound C-9

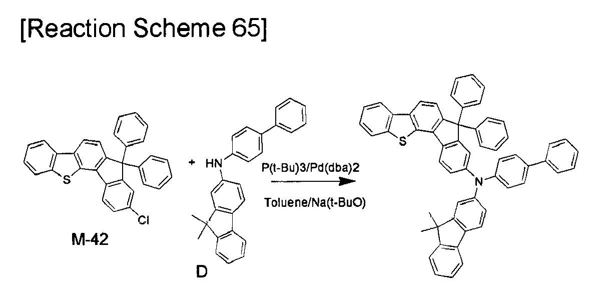

5.0 g (10.86 mmol) of the intermediate M-42, 3.94 g (10.86 mmol) of the intermediate D, 3.14 g (32.68 mmol) of sodium t-butoxide, and 0.07g (0.33 mmol) of tri-tert-butylphosphine were dissolved in 200 ml of toluene, and 0.19g (0.33 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-9, in a white solid of 7.4g (yield 86.6%).

Calculated value: C, 88.85; H, 5.27; N, 1.79; S, 4.09

Analyzed value: C, 88.83; H, 5.29; N, 1.79; S, 4.09

### Example 24: Preparation of Compound C-41

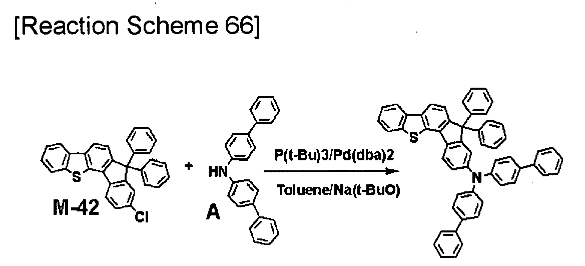

5.0 g (10.86 mmol) of the intermediate M-42, 3.5 g (10.86 mmol) of the intermediate A, 3.14 g (32.68 mmol) of sodium t-butoxide, and 0.07g (0.33 mmol) of tri-tert-butylphosphine were dissolved in 200 ml of toluene, and 0.19g (0.33 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound C-41, in a white solid of 6.9g (yield: 85.1 %).

Calculated value: C, 88.79; H, 5.01; N, 1.88; S, 4.31

Analyzed value: C, 88.77; H, 5.03; N, 1.88; S, 4.31

### Example 25: Preparation of Compound A-28

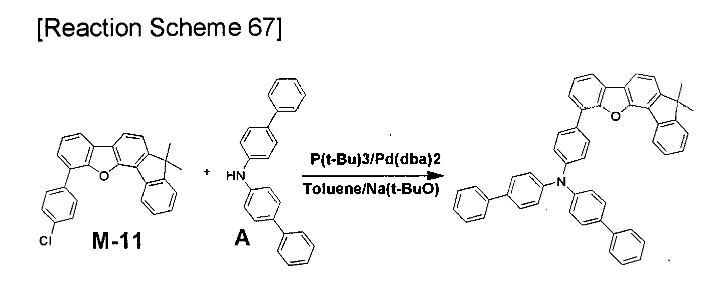

5.0 g (12.66 mmol) of the intermediate M-11, 4.07 g (12.66 mmol) of the intermediate A, 3.65 g (37.99 mmol) of sodium t-butoxide, and 0.08g (0.38 mmol) of tri-tert-butylphosphine were dissolved in 200 ml of toluene, and 0.22g (0.38 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound A-28, in a white solid of 7.3g (yield: 84.8%).

Calculated value: C, 90.10; H, 5.49; N, 2.06; O, 2.35

Analyzed value: C, 90.12; H, 5.47; N, 2.06; O, 2.35

### Example 26: Preparation of Compound A-30

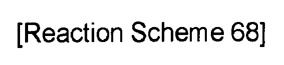

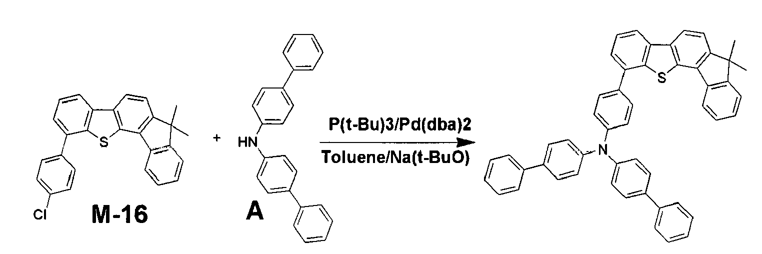

5.0 g (12.17 mmol) of the intermediate M-16, 3.91 g (12.17 mmol) of the intermediate A, 3.65 g (37.99 mmol) of sodium t-butoxide, and 0.07g (0.36 mmol) of tri-tert-butylphosphine were dissolved in 200 ml of toluene, and 0.22g (0.38 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound A-30, in a white solid of 7.1 g (yield: 83.8%).

Calculated value: C, 88.02; H, 5.36; N, 2.01; S, 4.61

Analyzed value: C, 88.04; H, 5.34; N, 2.01; S, 4.61

### Example 27: Preparation of Compound B-19

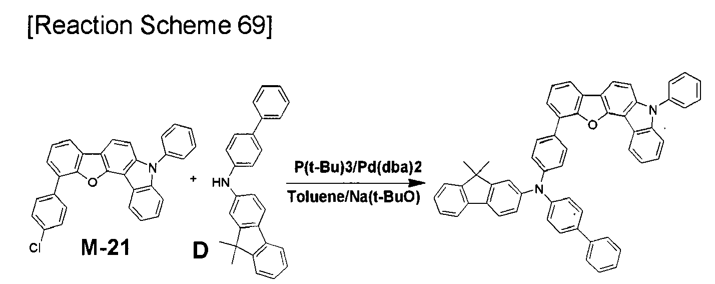

5.0 g (11.26 mmol) of the intermediate M-21, 4.07 g (11.26 mmol) of the intermediate D, 3.25 g (33.79 mmol) of sodium t-butoxide, and 0.07g (0.34 mmol) of tri-tert-butylphosphine were dissolved in 200 ml of toluene, and 0.19g (0.34 mmol) of Pd(dba)₂ was added thereto. The mixture was refluxed and agitated under a nitrogen atmosphere for 12 hours. When the reaction was complete, the reactant was extracted with toluene and distilled water. The extracted organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound B-19 in a white solid of 7.2g (yield: 83.1 %).

Calculated value: C, 89.03; H, 5.24; N, 3.64; O, 2.08

Analyzed value: C, 89.05; H, 5.22; N, 3.64; O, 2.08

### Example 28: Preparation of Compound B-20

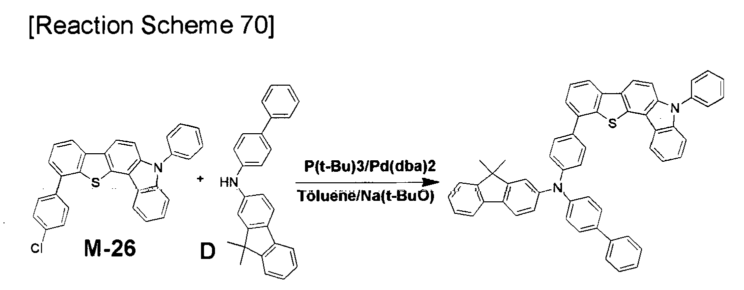

5.0 g (10.87 mmol) of the intermediate M-26, 3.93 g (10.87 mmol) of the intermediate D, 3.25 g (33.79 mmol) of sodium t-butoxide, and 0.07g (0.34 mmol) of tri-tert-butylphosphine were dissolved in 200 ml of toluene, and 0.19g (0.34 mmol) of Pd(dba)₂ was added thereto. The mixture was refluxed and agitated under a nitrogen atmosphere for 12 hours. When the reaction was complete, the reactant was extracted with toluene and distilled water. The obtained organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound B-20, in a white solid of 7.3g (yield 85.5%).

Calculated value: C, 87.21; H, 5.14; N, 3.57; S, 4.08

Analyzed value: C, 87.24; H, 5.11; N, 3.57; S, 4.08

### Example 29: Preparation of Compound D-09

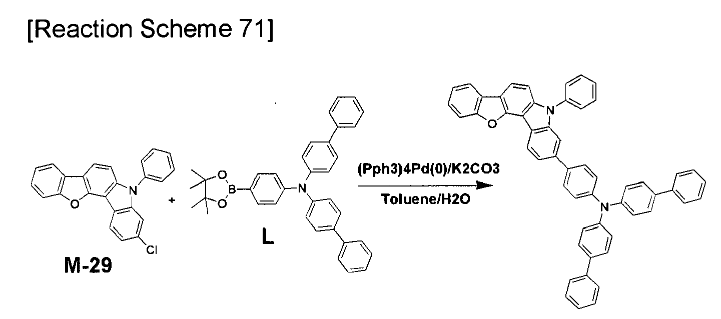

3.86 g (10.51 mmol) of the intermediate M-29, 5 g (9.55 mmol) of the intermediate L, and 0.33 g (0.29 mmol) of tetrakistriphenyl phosphine palladium were dissolved in 200 ml of toluene in a 500mL round-bottomed flask under a nitrogen atmosphere, and 2.64 g (19.1 mmol) of potassium acetate and 100 Mℓ of water were added thereto. The mixture was refluxed at 90 °C for 24 hours. When the reaction was complete, the reactant was extracted with toluene and distilled water. The extracted organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound D-09 in a white solid of 5.4 g (yield: 77.5 %).

Calculated value: C, 88.98; H, 4.98; N, 3.84; O, 2.20

Analyzed value: C, 88.99; H, 4.97; N, 3.84; O, 2.20

### Example 30: Preparation of Compound D-10

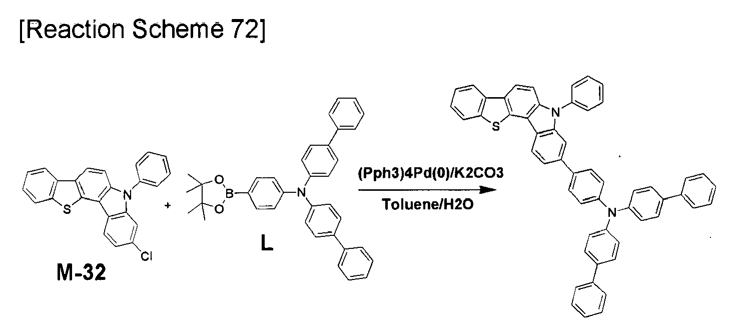

4.03 g (10.51 mmol) of the intermediate M-32, 5 g (9.55 mmol) of the intermediate L, and 0.33 g (0.29 mmol) of tetrakistriphenyl phosphine palladium were dissolved in 200 ml of toluene in a 500mL round-bottomed flask under a nitrogen atmosphere, and 2.64 g (19.1 mmol) of potassium acetate and 100 Mℓ of water were added thereto. The mixture was refluxed at 90 °C for 24 hours. When the reaction was complete, the reactant was extracted with toluene and distilled water. The extracted organic layer was dried with anhydrous magnesium sulfate, filtered, and concentrated under a reduced pressure. The resulting product was purified through a silica gel column chromatography using n-hexane/dichloromethane mixed in a volume ratio of 2 :1, obtaining a desired compound D-10 in a white solid of 5.5 g (yield: 77.3 %).

Calculated value: C, 87.06; H, 4.87; N, 3.76; S, 4.30

Analyzed value: C, 87.04; H, 4.89; N, 3.76; S, 4.30

### Fabrication of Organic light-emitting diode

### Example 31: Fabrication of Organic photoelectric device

A glass substrate coated with ITO (Indium tin oxide) as a 1500Å-thick thin film was cleaned with distilled water ultrasonic wave. Next, the glass substrate was cleaned with ultrasonic wave using a solvent such as isopropyl alcohol, acetone, methanol, and the like and dried. The dried substrate was moved in a plasma cleaner and cleaned with oxygen plasma for 5 minutes and then, moved in a vacuum depositor. This ITO transparent electrode was used as a positive electrode, and 4,4'-bis[N-[4-{N,N-bis(3-methylphenyl)amino}-phenyl]-N-phenylamino]biphenyl (DNTPD) was vacuum deposited to form a 60 nm-thick hole injection layer (HIL) thereon. Then, the compound according to Example 1 was vacuum-deposited to form a 30 nm-thick hole transport layer (HTL). On the hole transport layer (HTL), a 25 nm-thick emission layer was vacuum-deposited with 9,10-di-(2-naphthyl)anthracene (ADN) as a host and doped with 3wt% of 2,5,8,11-tetra(tert-butyl)perylene (TBPe) as a dopant.

On the emission layer, Alq3 was vacuum deposited to form a 25 nm-thick electron transport layer (ETL). On the electron transport layer (ETL), a 10 nm-thick LiF layer and a 100 nm-thick Al layer were sequentially vacuum deposited to fabricate a cathode. Then, the cathode was used to fabricate an organic light emitting diode.

The organic light emitting diode has a structure of five thin organic layers, in particular,
100 nm Al/ 1 nm LiF/ 25 nm Alq3/ 25 nm EML[ADN:TBPe=97:3]/ 30 nm C-1 /60 nm DNTPD /150 nm ITO.

### Example 32

An organic light emitting diode was fabricated according to the same method as Example 31 by using the C-32 of Example 3 instead of the C-1 of Example 1.

### Example 33

An organic light emitting diode was fabricated according to the same method as Example 31 by using the C-5 of Example 4 instead of the C-1 of Example 1.

### Example 34

An organic light emitting diode was fabricated according to the same method as Example 31 by using the C-19 of Example 5 instead of the C-1 of Example 1.

### Example 35

An organic light emitting diode was fabricated according to the same method as Example 31 by using the C-2 of Example 11 instead of the C-1 of Example 1.

### Example 36

An organic light emitting diode was fabricated according to the same method as Example 31 by using the C-35 of Example 12 instead of the C-1 of Example 1.

### Example 37

An organic light emitting diode was fabricated according to the same method as Example 31 by using the C-37 of Example 14 instead of the C-1 of Example 1.

### Example 38

An organic light emitting diode was fabricated according to the same method as Example 31 by using the C-8 of Example 15 instead of the C-1 of Example 1.

### Example 39

An organic light emitting diode was fabricated according to the same method as Example 31 by using the C-20 of Example 16 instead of the C-1 of Example 1.

### Example 40

An organic light emitting diode was fabricated according to the same method as Example 31 by using the C-9 of Example 23 instead of the C-1 of Example 1.

### Example 41

An organic light emitting diode was fabricated according to the same method as Example 31 by using the C-41 of Example 24 instead of the C-1 of Example 1.

### Comparative Example 1

An organic light emitting diode was fabricated according to the same method as Example 31 except for using NPB instead of the C-1 of Example 1. The NPB structure is shown below.

### Comparative Example 2

An organic light emitting diode was fabricated according to the same method as Example 31 except for using HT1 instead of the C-1 of Example 1. The HT1 structure is shown below.

The DNTPD, ADN, TBPe, NPB, and HT1 structures used to fabricate an organic light emitting diode are shown below.

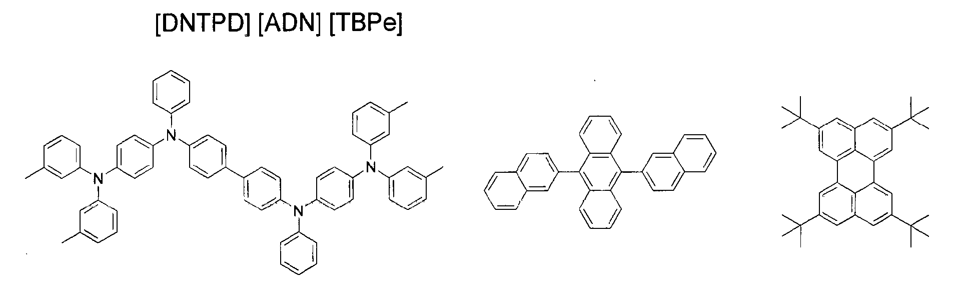

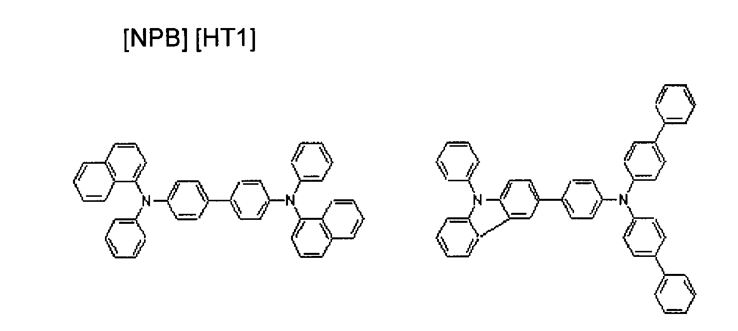

### (Performance Evaluation of Organic light emitting diode)

Each organic light emitting diode according to Examples 31 to 41 and Comparative Example were measured regarding current density change, luminance change, and luminous efficiency depending on voltage. A method of measuring the above values is as follows. The results are provided in the following Table 1.

### (1) Current density change depending on voltage change

The organic light emitting diodes were measured regarding current flowing in a unit device by using a current-voltage meter (Keithley 2400) while their voltages were increased from 0 V to 10 V. The current value was divided by an area to obtain a current density.

### (2) Luminance change depending on voltage change

The organic light emitting diodes were measured regarding luminance by using a luminance meter (Minolta Cs-1000A), while their voltage were increased from 0 V to 10 V.

### (3) Luminous efficiency

The luminance and current density obtained from the above (1) and (2) and voltage were used to calculate current efficiency(cd/A) at the same current density (10 mA/cm²).

**[Table 1]**

| Devices | Compound of hole transport layer (HTL) | driving voltage (V) | Color (EL color) | Efficiency (cd/A) |
|---|---|---|---|---|
| Example 31 | C-1 | 6.3 | Blue | 6.3 |
| Example 32 | C-32 | 6.3 | Blue | 6.2 |
| Example 33 | C-5 | 6.2 | Blue | 6.0 |
| Example 34 | C-19 | 6.2 | Blue | 6.0 |
| Example 35 | C-2 | 6.4 | Blue | 6.1 |
| Example 36 | C-35 | 6.2 | Blue | 6.2 |
| Example 37 | C-37 | 6.3 | Blue | 6.3 |
| Example 38 | C-8 | 6.4 | Blue | 6.3 |
| Example 39 | C-20 | 6.5 | Blue | 6.2 |
| Example 40 | C-9 | 6.3 | Blue | 6.3 |
| Example 41 | C-41 | 6.4 | Blue | 6.2 |
| Comparative Example 1 | NPB | 7.1 | Blue | 4.9 |
| Comparative Example 2 | HT1 | 6.6 | Blue | 5.7 |

| | | | | |
|---|---|---|---|---|
| Current density: 10mA/cm² | | | | |

As shown in Table 1, an organic light emitting diode according to one embodiment of the present invention had low driving voltage but excellent efficiency compared with the organic light emitting diode according to Comparative Examples 1 and 2.

### <Description of symbols>

- 100:: organic light emitting diode
- 105:: organic thin layer
- 110:: cathode
- 120:: anode
- 130:: emission layer
- 140:: hole transport layer (HTL)
- 150:: electron transport layer (ETL)
- 160:: electron injection layer (EIL)
- 170:: hole injection layer (HIL)
- 230:: emission layer + electron transport layer (ETL)

## Claims

1. A compound for an organic optoelectronic device, which is represented by the following Chemical Formula 4 and 5 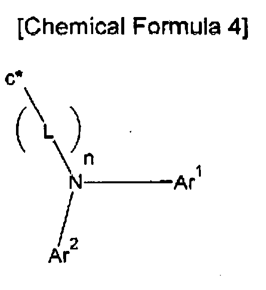 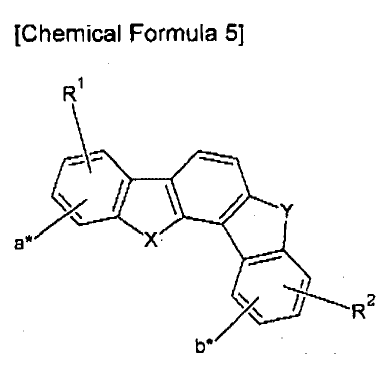 wherein in Chemical Formula 4 and 5,
X is O, S, SO₂(O=S=O), PO(P=O) or CO(C-O),
Y is CR'R" or NR',
R', R" , R¹ and R² are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof,
Ar¹ and Ar² are the same or different and independently a substituted or unsubstituted C6 to C30 aryl group, or a substituted or unsubstituted C2 to C30 heteroaryl group,
L is a single bond, a substituted or unsubstituted C2 to C6 alkenylene group, a substituted or unsubstituted C2 to C6 alkynylene group, a substituted or unsubstituted C6 to C30 arylene group, a substituted or unsubstituted heteroarylene group, or a combination thereof,
n is 0 or 1, and
the a* or b* of the above Chemical Formula 5 has a sigma bonding with the c* of the above Chemical Formula 4, but the rest of a* or b* with no connection to the c* are hydrogen.

2. The compound of claim 1, which is represented by the following Chemical Formula 6: 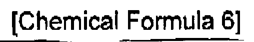 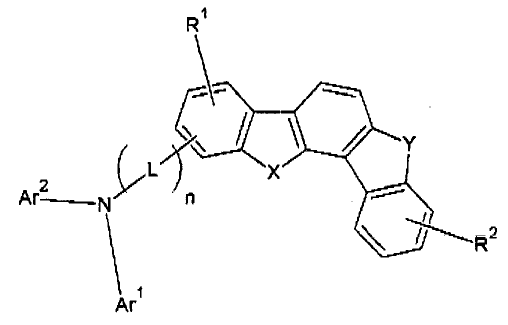 wherein, in Chemical Formula 6,
X is O, S, SO₂ (O=S=O), PO(P=O) or CO(C=O),
Y is CR'R" or NR',
R', R" , R¹ and R² are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof,
Ar¹ and Ar² are the same or different and independently a substituted or unsubstituted C6 to C30 aryl group, or a substituted or unsubstituted C2 to C30 heteroaryl group,
L is a single bond, a substituted or unsubstituted C2 to C6 alkenylene group, a substituted or unsubstituted C2 to C6 alkynylene group, a substituted or unsubstituted C6 to C30 arylene group, a substituted or unsubstituted heteroarylene group, or a combination thereof, and
the n is 0 or 1.

3. The compound of claim 1 or 2 which is represented by the following Chemical Formula 7: 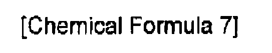 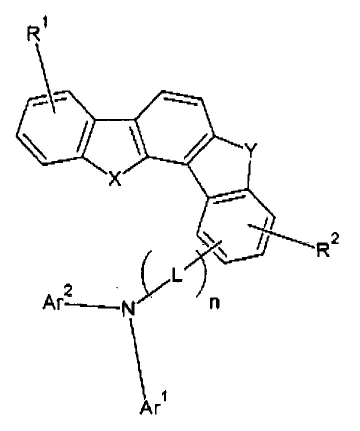 wherein, in Chemical Formula 7,
X is O, S, SO₂(O=S=O), PO(P=O) or CO(C=O)
Y is CR'R" or NR',
R', R", R¹, and R² are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof,
Ar¹ and Ar² are the same or different and independently a substituted or unsubstituted C6 to C30 aryl group, or a substituted or unsubstituted C2 to C30 heteroaryl group,
L is a single bond, a substituted or unsubstituted C2 to C6 alkenylene group, a substituted or unsubstituted C2 to C6 alkynylene group, a substituted or unsubstituted C6 to C30 arylene group, a substituted or unsubstituted heteroarylene group, or a combination thereof, and
n is 0 or 1.

4. The compound of claim 2, wherein:
Y is CR'R", and
R', R" are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

5. The compound of claim 2, wherein:
Y is NR', and
R' is hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

6. The compound of claim 3, wherein:
Y is CR'R", and
R', R" are the same or different and independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 an arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

7. The compound of claim 3, wherein:
Y is NR', and
R' is hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 an alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof.

8. The compound of any of the claims 1 to 7, which is represented by one of the following Chemical Formulas A-1 to A-51:
[Chemical Formula A-1] [Chemical Formula A-2]
[Chemical Formula A-3] [Chemical Formula A-4] 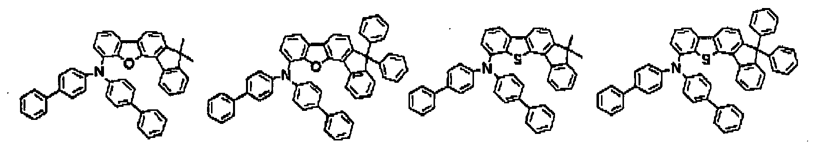
[Chemical Formula A-5] [Chemical Formula A-6]
[Chemical Formula A-7] [Chemical Formula A-8] 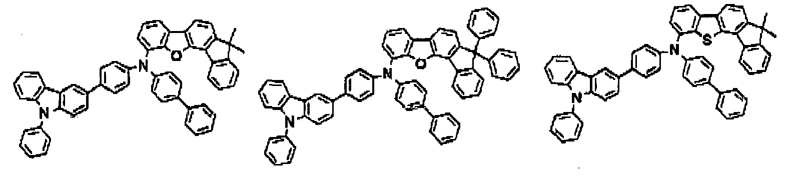
[Chemical Formula A-9] [Chemical Formula A-10] 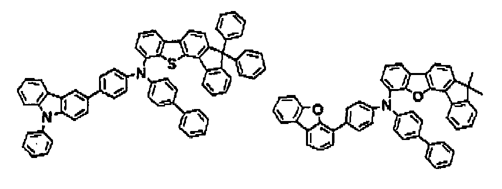
[Chemical Formula A-11] [Chemical Formula A-12]
[Chemical Formula A-13] 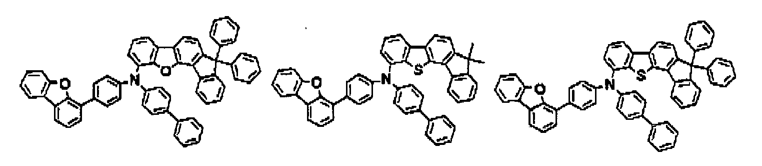
[Chemical Formula A-14] [Chemical Formula A-15]
[Chemical Formula A-16] 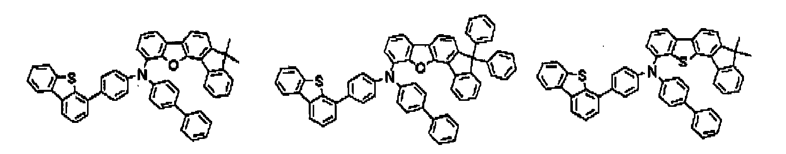
[Chemical Formula A-17] [Chemical Formula A-18] 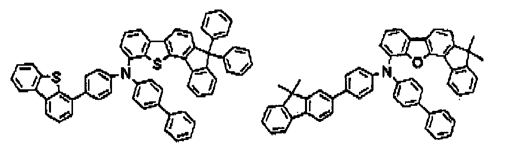
[Chemical Formula A-19] [Chemical Formula A-20]
[Chemical Formula A-21] 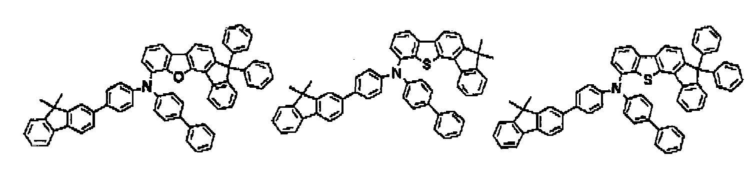
[Chemical Formula A-22] [Chemical Formula A-23] 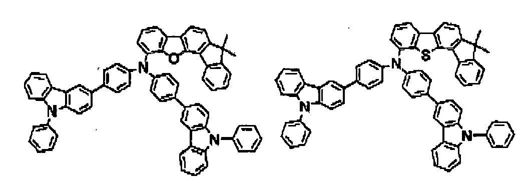
[Chemical Formula A-24] [Chemical Formula A-25]
[Chemical Formula A-26] [Chemical Formula A-27] 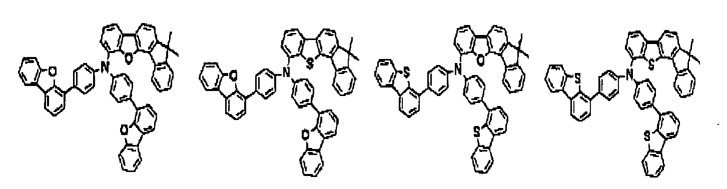
[Chemical Formula A-28] [Chemical Formula A-29]
[Chemical Formula A-30] [Chemical Formula A-31] 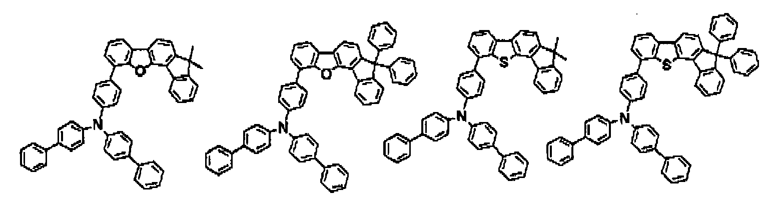
[Chemical Formula A-32] [Chemical Formula A-33]
[Chemical Formula A-34] [Chemical Formula A-35] 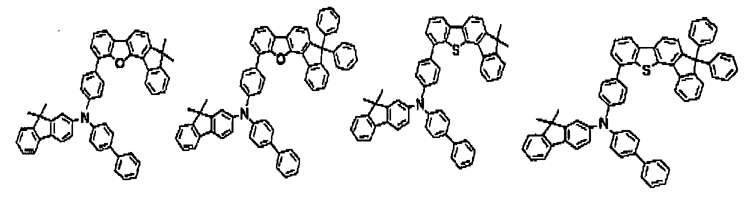
[Chemical Formula A-36] [Chemical Formula A-37]
[Chemical Formula A-38] [Chemical Formula A-39] 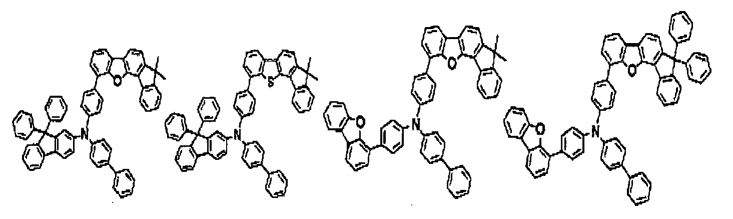
[Chemical Formula A-40] [Chemical Formula A-41] 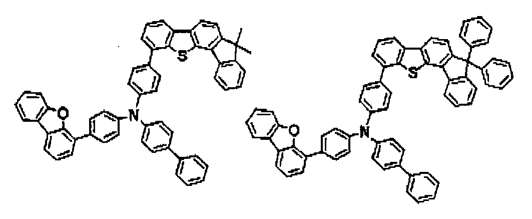
[Chemical Formula A-42] [Chemical Formula A-43]
Chemical Formula A-44] [Chemical Formula A-45] 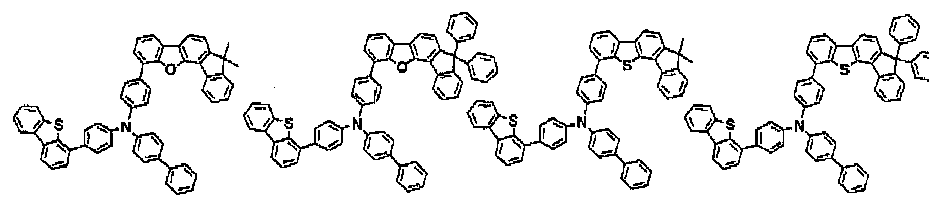
[Chemical Formula A-46] [Chemical Formula A-47]
[Chemical Formula A-48] [Chemical Formula A-49] 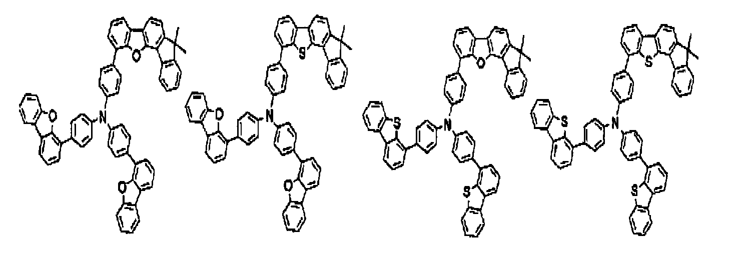
[Chemical Formula A-50] [Chemical Formula A-51]

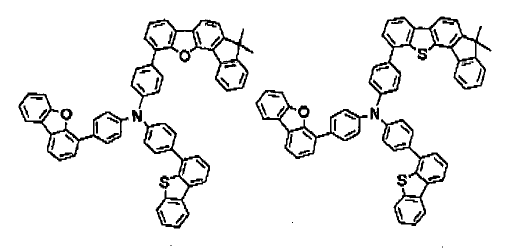

9. The compound of any of the claims 1 to 8, which is represented by one of the following Chemical Formulas B-1 to B-32:
[Chemical Formula B-1] [Chemical Formula B-2]
[Chemical Formula B-3] [Chemical Formula B-4] 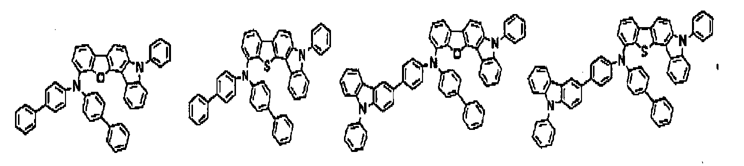
[Chemical Formula B-5] [Chemical Formula B-6]
[Chemical Formula B-7] [Chemical Formula B-8] 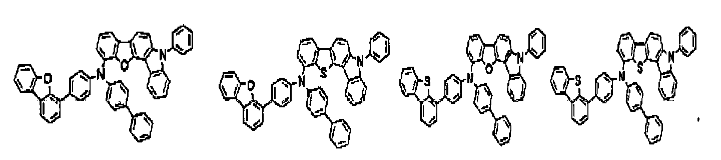
[Chemical Formula B-9] [Chemical Formula B-10]
[Chemical Formula B-11] 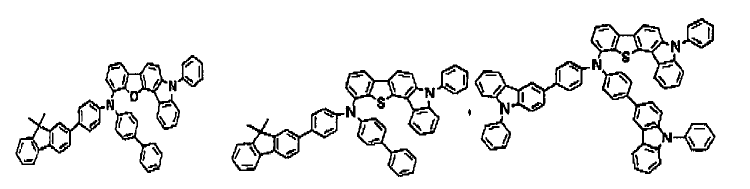
[Chemical Formula B-12] [Chemical Formula B-13]
[Chemical Formula B-14] 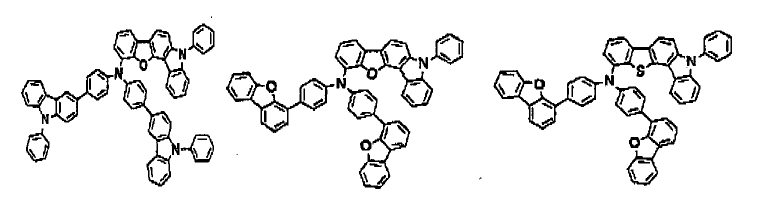
[Chemical Formula B-15] [Chemical Formula B-16]
[Chemical Formula B-17] [Chemical Formula B-18] 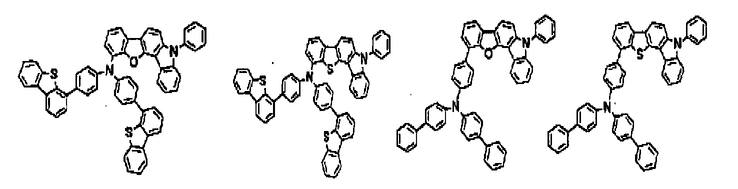
[Chemical Formula B-19] [Chemical Formula B-20]
[Chemical Formula B-21] [Chemical Formula B-22] 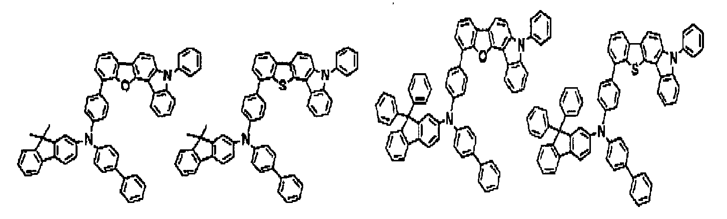
[Chemical Formula B-23] [Chemical Formula B-24]
[Chemical Formula B-25] [Chemical Formula B-26] 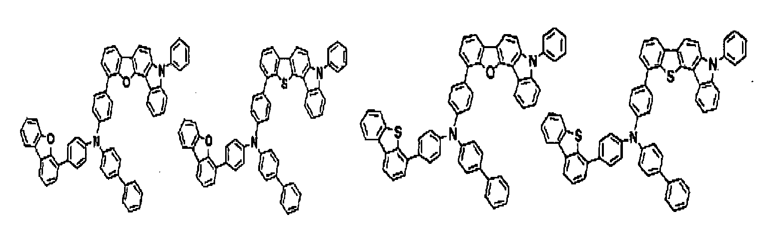
[Chemical Formula B-27] [Chemical Formula B-28]
[Chemical Formula B-29] [Chemical Formula B-30] 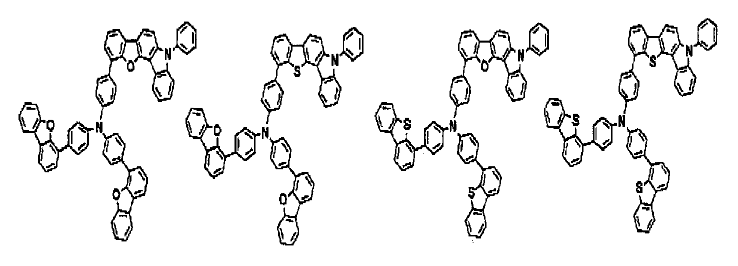
[Chemical Formula B-31] [Chemical Formula B-32]

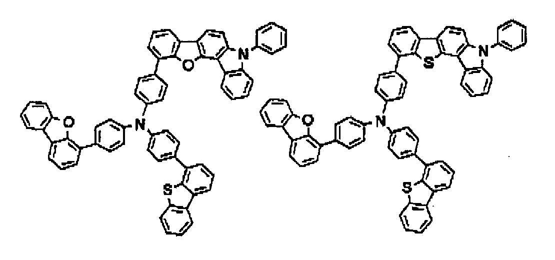

10. The compound of any of the claims 1 to 9, which is represented by one of the following Chemical Formula C-1 to C-41:
[Chemical Formula C-1] [Chemical Formula C-2] 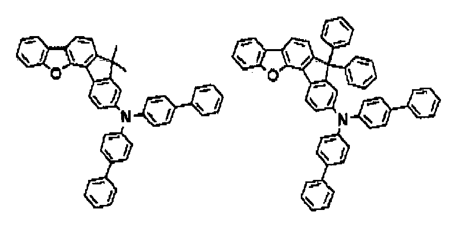
[Chemical Formula C-3] [Chemical Formula C-4]
[Chemical Formula C-5] [Chemical Formula C-6]
[Chemical Formula C-7] 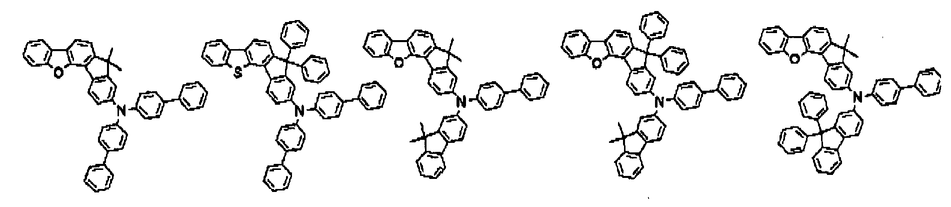
[Chemical Formula C-8] [Chemical Formula C-9]
[Chemical Formula C-10] 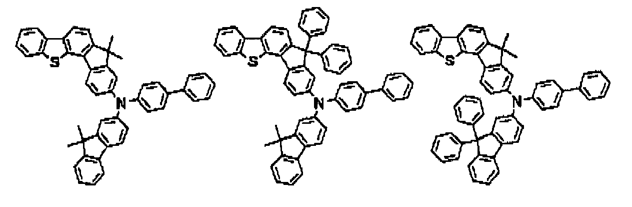
[Chemical Formula C-11] [Chemical Formula C-12]
[Chemical Formula C-13] [Chemical Formula C-14] 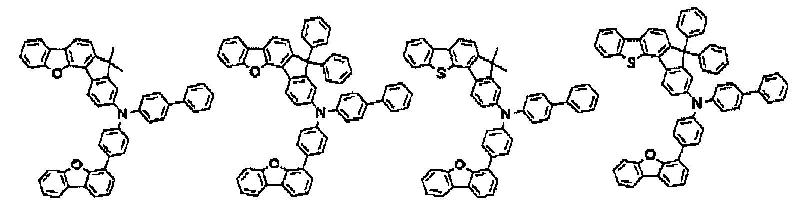
[Chemical Formula C-15] [Chemical Formula C-16]
[Chemical Formula C-17] [Chemical Formula C-18] 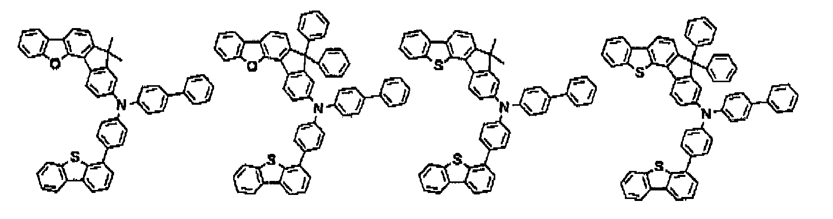
[Chemical Formula C-19] [Chemical Formula C-20]
[Chemical Formula C-21] [Chemical Formula C-22] 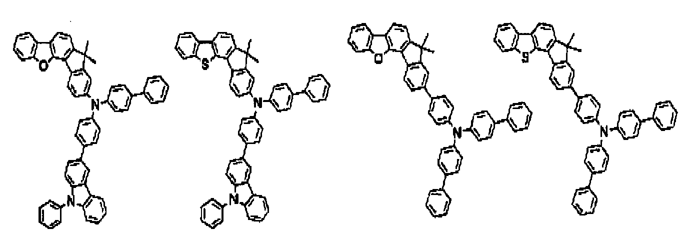
[Chemical Formula C-23] [Chemical Formula C-24]
[Chemical Formula C-25] [Chemical Formula C-26] 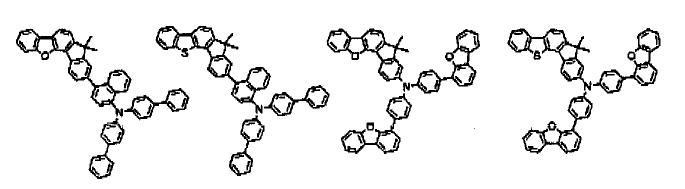
[Chemical Formula C-27] [Chemical Formula C-28]
[Chemical Formula C-29] [Chemical Formula C-30] 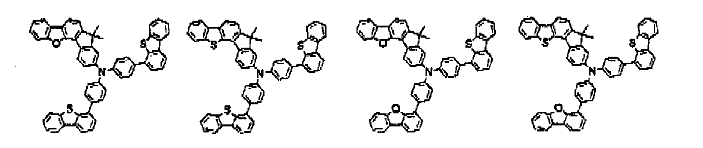
[Chemical Formula C-31] [Chemical Formula C-32]
[Chemical Formula C-33] 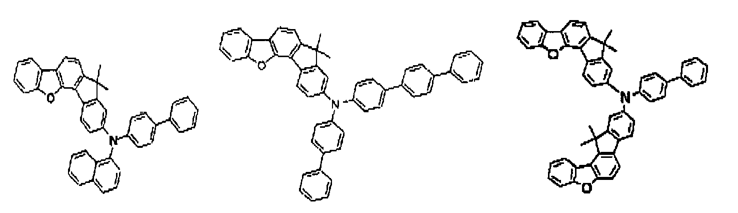
[Chemical Formula C-34] [Chemical Formula C-35]
[Chemical Formula C-36] 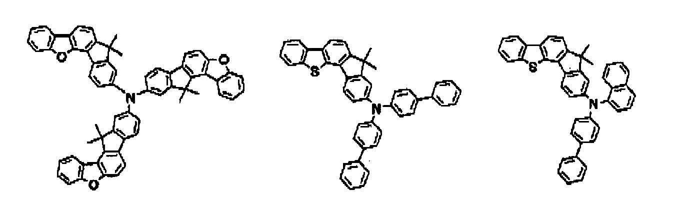
[Chemical Formula C-37] [Chemical Formula C-38]
[Chemical Formula C-39] 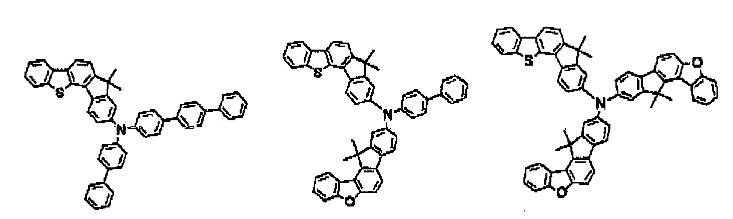
[Chemical Formula C-40] [Chemical Formula C-41]

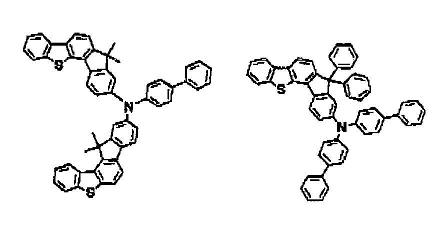

11. The compound of any of the claims 1 to 10, which is represented by one of the following Chemical Formulas D-1 to D-20:
[Chemical Formula D-1] [Chemical Formula D-2]
[Chemical Formula D-3] [Chemical Formula D-4] 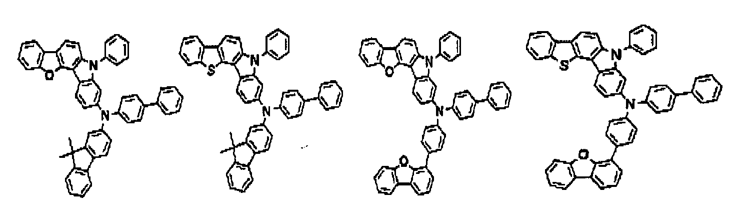
[Chemical Formula D-5] [Chemical Formula D-6]
[Chemical Formula D-7] 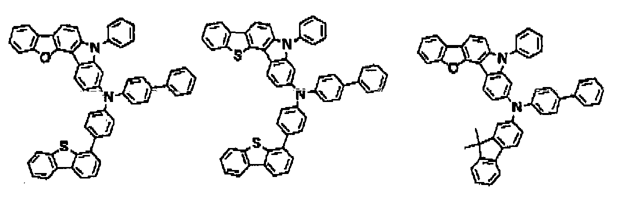
[Chemical Formula D-8] [Chemical Formula D-9]
[Chemical Formula D-10] [Chemical Formula D-11]
[Chemical Formula D-12] 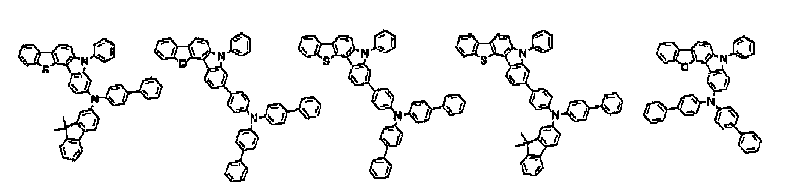
[Chemical Formula D-13] [Chemical Formula D-14]
[Chemical Formula D-15] [Chemical Formula D-16]
[Chemical Formula D-17] 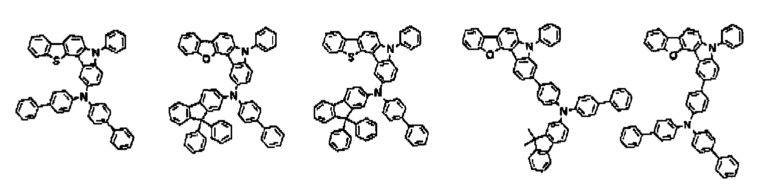
[Chemical Formula D-18] [Chemical Formula D-19]
[Chemical Formula D-20]

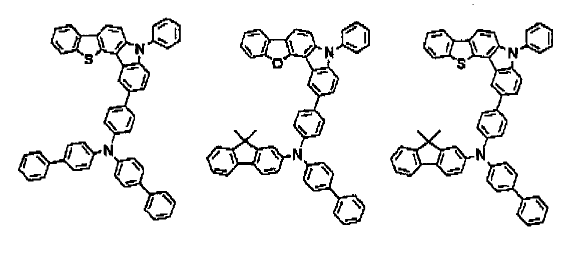

12. Use of a compound of any of the claims 1 to 11 in an organic optoelectronic device, wherein the organic optoelectronic device is selected from the group consisting of an organic photoelectronic device, an organic light emitting diode, an organicsolar cell, an organic transistor, an organic photoconductor drum, and an organic memory device.

13. Use of a compound of any of the claims 1 to 11 as a hole transport material or a hole injection material for an organic light emitting diode.

14. An organic light emitting diode comprising an anode, a cathode, and more than one organic thin layer interposed between the anode and the cathode, and
wherein at least one layer among the organic thin layers comprises the compound for an organic optoelectronic device according to claims 1 to 11.

## Patentansprüche

1. Eine Verbindung für eine organisch opto-elektronische Vorrichtung, welche durch die folgenden chemischen Formeln 4 und 5 wiedergegeben wird 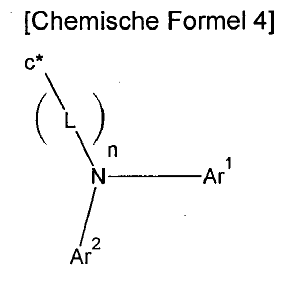 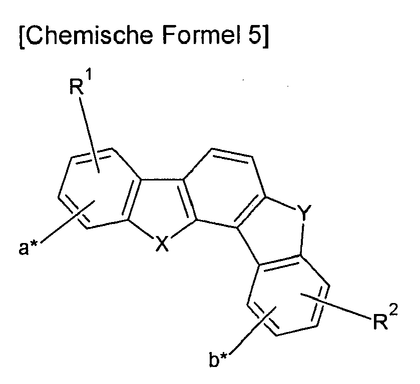 wobei in der chemischen Formel 4 und 5
X ist O, S, SO₂(O=S=O), PO(P=O) oder CO(C=O),
Y ist CR'R" oder NR',
R', R" , R¹ und R² sind gleich oder unterschiedlich und unabhängig voneinander Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierte C6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyclothiol-Gruppe, eine substituierte oder unsubstituierte C1 zu C20 Ureid-Gruppe, eine substituierte oder unsubstituierte C3 zu C40 Silyl-Gruppe, oder eine Kombination derselben,
Ar¹ und Ar² sind gleich oder unterschiedlich und unabhängig voneinander eine substituierte oder nicht substituierte C6 zu C30 Aryl-Gruppe oder eine substituierte oder nicht substituierte C2 zu C30 Heteroaryl-Gruppe, L ist eine Einfachbindung, eine substituierte oder nicht substituierte C2 zu C6 Alkenyl-Gruppe, eine substituierte oder nicht substituierte C2 zu C6 Alkynyl-Gruppe, eine substituierte oder nicht substituierte C6 zu C30 Aryl-Gruppe, eine substituierte oder nicht substituierte Heteroaryl-Gruppe, oder eine Kombination derselben,
n ist 0 oder 1,
und das a* und b* der oben dargestellten chemischen Formel 5 weist eine Sigma-Bindung mit dem c* der oben dargestellten chemischen Formel 4 auf, wobei der Rest von a* und b* ohne Anbindung zu dem c* Wasserstoff ist.

2. Die Verbindung nach Anspruch 1, welche durch folgende chemische Formel 6 wiedergegeben wird 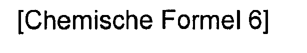 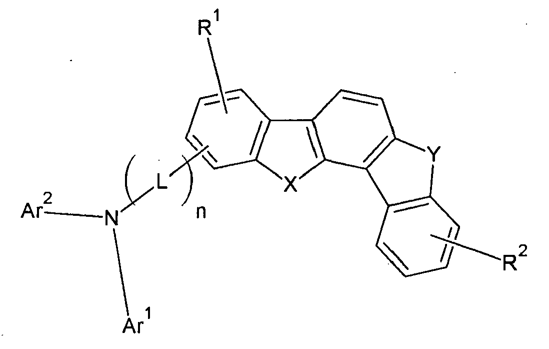 wobei in der chemischen Formel 6
X ist O, S, SO₂(O=S=O), PO(P=O) oder CO(C=O),
Y ist CR'R" oder NR',
R', R " , R¹ und R² sind gleich oder unterschiedlich und unabhängig voneinander Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierte C6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyclothiol-Gruppe, eine substituierte oder unsubstituierte C1 zu C20 Ureid-Gruppe, eine substituierte oder unsubstituierte C3 zu C40 Silyl-Gruppe, oder eine Kombination derselben,
Ar¹ und Ar² sind gleich oder unterschiedlich und unabhängig voneinander eine substituierte oder nicht substituierte C6 zu C30 Aryl-Gruppe oder eine substituierte oder nicht substituierte C2 zu C30 Heteroaryl-Gruppe,
L ist eine Einfachbindung, eine substituierte oder nicht substituierte C2 zu C6 Alkenyl-Gruppe, eine substituierte oder nicht substituierte C2 zu C6 Alkynyl-Gruppe, eine substituierte oder nicht substituierte C6 zu C30 Aryl-Gruppe, eine substituierte oder nicht substituierte Heteroaryl-Gruppe, oder eine Kombination derselben, und
n ist 0 oder 1.

3. Die Verbindung nach Anspruch 1 oder 2, welche durch folgende chemische Formel 7 wiedergegeben wird 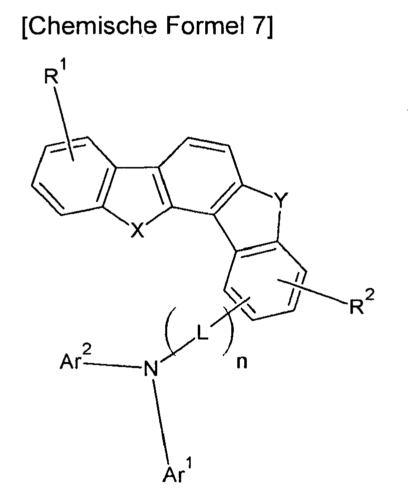 wobei in der chemischen Formel 7
X ist O, S, SO₂(O=S=O), PO(P=O) oder CO(C=O),
Y ist CR'R" oder NR',
R', R", R¹ und R² sind gleich oder unterschiedlich und unabhängig voneinander Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierte C6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyclothiol-Gruppe, eine substituierte oder unsubstituierte C1 zu C20 Ureid-Gruppe, eine substituierte oder unsubstituierte C3 zu C40 Silyl-Gruppe, oder eine Kombination derselben,
Ar¹ und Ar² sind gleich oder unterschiedlich und unabhängig voneinander eine substituierte oder nicht substituierte C6 zu C30 Aryl-Gruppe oder eine substituierte oder nicht substituierte C2 zu C30 Heteroaryl-Gruppe,
L ist eine Einfachbindung, eine substituierte oder nicht substituierte C2 zu C6 Alkenyl-Gruppe, eine substituierte oder nicht substituierte C2 zu C6 Alkynyl-Gruppe, eine substituierte oder nicht substituierte C6 zu C30 Aryl-Gruppe, eine substituierte oder nicht substituierte Heteroaryl-Gruppe, oder eine Kombination derselben, und
n ist 0 oder 1.

4. Die Verbindung nach Anspruch 2, wobei
Y ist CR'R",
R', R" sind gleich oder unterschiedlich und unabhängig voneinander Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierte C6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyclothiol-Gruppe, eine substituierte oder unsubstituierte C1 zu C20 Ureid-Gruppe, eine substituierte oder unsubstituierte C3 zu C40 Silyl-Gruppe, oder eine Kombination derselben.

5. Die Verbindung nach Anspruch 2, wobei
Y ist NR', und
R' entspricht Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierteC6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyclothiol-Gruppe, eine substituierte oder unsubstituierte C1 zu C20 Ureid-Gruppe, eine substituierte oder unsubstituierte C3 zu C40 Silyl-Gruppe, oder eine Kombination derselben.

6. Die Verbindung nach Anspruch 3, wobei
Y ist CR'R", und
R', R " sind gleich oder unterschiedlich und unabhängig voneinander Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierteC6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyctothiol-Gruppe, eine substituierte oder unsubstituierte C1 zu C20 Ureid-Gruppe, eine substituierte oder unsubstituierte C3 zu C40 Silyl-Gruppe, oder eine Kombination derselben,

7. Die Verbindung nach Anspruch 3, wobei
Y ist NR', und
R' entspricht Wasserstoff, Deuterium, ein Halogen, eine Cyano-Gruppe, eine Hydroxyl-Gruppe, eine Amino-Gruppe, eine substituierte oder unsubstituierte C1 zu C20 Amin-Gruppe, eine Nitro-Gruppe, eine Carboxyl-Gruppe, eine Ferrocenyl-Gruppe, eine substituierte oder unsubstituierte C1 zu C20 Alkyl-Gruppe, eine substituierte oder unsubstituierteC6 zu C30 Aryl-Gruppe, eine substituierte oder unsubstituierte C2 zu C30 Heteroaryl-Gruppe, eine substituierte oder unsubstituierte C1 zu C20 Alkoxy-Gruppe, eine substituierte oder unsubstituierte C6 zu C20 Aryloxy-Gruppe, eine substituierte oder unsubstituierte C3 zu C40 Silyloxy-Gruppe, eine substituierte oder unsubstituierte C1 zu C20 Acyl-Gruppe, eine substituierte oder unsubstituierte C2 zu C20 Alkoxycarbonyl-Gruppe, eine substituierte oder unsubstituierte C2 zu C20 Acyloxy-Gruppe, eine substituierte oder unsubstituierte C2 zu C20 Acylamino-Gruppe, eine substituierte oder unsubstituierte C2 zu C20 Alkoxycarbonylamino-Gruppe, eine substituierte oder unsubstituierte C7 zu C20 Aryloxycarbonylamino-Gruppe, eine substituierte oder unsubstituierte C1 zu C20 Sulfamoylamino-Gruppe, eine substituierte oder unsubstituierte C1 zu C20 Sulfonyl-Gruppe, eine substituierte oder unsubstituierte C1 zu C20 Alkylthiol-Gruppe, eine substituierte oder unsubstituierte C6 zu C20 Arylthiol-Gruppe, eine substituierte oder unsubstituierte C1 zu C20 Heterocyclothiol-Gruppe, eine substituierte oder unsubstituierte C1 zu C20 Ureid-Gruppe, eine substituierte oder unsubstituierte C3 zu C40 Silyl-Gruppe, oder eine Kombination derselben.

8. Die Verbindung nach einem der Ansprüche 1-7, wobei die Verbindung durch eine der folgenden chemischen Formeln A-1 bis A-51 repräsentiert wird:
[Chemische Formel A-1] [Chemische Formel A-2]
[Chemische Formel A-3] [Chemische Formel A-4] 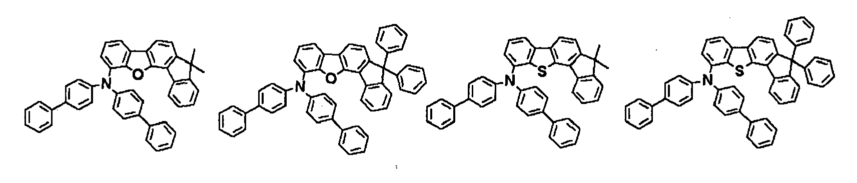
[Chemische Formel A-5] [Chemische Formel A-6]
[Chemische Formel A-7] [Chemische Formel A-8] 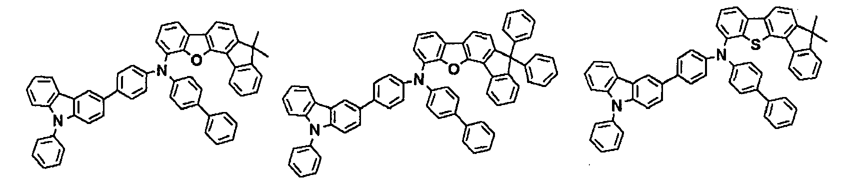
[Chemische Formel A-9] [Chemische Formel A-10] 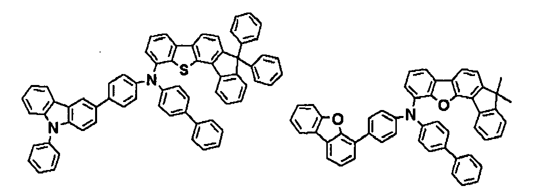
[Chemische Formel A-11] [Chemische Formel A-12]
[Chemische Formel A-13] 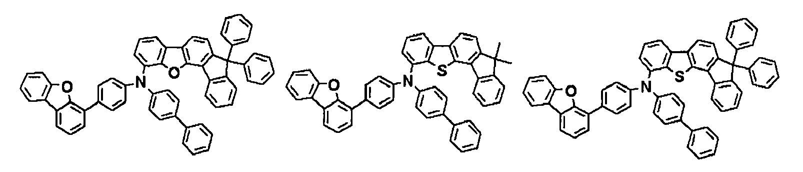
[Chemische Formel A-14] [Chemische Formel A-15]
[Chemische Formel A-16] 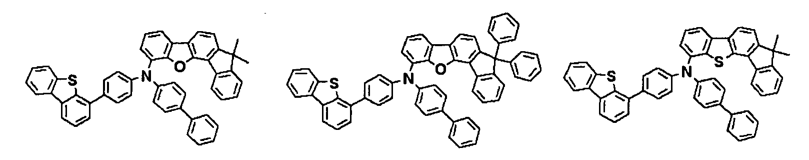
[Chemische Formel A-17] [Chemische Formel A-18] 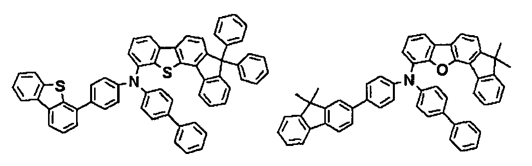
[Chemische Formel A-19] [Chemische Formel A-20]
[Chemische Formel A-21] 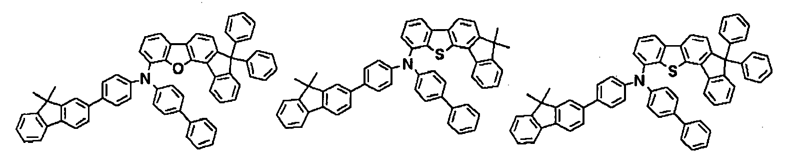
[Chemische Formel A-22] [Chemische Formel A-23] 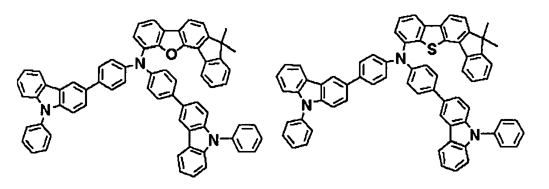
[Chemische Formel A-24] [Chemische Formel A-25]
[Chemische Formel A-26] [Chemische Formel A-27] 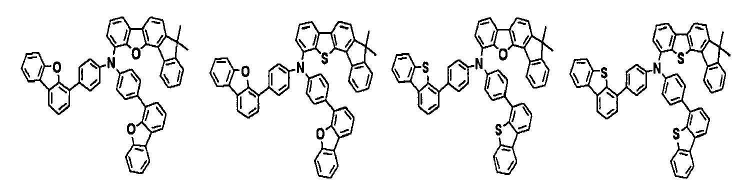
[Chemische Formel A-28] [Chemische Formel A-29]
[Chemische Formel A-30] [Chemische Formel A-31] 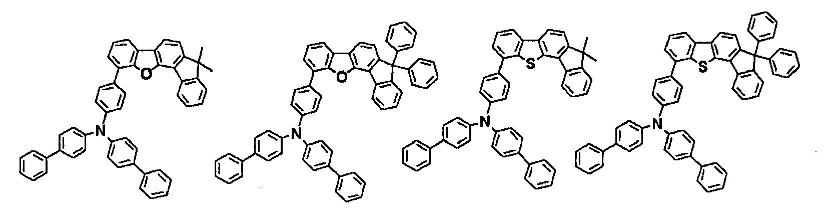
[Chemische Formel A-32] [Chemische Formel A-33]
[Chemische Formel A-34] [Chemische Formel A-35] 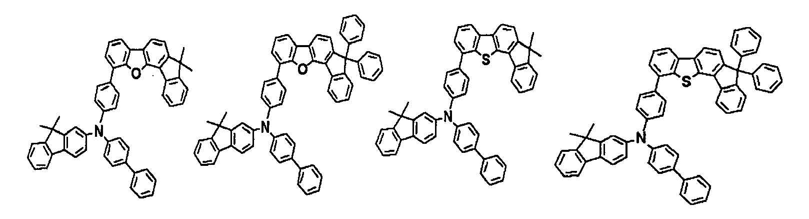
[Chemische Formel A-36] [Chemische Formel A-37]
[Chemische Formel A-38] [Chemische Formel A-39] 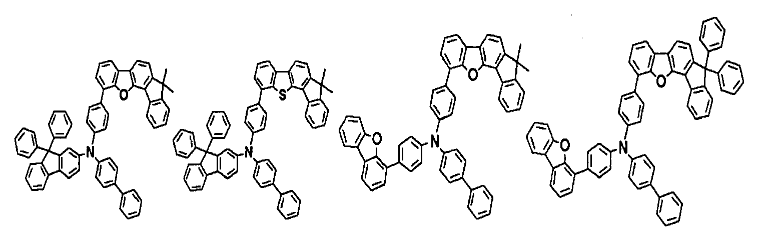
[Chemische Formel A-40] [Chemische Formel A-41] 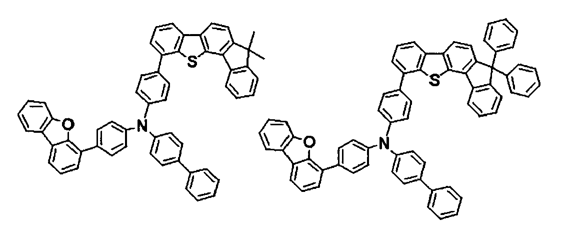
[Chemische Formel A-42] [Chemische Formel A-43]
Chemische Formel A-44] [Chemische Formel A-45] 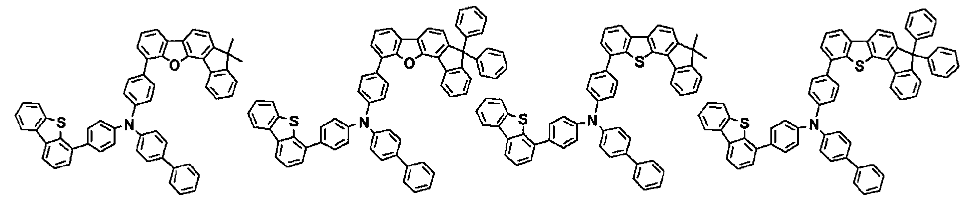
[Chemische Formel A-46] [Chemische Formel A-47]
[Chemische Formel A-48] [Chemische Formel A-49] 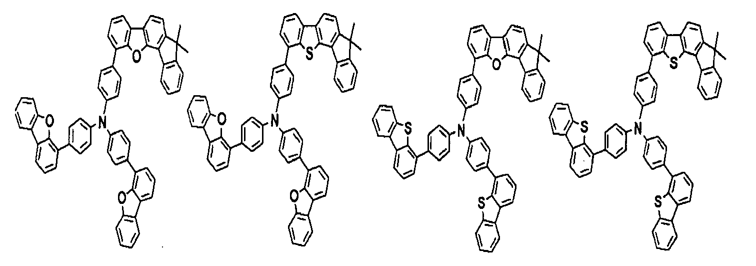
[Chemische Formel A-50] [Chemische Formel A-51]

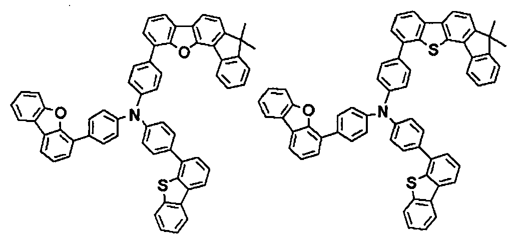

9. Verbindung nach einem der Ansprüche 1-8, wobei die Verbindung durch eine der folgenden chemischen Formeln B-1 bis B-32 repräsentiert wird:
[Chemische Formel B-1] [Chemische Formel B-2]
[Chemische Formel B-3] [Chemische Formel B-4] 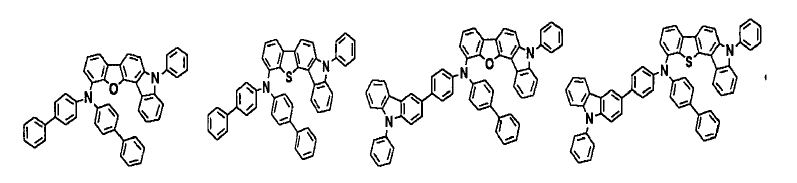
[Chemische Formel B-5] [Chemische Formel B-6]
[Chemische Formel B-7] [Chemische Formel B-8] 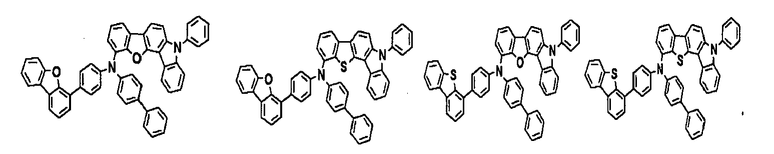
[Chemische Formel B-9] [Chemische Formel B-10]
[Chemische Formel B-11] 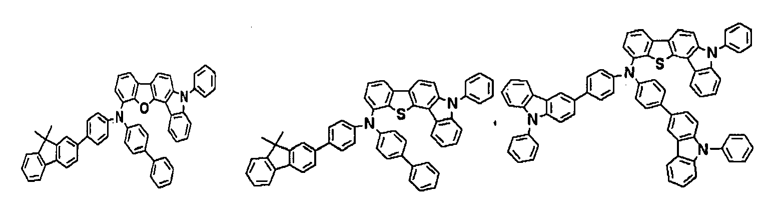
[Chemische Formel B-12] [Chemische Formel B-13]
[Chemische Formel B-14] 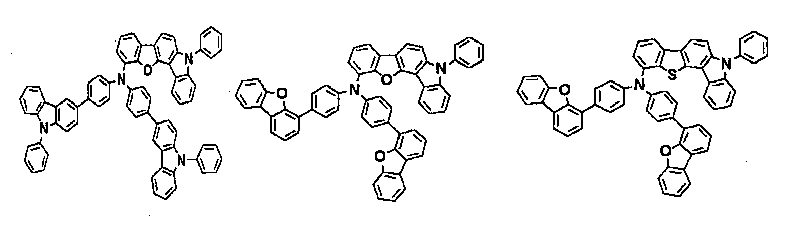
[Chemische Formel B-15] [Chemische Formel B-16]
[Chemische Formel B-17] [Chemische Formel B-18] 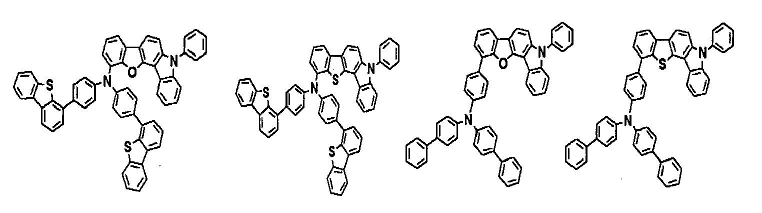
[Chemische Formel B-19] [Chemische Formel B-20]
[Chemische Formel B-21] [Chemische Formel B-22] 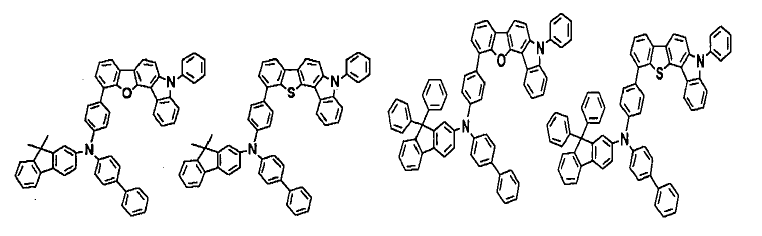
[Chemische Formel B-23] [Chemische Formel B-24]
[Chemische Formel B-25] [Chemische Formel B-26] 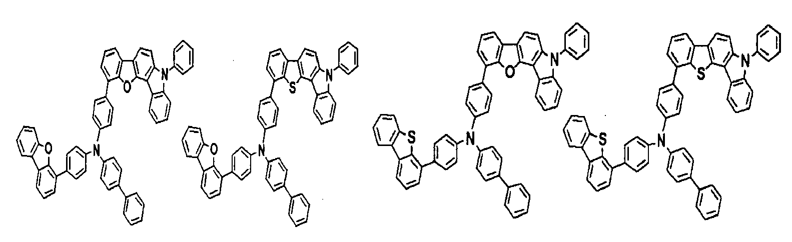
[Chemische Formel B-27] [Chemische Formel B-28]
[Chemische Formel B-29] [Chemische Formel B-30] 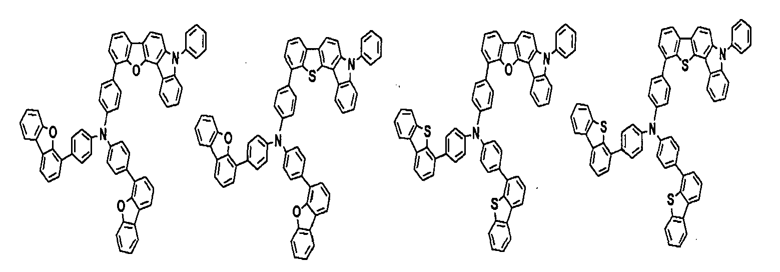
[Chemische Formel B-31] [Chemische Formel B-32]

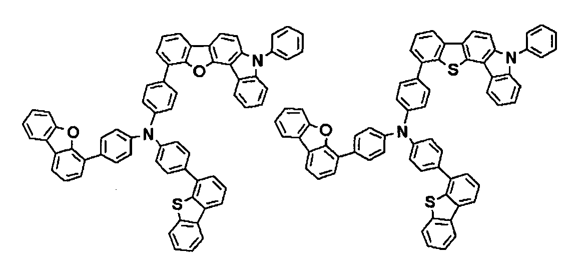

10. Verbindung nach einem der Ansprüche 1-9, wobei die Verbindung durch eine der folgenden chemischen Formeln C-1 bis C-41 repräsentiert wird:
[Chemische Formel C-1] [Chemische Formel C-2] 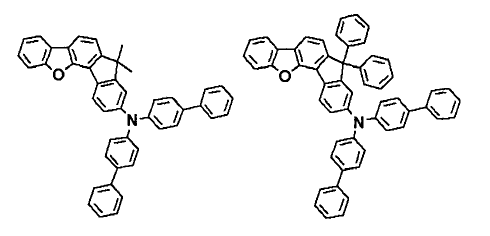
[Chemische Formel C-3] [Chemische Formel C-4]
[Chemische Formel C-5] [Chemische Formel C-6]
[Chemische Formel C-7] 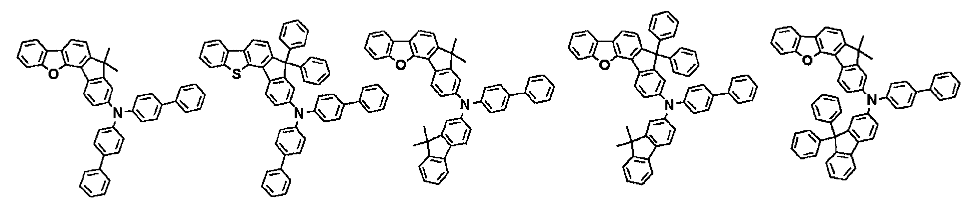
[Chemische Formel C-8] [Chemische Formel C-9]
[Chemische Formel C-10] 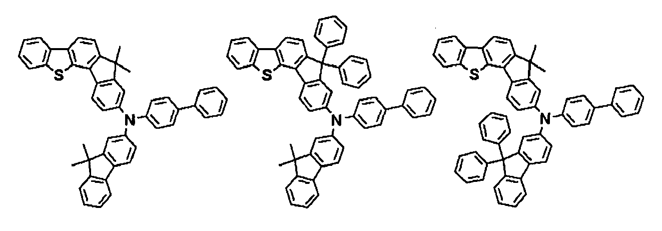
[Chemische Formel C-11] [Chemische Formel C-12]
[Chemische Formel C-13] [Chemische Formel C-14] 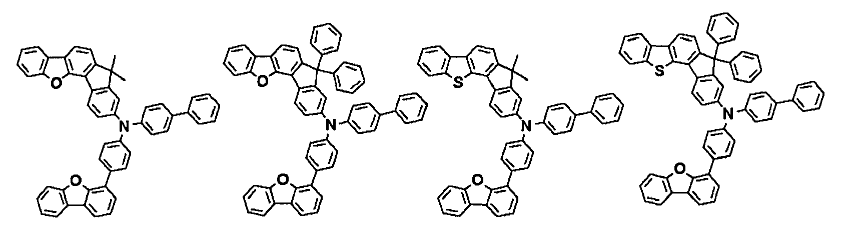
[Chemische Formel C-15] [Chemische Formel C-16]
[Chemische Formel C-17] [Chemische Formel C-18] 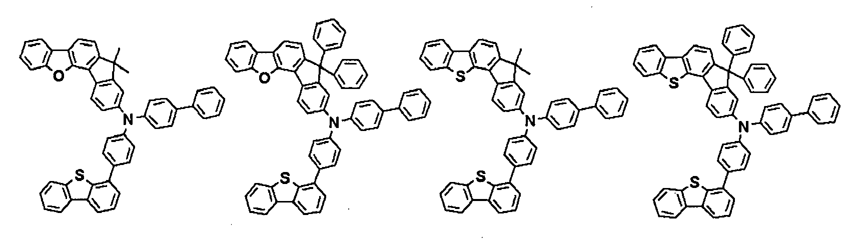
[Chemische Formel C-19] [Chemische Formel C-20]
[Chemische Formel C-21] [Chemische Formel C-22] 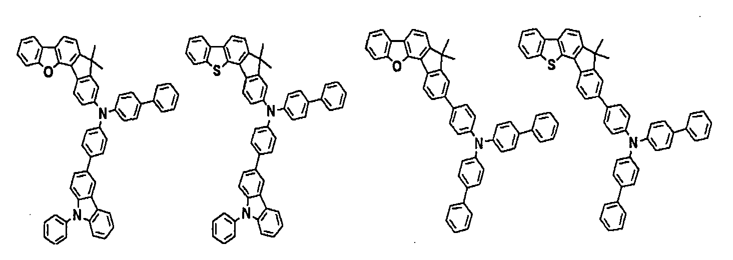
[Chemische Formel C-23] [Chemische Formel C-24]
[Chemische Formel C-25] [Chemische Formel C-26] 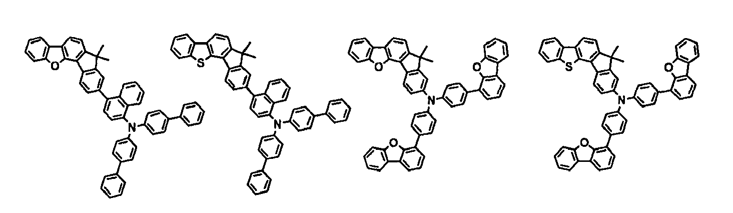
[Chemische Formel C-27] [Chemische Formel C-28]
[Chemische Formel C-29] [Chemische Formel C-30] 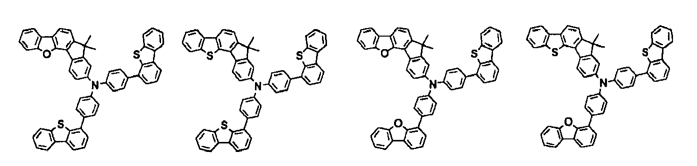
[Chemische Formel C-31] [Chemische Formel C-32]
[Chemische Formel C-33] 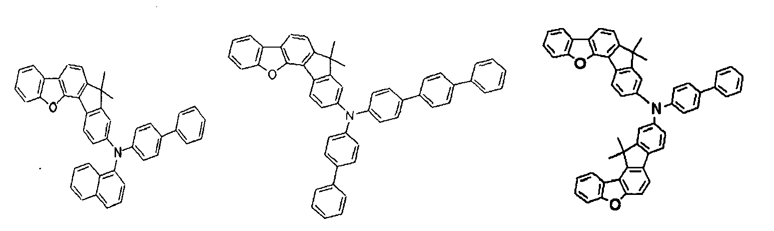
[Chemische Formel C-34] [Chemische Formel C-35]
[Chemische Formel C-36] 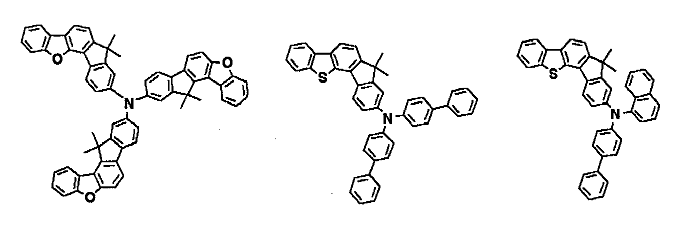
[Chemische Formel C-37] [Chemische Formel C-38]
[Chemische Formel C-39] 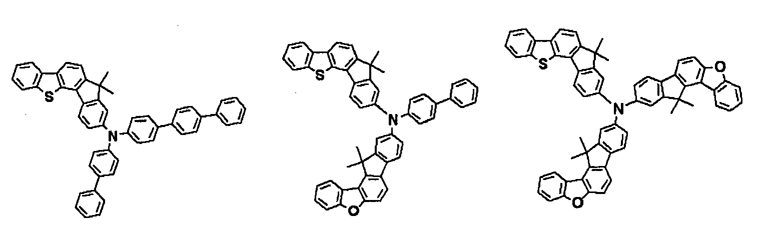
[Chemische Formel C-40] [Chemische Formel C-41]

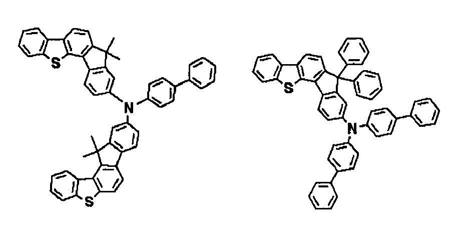

11. Verbindung nach einem der Ansprüche 1-10, wobei die Verbindung durch eine der folgenden chemischen Formeln D-1 bis D-20 repräsentiert wird:
[Chemische Formel D-1] [Chemische Formel D-2]
[Chemische Formel D-3] [Chemische Formel D-4] 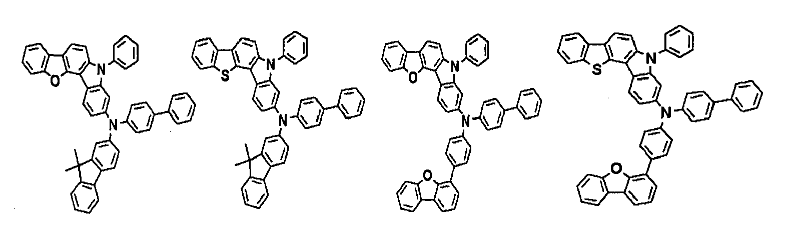
[Chemische Formel D-5] [Chemische Formel D-6]
[Chemische Formel D-7] 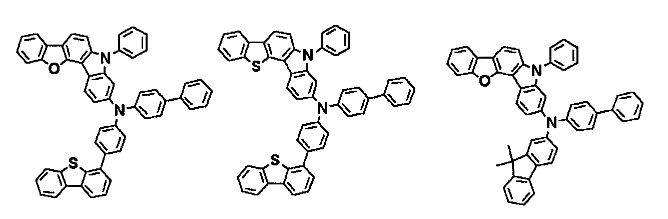
[Chemische Formel D-8] [Chemische Formel D-9]
[Chemische Formel D-10] [Chemische Formel D-11]
[Chemische Formel D-12] 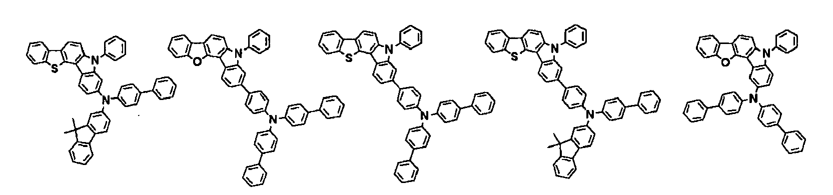
[Chemische Formel D-13] [Chemische Formel D-14]
[Chemische Formel D-15] [Chemische Formel D-16]
[Chemische Formel D-17] 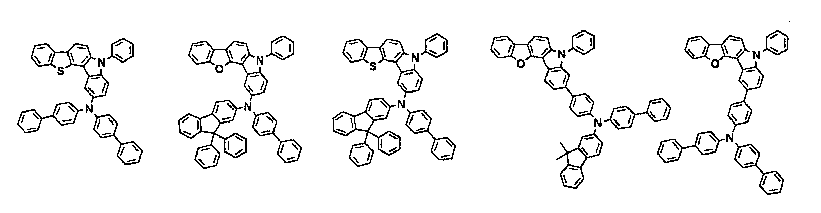
[Chemische Formel D-18] [Chemische Formel D-19]
[Chemische Formel D-20]

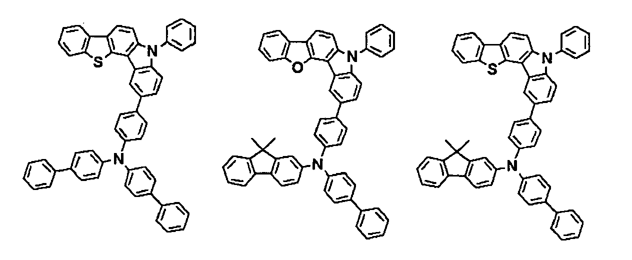

12. Verwendung einer Verbindung nach einem der Ansprüche 1 bis 11 in einer organisch opto-elektronischen Vorrichtung, wobei die organisch opto-elektronische Vorrichtung ausgesucht ist aus der Gruppe bestehend aus einer organisch photo-elektronischen Vorrichtung, einer organischen lichtemittierenden Diode, einer organischen Solarzelle, einem organischen Transistor, einer organischen photo-leitfähigen Trommel und einer organischen Speicher-Vorrichtung.

13. Verwendung einer Verbindung nach einem der Ansprüche 1 bis 11 als ein Lochtransport-Material oder eine Loch-Injektions-Material für eine Licht emittierende Diode.

14. Eine organische Licht emittierende Diode aufweisend eine Anode, eine Kathode und mehr als eine organische Dünnschicht eingefügt zwischen der Anode und der Kathode, und wobei wenigstens eine Schicht der organischen Dünnschichten die Verbindung für eine organisch opto-elektronische Vorrichtung nach Anspruch 1 bis 11 aufweist.

## Revendications

1. Composé pour un dispositif optoélectronique organique, qui est représenté par les Formules Chimiques 4 et 5 suivantes : 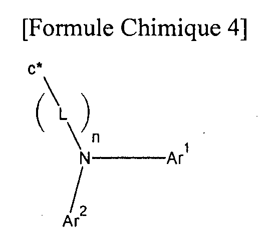 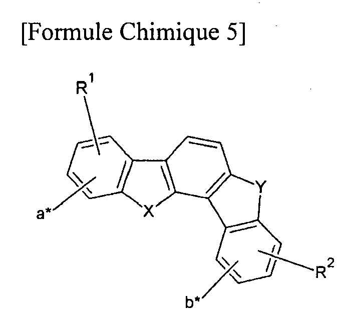 dans lequel, dans les Formules Chimiques 4 et 5,
X est O, S, SO2(O=S=O), PO(P=O) ou CO(C=O),
Y est CR'R" ou NR',
R', R", R1 et R2 sont identiques ou différents et sont indépendamment hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci,
Ar¹ et Ar² sont identiques ou différents et sont indépendamment un groupe aryle en C6 à C30 substitué ou non ou un groupe hétéroaryle en C2 à C30 substitué ou non,
L est une liaison simple, un groupe alcénylène en C2 à C6 substitué ou non, un groupe alcynylène en C2 à C6 substitué ou non, un groupe arylène en C6 à C30 substitué ou non, un groupe hétéroarylène substitué ou non, ou une combinaison de ceux-ci,
n est 0 ou 1, et
le a* ou b* de la Formule Chimique 5 ci-dessus a une liaison sigma avec le c* de la Formule Chimique 4 ci-dessus, mais le a* ou b* restant qui n'est pas connecté au c* est hydrogène.

2. Composé de la revendication 1, qui est représenté par la Formule Chimique 6 suivante : 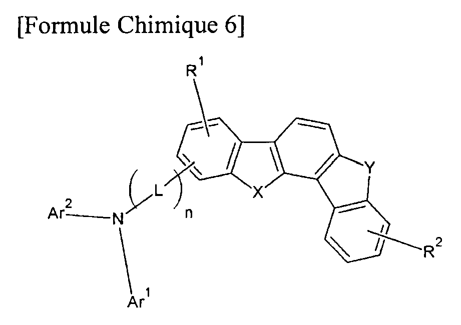 dans lequel, dans la Formule Chimique 6,
X est O, S, SO₂(O=S=O), PO(P=O) ou CO(C=O),
Y est CR'R" ou NR',
R', R", R¹ et R² sont identiques ou différents et sont indépendamment hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci,
Ar¹ et Ar² sont identiques ou différents et sont indépendamment un groupe aryle en C6 à C30 substitué ou non ou un groupe hétéroaryle en C2 à C30 substitué ou non,
L est une liaison simple, un groupe alcénylène en C2 à C6 substitué ou non, un groupe alcynylène en C2 à C6 substitué ou non, un groupe arylène en C6 à C30 substitué ou non, un groupe hétéroarylène substitué ou non, ou une combinaison de ceux-ci, et
n est 0 ou 1.

3. Composé de la revendication 1 ou de la revendication 2, qui est représenté par la Formule Chimique 7 suivante : 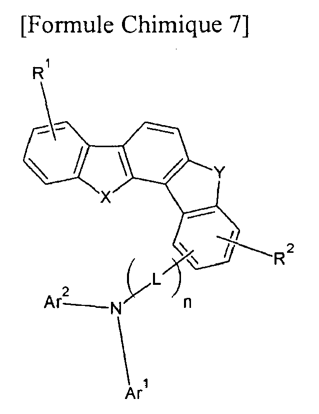 dans lequel, dans la Formule Chimique 7,
X est O, S, SO₂(O=S=O), PO(P=O) ou CO(C=O),
Y est CR'R" ou NR',
R', R", R¹ et R² sont identiques ou différents et sont indépendamment hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci,
Ar¹ et Ar² sont identiques ou différents et sont indépendamment un groupe aryle en C6 à C30 substitué ou non ou un groupe hétéroaryle en C2 à C30 substitué ou non,
L est une liaison simple, un groupe alcénylène en C2 à C6 substitué ou non, un groupe alcynylène en C2 à C6 substitué ou non, un groupe arylène en C6 à C30 substitué ou non, un groupe hétéroarylène substitué ou non, ou une combinaison de ceux-ci, et
n est 0 ou 1.

4. Composé de la revendication 2, dans lequel :
Y est CR'R", et
R', R" sont identiques ou différents et sont indépendamment hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci.

5. Composé de la revendication 2, dans lequel :
Y est NR', et
R' est hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci.

6. Composé selon la revendication 3, dans lequel :
Y est CR'R", et
R', R" sont identiques ou différents et sont indépendamment hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci.

7. Composé de la revendication 3, dans lequel :
Y est NR', et
R' est hydrogène, deutérium, un halogène, un groupe cyano, un groupe hydroxyle, un groupe amino, un groupe amine en C1 à C20 substitué ou non, un groupe nitro, un groupe carboxyle, un groupe ferrocenyl, un groupe alkyle en C1 à C20 substitué ou non, un groupe aryle en C6 à C30 substitué ou non, un groupe hétéroaryle en C2 à C30 substitué ou non, un groupe alkoxy en C1 à C20 substitué ou non, un groupe aryloxy en C6 à C20 substitué ou non, un groupe silyloxy en C3 à C40 substitué ou non, un groupe acyle en C1 à C20 substitué ou non, un groupe alkoxycarbonyle en C2 à C20 substitué ou non, un groupe acyloxy en C2 à C20 substitué ou non, un groupe acylamino en C2 à C20 substitué ou non, un groupe alkoxycarbonylamino en C2 à C20 substitué ou non, un groupe aryloxycarbonylamino en C7 à C20 substitué ou non, un groupe sulfamoylamino en C1 à C20 substitué ou non, un groupe sulfonyle en C1 à C20 substitué ou non, un groupe alkylthiol en C1 à C20 substitué ou non, un groupe arylthiol en C6 à C20 substitué ou non, un groupe hétérocyclothiol en C1 à C20 substitué ou non, un groupe uréide en C1 à C20 substitué ou non, un groupe silyle en C3 à C40 substitué ou non, ou une combinaison de ceux-ci.

8. Composé de l'une quelconque des revendications 1 à 7, qui est représenté par l'une des Formules Chimiques A-1 à A-51 suivantes :
[Formule Chimique A-1] [Formule Chimique A-2]
[Formule Chimique A-3] [Formule Chimique A-4] 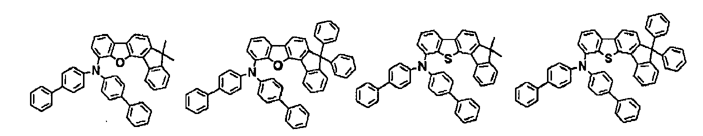
[Formule Chimique A-5] [Formule Chimique A-6]
[Formule Chimique A-7] [Formule Chimique A-8] 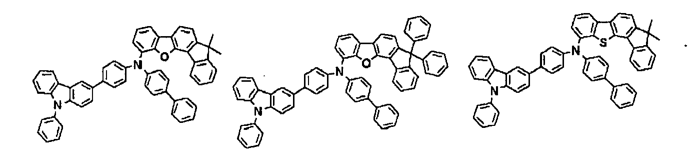
[Formule Chimique A-9] [Formule Chimique A-10] 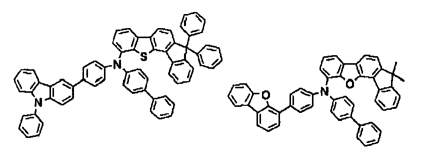
[Formule Chimique A-11] [Formule Chimique A-12]
[Formule Chimique A-13] 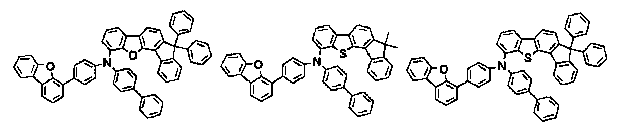
[Formule Chimique A-14] [Formule Chimique A-15]
[Formule Chimique A-16] 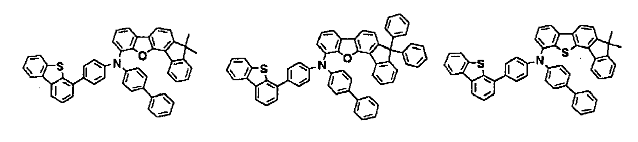
[Formule Chimique A-17] [Formule Chimique A-18] 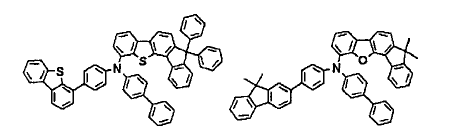
[Formule Chimique A-19] [Formule Chimique A-20]
[Formule Chimique A-21] 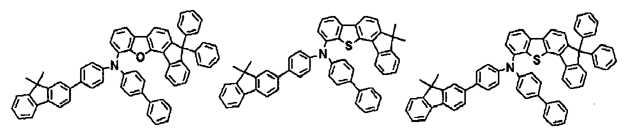
[Formule Chimique A-22] [Formule Chimique A-23] 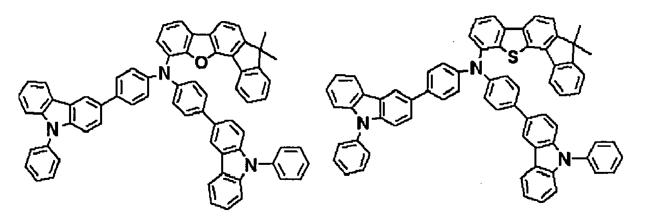
[Formule Chimique A-24] [Formule Chimique A-25]
[Formule Chimique A-26] [Formule Chimique A-27] 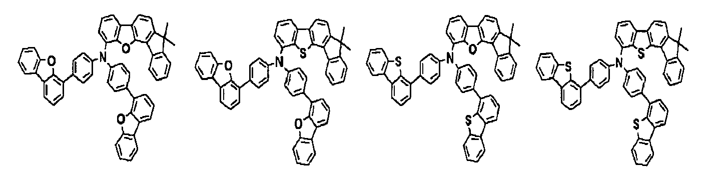
[Formule Chimique A-28] [Formule Chimique A-29]
[Formule Chimique A-30] [Formule Chimique A-31] 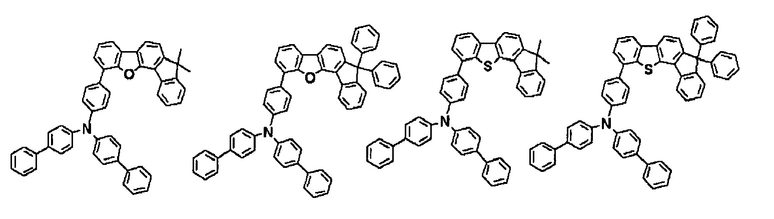
[Formule Chimique A-32] [Formule Chimique A-33]
[Formule Chimique A-34] [Formule Chimique A-35] 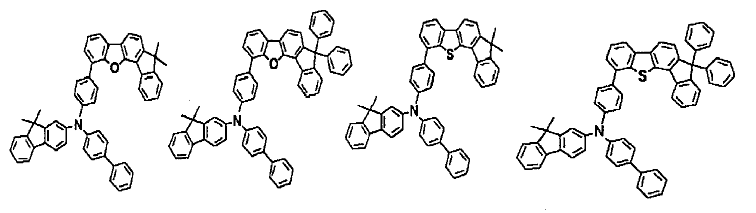
[Formule Chimique A-36] [Formule Chimique A-37]
[Formule Chimique A-38] [Formule Chimique A-39] 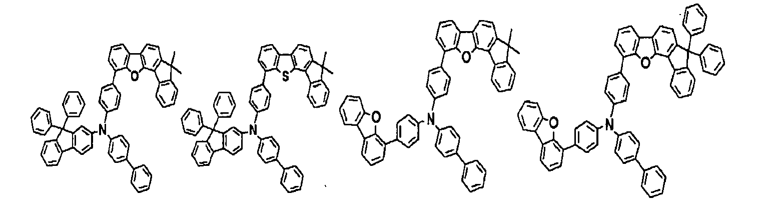
[Formule Chimique A-40] [Formule Chimique A-41] 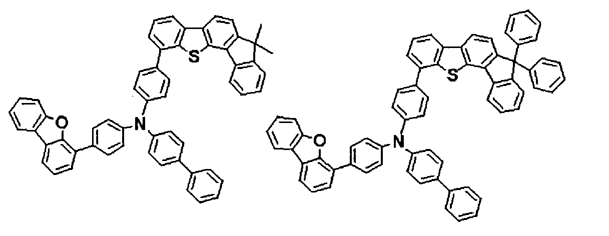
[Formule Chimique A-42] [Formule Chimique A-43]
[Formule Chimique A-44] [Formule Chimique A-45] 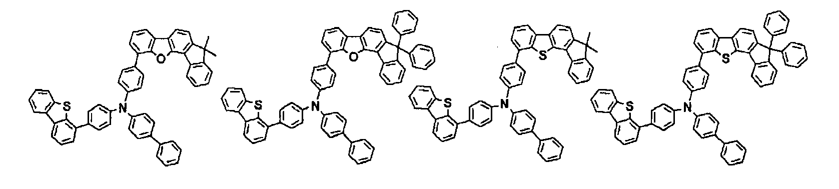
[Formule Chimique A-46] [Formule Chimique A-47]
[Formule Chimique A-48] [Formule Chimique A-49] 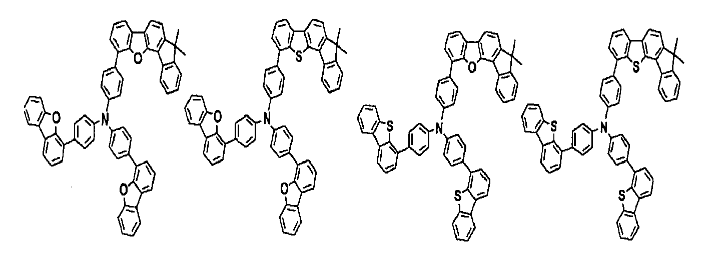
[Formule Chimique A-50] [Formule Chimique A-51]

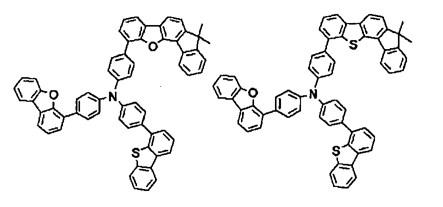

9. Composé de l'une quelconque des revendications 1 à 8, qui est représenté par l'une des Formules Chimiques B-1 à B-32 suivantes :
[Formule Chimique B-1] [Formule Chimique B-2]
[Formule Chimique B-3] [Formule Chimique B-4] 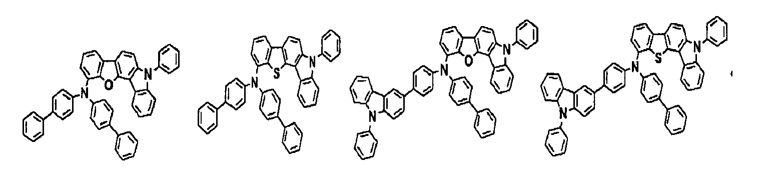
[Formule Chimique B-5] [Formule Chimique B-6]
[Formule Chimique B-7] [Formule Chimique B-8] 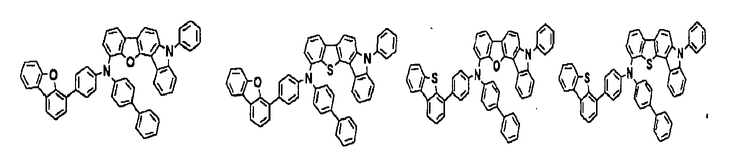
[Formule Chimique B-9] [Formule Chimique B-10]
[Formule Chimique B-11] 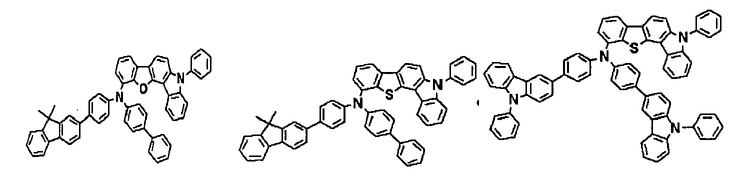
[Formule Chimique B-12] [Formule Chimique B-13]
[Formule Chimique B-14] 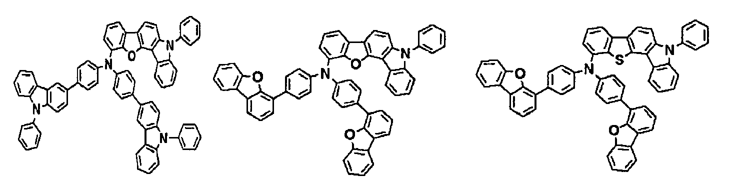
[Formule Chimique B-15] [Formule Chimique B-16]
[Formule Chimique B-17] [Formule Chimique B-18] 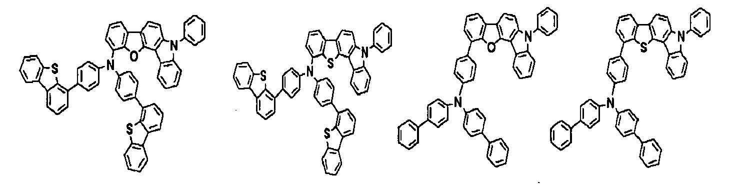
[Formule Chimique B-19] [Formule Chimique B-20]
[Formule Chimique B-21] [Formule Chimique B-22] 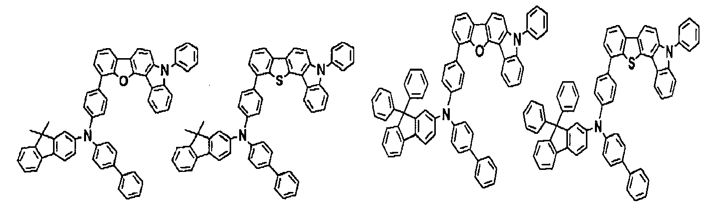
[Formule Chimique B-23] [Formule Chimique B-24]
[Formule Chimique B-25] [Formule Chimique B-26] 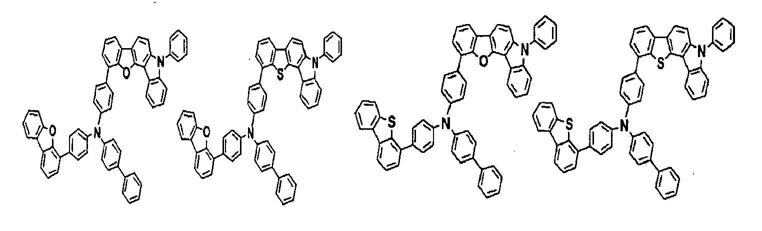
[Formule Chimique B-27] [Formule Chimique B-28]
[Formule Chimique B-29] [Formule Chimique B-30] 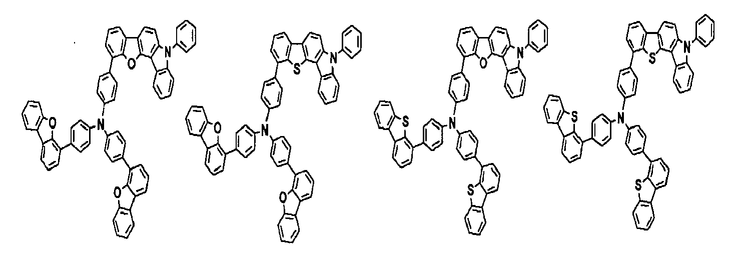
[Formule Chimique B-31] [Formule Chimique B-32]

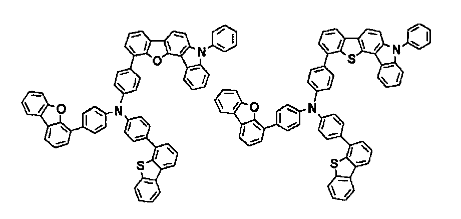

10. Composé de l'une quelconque des revendications 1 à 9, qui est représenté par l'une des Formules Chimiques C-1 à C-41 suivantes :
[Formule Chimique C-1] [Formule Chimique C-2] 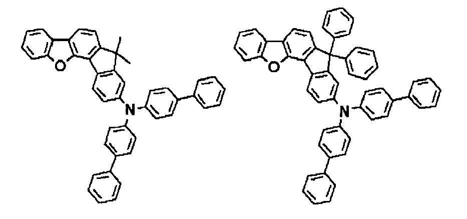
[Formule Chimique C-3] [Formule Chimique C-4]
[Formule Chimique C-5] [Formule Chimique C-6]
[Formule Chimique C-7] 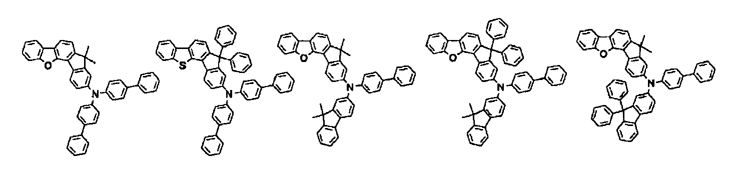
[Formule Chimique C-8] [Formule Chimique C-9]
[Formule Chimique C-10] 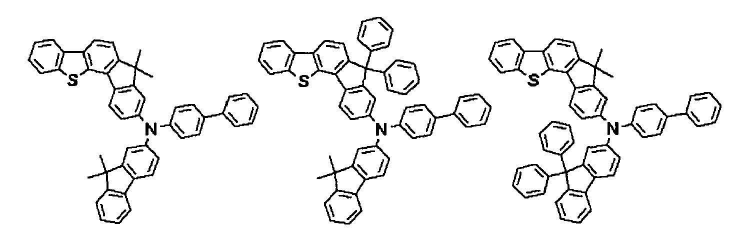
[Formule Chimique C-11] [Formule Chimique C-12]
[Formule Chimique C-13] [Formule Chimique C-14] 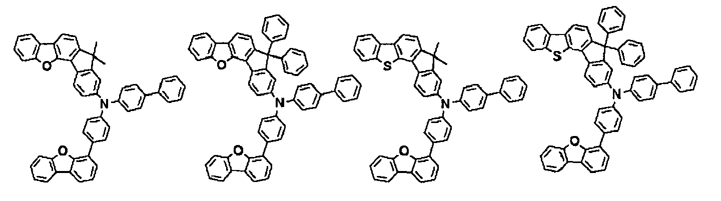
[Formule Chimique C-15] [Formule Chimique C-16]
[Formule Chimique C-17] [Formule Chimique C-18] 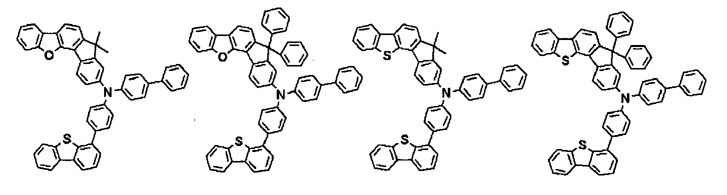
[Formule Chimique C-19] [Formule Chimique C-20]
[Formule Chimique C-21] [Formule Chimique C-22] 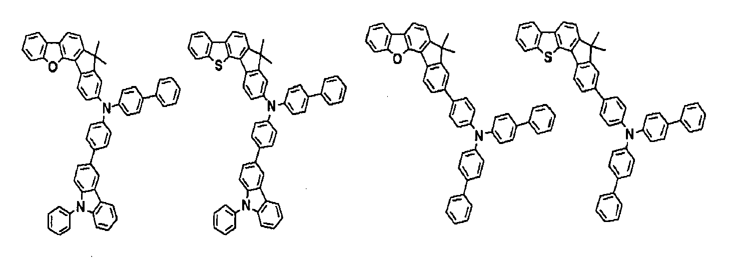
[Formule Chimique C-23] [Formule Chimique C-24]
[Formule Chimique C-25] [Formule Chimique C-26] 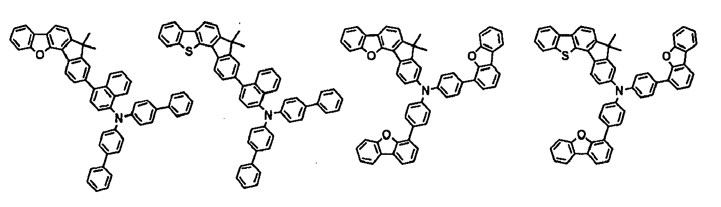
[Formule Chimique C-27] [Formule Chimique C-28]
[Formule Chimique C-29] [Formule Chimique C-30] 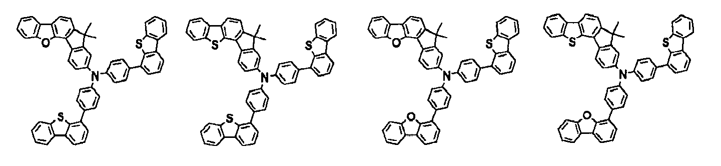
[Formule Chimique C-31] [Formule Chimique C-32]
[Formule Chimique C-33] 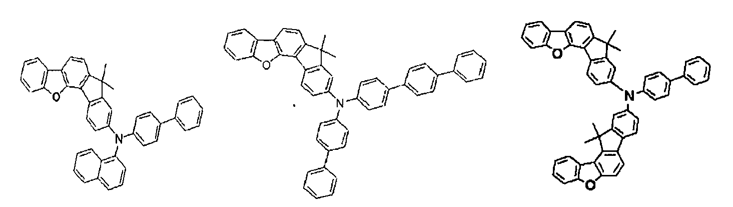
[Formule Chimique C-34] [Formule Chimique C-35]
[Formule Chimique C-36] 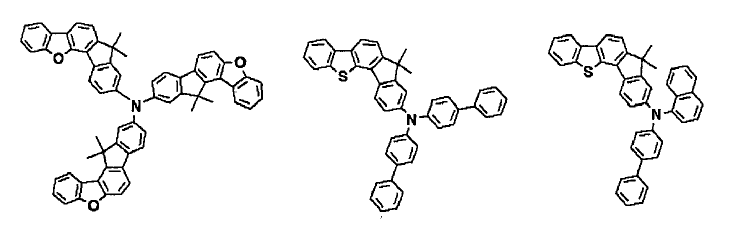
[Formule Chimique C-37] [Formule Chimique C-38]
[Formule Chimique C-39] 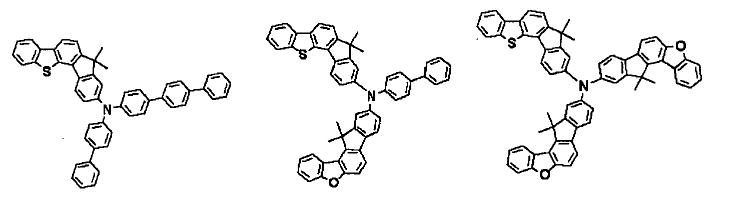
[Formule Chimique C-40] [Formule Chimique C-41]

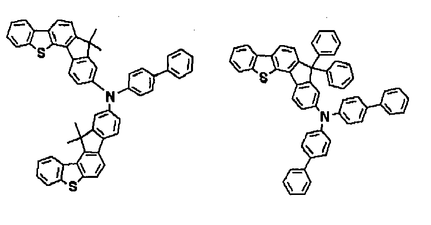

11. Composé de l'une quelconque des revendications 1 à 10, qui est représenté par l'une des Formules Chimiques D-1 à D-20 suivantes :
[Formule Chimique D-1] [Formule Chimique D-2]
[Formule Chimique D-3] [Formule Chimique D-4] 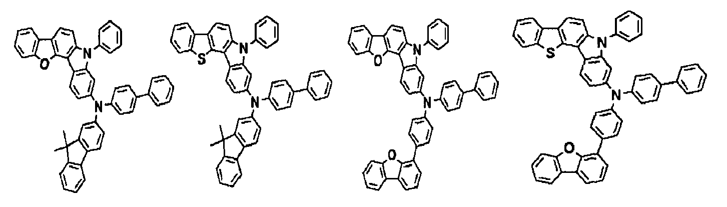
[Formule Chimique D-5] [Formule Chimique D-6]
[Formule Chimique D-7] 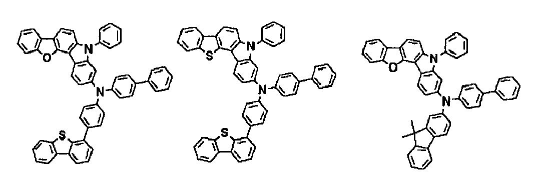
[Formule Chimique D-8] [Formule Chimique D-9
[Formule Chimique D-10] [Formule Chimique D-11]
[Formule Chimique D-12] 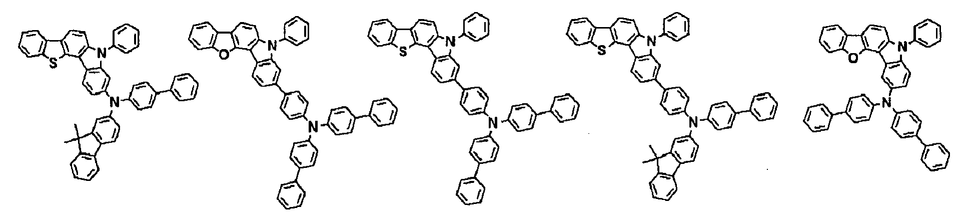
[Formule Chimique D-13] [Formule Chimique D-14]
[Formule Chimique D-15] [Formule Chimique D-16]
[Formule Chimique D-17] 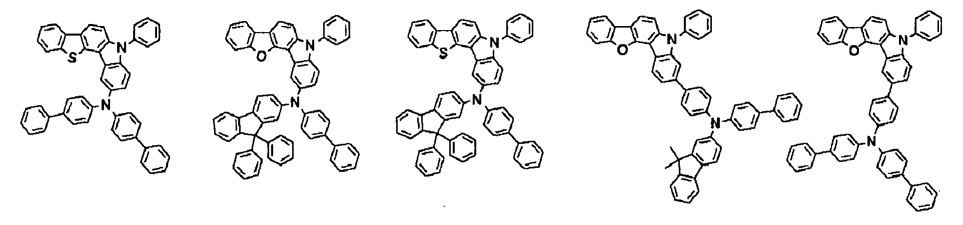
[Formule Chimique D-18] [Formule Chimique D-19]
[Formule Chimique D-20]

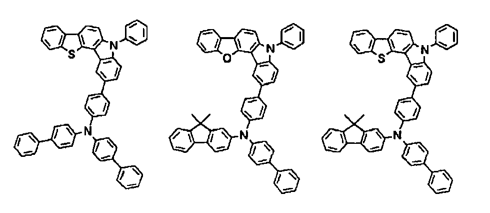

12. Utilisation d'un composé de l'une quelconque des revendications 1 à 11 dans un dispositif optoélectronique organique, dans laquelle le dispositif optoélectronique organique est sélectionné dans le groupe constitué d'un dispositif photoélectronique organique, une diode électroluminescente organique, une cellule solaire organique, un transistor organique, un tambour photoconducteur organique et un dispositif de mémoire organique.

13. Utilisation d'un composé de l'une quelconque des revendications 1 à 11 en tant qu'un matériau de transport de trou ou qu'un matériau d'injection de trou pour une diode électroluminescente organique.

14. Diode électroluminescente organique comprenant une anode, une cathode, et plus d'une couche mince organique interposée entre l'anode et la cathode, et dans laquelle au moins une couche parmi les couches minces organiques comprend le composé pour un dispositif optoélectronique organique selon les revendications 1 à 11.
